(19) Europäisches Patentamt
European Patent Office
Office européen des brevets

(11) EP 2 116 246 A1

(12) EUROPEAN PATENT APPLICATION
published in accordance with Art. 153(4) EPC

(43) Date of publication:
**11.11.2009 Bulletin 2009/46**

(21) Application number: **08703851.9**

(22) Date of filing: **18.01.2008**

(51) Int Cl.:
***A61K 31/47*** *(2006.01)* ***A61K 31/517*** *(2006.01)*
***A61K 31/7068*** *(2006.01)* ***A61P 35/00*** *(2006.01)*
***C07D 215/48*** *(2006.01)*

(86) International application number:
**PCT/JP2008/051024**

(87) International publication number:
**WO 2008/088088 (24.07.2008 Gazette 2008/30)**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **19.01.2007 US 885733 P**
**29.01.2007 US 887010 P**

(71) Applicant: **Eisai R&D Management Co., Ltd.**
**Tokyo 112-8088 (JP)**

(72) Inventor: **YAMAMOTO, Yuji**
**Tsukuba-shi**
**Ibaraki 300-2635 (JP)**

(74) Representative: **Woods, Geoffrey Corlett et al**
**J.A. KEMP & CO.**
**14 South Square**
**Gray's Inn**
**London**
**WC1R 5JJ (GB)**

# (54) COMPOSITION FOR TREATMENT OF PANCREATIC CANCER

(57) Disclosed are a pharmaceutical composition having excellent antitumor activity, and a method for treating a cancer. Specifically, excellent antitumor activity is achieved when 4-(3-chloro-4-(cyclopropylaminocarbonyl)aminophenoxy)-7-methoxy-6-quinolinecarboxamide or an analogous compound thereof, a pharmacologically acceptable salt thereof or a solvate thereof is used in combination with gemcitabine or erlotinib, a pharmacologically acceptable salt thereof or a solvate of any of them.



Printed by Jouve, 75001 PARIS (FR)

## Description

FIELD OF THE INVENTION

The present invention relates to a pharmaceutical composition and a kit comprising a compound represented by General Formula (I), a pharmacologically acceptable salt thereof or a solvate thereof (hereinafter, also referred to as the "compound of the invention") used in combination with gemcitabine or erlotinib, a pharmacologically acceptable salt thereof or a solvate thereof, to a method for treating cancer characterized by administering an effective dosage of the pharmaceutical composition to a patient, to use of a compound of the invention for producing the pharmaceutical composition, and to a compound of the invention for the pharmaceutical composition.

BACKGROUND OF THE INVENTION

Examples of substances conventionally used as chemotherapeutic agents for cancer include alkylating agents such as cyclophosphamide, antimetabolites such as methotrexate and fluorouracil, antibiotics such as adriamycin, mitomycin and bleomycin, plant-derived agents such as taxol, vincristine and etoposide and metal complexes such as cisplatin. None of them, however, have satisfactory anti-tumor effect and thus there has been a strong need for development of a novel anti-tumor agent.
An antimetabolite gemcitabine hydrochloride has been approved or developed for application to pancreatic cancers (locally advanced pancreatic cancer, metastatic pancreatic cancer), non-small cell lung cancer, bladder cancer, breast cancer, uterine cervix cancer and biliary tract cancer. In addition, combination therapy for various cancers has also been approved or developed by combining gemcitabine with various drugs, for example, with paclitaxel for unresectable locally recurrent or metastatic breast cancer, with cisplatin for progressive non-small cell lung cancer, with carboplatin for recurrent epithelial ovarian cancer, with gefitinib for non-small cell lung cancer and with erlotinib for pancreatic cancer (References 1-9).
Furthermore, 4-(3-chloro-4-(cyclopropylaminocarbonyl)aminophenoxy)-7-methoxy-6-quinolinecarboxamide is known as a VEGF receptor kinase inhibitor (References 10-11).
However, there is no report as to whether or not pharmaceutical compositions comprising these substances in combination have any anti-tumor effect.

[References]

1. Haller, DG., Int. J. Radiation Oncology Biol. Phys., 56, 16-23, 2003
2. Baker, et al, Cancer Research, 62, 1996-2003, 2002
3. Bruns, et al., International Journal of Cancer, 102, 101-108, 2002
4. International Publication No. WO2004/043472
5. International Publication No. WO2004/041308
6. International Publication No. WO2004/032872
7. International Publication No. WO2004/032937
8. International Publication No. WO2002/080975
9. Asu no Shinyaku (New Drugs of Tomorrow) 06/DEC, pp. 81-83, 2006
10. International Publication No. WO2002/32872
11. International Publication No. WO2005/063713


DISCLOSURE OF THE INVENTION

Problems to be Solved by the Invention

The present invention was achieved regarding the circumstances described above and the problems to be solved by the invention are to find a pharmaceutical composition that shows excellent anti-tumor effect and a method for treating cancer.

Means for Solving the Problems

In order to solve the above-mentioned problems, the present inventors have gone through keen research and found that 4-(3-chloro-4-(cyclopropylaminocarbonyl)aminophenoxy)-7-methoxy-6-quinolinecarboxamide shows excellent anti-tumor effect when combined with gemcitabine hydrochloride or erlotinib hydrochloride.

Thus, the present invention relates to the followings.

(1) A pharmaceutical composition comprising a compound represented by General Formula (I), a pharmacologically acceptable salt thereof or a solvate thereof in combination with gemcitabine or erlotinib, a pharmacologically acceptable salt thereof or a solvate thereof.

(2) A kit comprising:

(a) at least one selected from the group consisting of a packaging container, an instruction and a package insert describing use of a compound represented by General Formula (I), a pharmacologically acceptable salt thereof or a solvate thereof in combination with gemcitabine or erlotinib, a pharmacologically acceptable salt thereof or a solvate thereof; and

(b) a pharmaceutical composition comprising a compound represented by General Formula (I), a pharmacologically acceptable salt thereof or a solvate thereof.

(3) A kit characterized by a set of a formulation containing a compound represented by General Formula (I), a pharmacologically acceptable salt thereof or a solvate thereof and a formulation containing gemcitabine or erlotinib, a pharmacologically acceptable salt thereof or a solvate thereof.

(4) A pharmaceutical composition comprising a compound represented by General Formula (I), a pharmacologically acceptable salt thereof or a solvate thereof that is administered to a patient in combination with gemcitabine or erlotinib, a pharmacologically acceptable salt thereof or a solvate thereof.

(5) A method for treating cancer characterized by administering effective dosages of a compound represented by General Formula (I), a pharmacologically acceptable salt thereof or a solvate thereof and gemcitabine or erlotinib, a pharmacologically acceptable salt thereof or a solvate thereof to a patient.

(6) Use of a compound represented by General Formula (I), a pharmacologically acceptable salt thereof or a solvate thereof for producing a pharmaceutical composition in combination with gemcitabine or erlotinib, a pharmacologically acceptable salt thereof or a solvate thereof.

(7) A compound represented by General Formula (I), a pharmacologically acceptable salt thereof or a solvate thereof for a pharmaceutical composition in combination with gemcitabine or erlotinib, a pharmacologically acceptable salt thereof or a solvate thereof.

(8) A compound represented by General Formula (I), a pharmacologically acceptable salt thereof or a solvate thereof for treating or preventing cancer, in combination with gemcitabine or erlotinib, a pharmacologically acceptable salt thereof or a solvate thereof.

The compound represented by General Formula (I), a pharmacologically acceptable salt thereof or a solvate thereof is as follows:

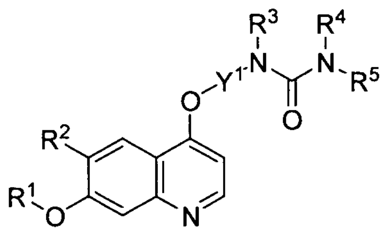

(I)

[wherein, $R^1$ represents a group represented by Formula $-V^1-V^2-V^3$ (wherein, $V^1$ represents an optionally substituted $C_{1-6}$ alkylene group; $V^2$ represents a single bond, an oxygen atom, a sulfur atom, a carbonyl group, a sulfinyl group, a sulfonyl group, a group represented by Formula $-CONR^6-$, a group represented by Formula $-SO_2NR^6-$, a group represented by Formula $-NR^6SO_2-$, a group represented by Formula $-NR^6CO-$ or a group represented by Formula $-NR^6-$ (wherein, $R^6$ represents a hydrogen atom, an optionally substituted $C_{1-6}$ alkyl group or an optionally substituted $C_{3-8}$ cycloalkyl group); $V^3$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group or an optionally substituted 3-10-membered nonaromatic heterocyclic group);

$R^2$ represents a cyano group, an optionally substituted $C_{1-6}$ alkoxy group, a carboxyl group, an optionally substituted $C_{2-7}$ alkoxycarbonyl group or a group represented by Formula $-CONV^{a11}V^{a12}$ (wherein, $V^{a11}$ represents a hydrogen

atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group or an optionally substituted 3-10-membered nonaromatic heterocyclic group; $V^{a12}$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group, an optionally substituted 3-10-membered nonaromatic heterocyclic group, a hydroxyl group, an optionally substituted $C_{1-6}$ alkoxy group or an optionally substituted $C_{3-8}$ cycloalkoxy group);
$Y^1$ represents a group represented by either one of the following formulae

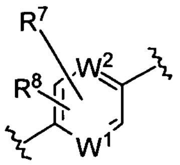

or

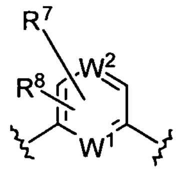

(wherein, $R^7$ and $R^8$ each independently represent a hydrogen atom, a halogen atom, a cyano group, a nitro group, an amino group, an optionally substituted $C_{1-6}$ alkyl group, an optionally substituted $C_{3-8}$ cycloalkyl group, an optionally substituted $C_{1-6}$ alkoxy group, an optionally substituted $C_{1-6}$ alkylthio group, a formyl group, an optionally substituted $C_{2-7}$ acyl group, an optionally substituted $C_{2-7}$ alkoxycarbonyl group or a group represented by Formula $-CONV^{d1}V^{d2}$ (wherein, $V^{d1}$ and $V^{d2}$ each independently represent a hydrogen atom or an optionally substituted $C_{1-6}$ alkyl group); $W^1$ and $W^2$ each independently represent an optionally substituted carbon atom or nitrogen atom);
$R^3$ and $R^4$ each independently represent a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{2-7}$ acyl group or an optionally substituted $C_{2-7}$ alkoxycarbonyl group; and
$R^5$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group or an optionally substituted 3-10-membered nonaromatic heterocyclic group],
a pharmacologically acceptable salt thereof or a solvate thereof.

Furthermore, the present invention preferably relates to the followings.

(1) A pharmaceutical composition comprising 4-(3-chloro-4-(cyclopropylaminocarbonyl)aminophenoxy)-7-methoxy-6-quinolinecarboxamide, a pharmacologically acceptable salt thereof or a solvate thereof in combination with gemcitabine or erlotinib, a pharmacologically acceptable salt thereof or a solvate thereof.

(2) A kit comprising:

(a) at least one selected from the group consisting of a packaging container, an instruction and a package insert describing use of 4-(3-chloro-4-(cyclopropylaminocarbonyl)aminophenoxy)-7-methoxy-6-quinolinecarboxamide, a pharmacologically acceptable salt thereof or a solvate thereof in combination with gemcitabine or erlotinib, a pharmacologically acceptable salt thereof or a solvate thereof; and

(b) a pharmaceutical composition comprising 4-(3-chloro-4-(cyclopropylaminocarbonyl)aminophenoxy)-7-methoxy-6-quinolinecarboxamide, a pharmacologically acceptable salt thereof or a solvate thereof.

(3) A kit characterized by a set of a formulation containing 4-(3-chloro-4-(cyclopropylaminocarbonyl)aminophenoxy)-7-methoxy-6-quinolinecarboxamide, a pharmacologically acceptable salt thereof or a solvate thereof and a formulation containing gemcitabine or erlotinib, a pharmacologically acceptable salt thereof or a solvate thereof.

(4) A pharmaceutical composition comprising 4-(3-chloro-4-(cyclopropylaminocarbonyl)aminophenoxy)-7-methoxy-6-quinolinecarboxamide, a pharmacologically acceptable salt thereof or a solvate thereof that is administered to a patient in combination with gemcitabine or erlotinib, a pharmacologically acceptable salt thereof or a solvate thereof.

(5) A method for treating cancer characterized by administering effective dosages of 4-(3-chloro-4-(cyclopropylaminocarbonyl)aminophenoxy)-7-methoxy-6-quinolinecarboxamide, a pharmacologically acceptable salt thereof or a solvate thereof and gemcitabine or erlotinib, a pharmacologically acceptable salt thereof or a solvate thereof to a patient.

(6) Use of 4-(3-chloro-4-(cyclopropylaminocarbonyl)aminophenoxy)-7-methoxy-6-quinolinecarboxamide, a pharmacologically acceptable salt thereof or a solvate thereof for producing a pharmaceutical composition in combination with gemcitabine or erlotinib, a pharmacologically acceptable salt thereof or a solvate thereof.

(7) 4-(3-Chloro-4-(cyclopropylaminocarbonyl)aminophenoxy)-7-methoxy-6-quinolinecarboxamide, a pharmacologically acceptable salt thereof or a solvate thereof for a pharmaceutical composition in combination with gemcitabine or erlotinib, a pharmacologically acceptable salt thereof or a solvate thereof.

(8) 4-(3-Chloro-4-(cyclopropylaminocarbonyl)aminophenoxy)-7-methoxy-6-quinolinecarboxamide, a pharmacologically acceptable salt thereof or a solvate thereof for treating or preventing cancer, in combination with gemcitabine or erlotinib, a pharmacologically acceptable salt thereof or a solvate thereof.

EFFECT OF THE INVENTION

The present invention provides a pharmaceutical composition comprising a compound represented by General Formula (I), a pharmacologically acceptable salt thereof or a solvate thereof in combination with gemcitabine or erlotinib, a pharmacologically acceptable salt thereof or a solvate thereof. The pharmaceutical composition of the invention can be used for the treatment of cancer.

BRIEF DESCRIPTION OF THE DRAWING

Figure 1 shows the effect of combination use of a VEGF receptor kinase inhibitor and gemcitabine hydrochloride on subcutaneous transplanted (xenograft) models of human cancer cell lines. In Figure 1, Compound A represents 4-(3-chloro-4-(cyclopropylaminocarbonyl)aminophenoxy)-7-methoxy-6-quinolinecarboxamide.

Figure 2 shows the effect of combination use of a VEGF receptor kinase inhibitor and gemcitabine hydrochloride on subcutaneous transplanted (xenograft) models of human cancer cell lines. In Figure 2, Compound A represents 4-(3-chloro-4-(cyclopropylaminocarbonyl)aminophenoxy)-7-methoxy-6-quinolinecarboxamide.

Figure 3 shows the effect of combination use of a VEGF receptor kinase inhibitor and gemcitabine hydrochloride on subcutaneous transplanted (xenograft) models of human cancer cell lines. In Figure 3, Compound A represents 4-(3-chloro-4-(cyclopropylaminocarbonyl)aminophenoxy)-7-methoxy-6-quinolinecarboxamide.

Figure 4 shows the effect of combination use of a VEGF receptor kinase inhibitor and erlotinib hydrochloride on subcutaneous transplanted (xenograft) models of human cancer cell lines. In Figure 4, Compound A represents 4-(3-chloro-4-(cyclopropylaminocarbonyl)aminophenoxy)-7-methoxy-6-quinolinecarboxamide.

BEST MODES FOR CARRYING OUT THE INVENTION

Hereinafter, embodiments of the present invention will be described. The following embodiments are examples provided for illustrating the present invention, and the present invention is not intended to be limited thereto. The present invention may be carried out in various embodiments without departing from the spirit of the invention.

The publications, laid-open patent publications, patent publications and other patent documents cited herein are entirely incorporated herein by reference. The present specification incorporates the content of the specifications of U.S. provisional application Nos. 60/885,733 (filed on 19 January, 2007) and 60/887,010 (filed on 29 January, 2007) based on which the present application claims priority.

1. Compound

Herein, a "halogen atom" refers to a fluorine atom, a chlorine atom, a bromine atom or an iodine atom.

Preferable examples of a "halogen atom" include a fluorine atom and a chlorine atom.

Herein, a "$C_{1-6}$ alkyl group" refers to a linear or branched alkyl group with a carbon number of 1-6, specific examples including a methyl group, an ethyl group, a 1-propyl group (n-propyl group), a 2-propyl group (i-propyl group), a 2-methyl-1-propyl group (i-butyl group), a 2-methyl-2-propyl group (t-butyl group), a 1-butyl group (n-butyl group), a 2-butyl group (s-butyl group), a 1-pentyl group, a 2-pentyl group, a 3-pentyl group, a 2-methyl-1-butyl group, a 3-methyl-1-butyl group, a 2-methyl-2-butyl group, a 3-methyl-2-butyl group, a 2,2-dimethyl-1-propyl group, a 1-hexyl group, a 2-hexyl group, a 3-hexyl group, a 2-methyl-1-pentyl group, a 3-methyl-1-pentyl group, a 4-methyl-1-pentyl group, a 2-methyl-2-pentyl group, a 3-methyl-2-pentyl group, a 4-methyl-2-pentyl group, a 2-methyl-3-pentyl group, a 3-methyl-3-pentyl group, a 2,3-dimethyl-1-butyl group, a 3,3-dimethyl-1-butyl group, a 2,2-dimethyl-1-butyl group, a 2-ethyl-1-butyl group, a 3,3-dimethyl-2-butyl group and a 2,3-dimethyl-2-butyl group.

Preferable examples of a "$C_{1-6}$ alkyl group" include a methyl group, an ethyl group, a 1-propyl group, a 2-propyl group, a 2-methyl-1-propyl group, a 2-methyl-2-propyl group, a 1-butyl group and a 2-butyl group.

Herein, a "$C_{1-6}$ alkylene group" refers to a divalent group derived from a "$C_{1-6}$ alkyl group" defined above by removing any one hydrogen atom therefrom, specific examples including a methylene group, a 1,2-ethylene group, a 1,1-ethylene group, a 1,3-propylene group, a tetramethylene group, a pentamethylene group and a hexamethylene group.
Herein, a "$C_{2-6}$ alkenyl group" refers to a linear or branched alkenyl group having one double bond and a carbon number of 2-6, specific examples including an ethenyl group (vinyl group), a 1-propenyl group, a 2-propenyl group (allyl group), a 1-butenyl group, a 2-butenyl group, a 3-butenyl group, a pentenyl group and a hexenyl group.
Herein, a "$C_{2-6}$ alkynyl group" refers to a linear or branched alkynyl group having one triple bond and a carbon number of 2-6, specific examples including an ethinyl group, a 1-propynyl group, a 2-propynyl group, a 1-butynyl group, a 2-butynyl group, a 3-butynyl group, a pentynyl group and a hexynyl group.
Herein, a "$C_{3-8}$ cycloalkyl group" refers to a monocyclic or bicyclic saturated aliphatic hydrocarbon group with a carbon number of 3-8, specific examples including a cyclopropyl group, a cyclobutyl group, a cyclopentyl group, a cyclohexyl group, a cycloheptyl group, a cyclooctyl group, a bicyclo[2. 1. 0]pentyl group, a bicyclo[3. 1. 0]hexyl group, a bicyclo[2. 1. 1]hexyl group, a bicyclo[4. 1. 0]heptyl group, a bicyclo[2. 2. 1]heptyl group (norbornyl group), a bicyclo[3. 3. 0]octyl group, a bicyclo[3. 2. 1]octal group and a bicyclo[2. 2. 2]octyl group.
Preferable examples of a "$C_{3-8}$ cycloalkyl group" include a cyclopropyl group, a cyclobutyl group and a cyclopentyl group.
Herein, a "$C_{6-10}$ aryl group" refers to an aromatic hydrocarbon cyclic group with a carbon number of 6-10, specific examples including a phenyl group, a 1-naphthyl group, a 2-naphthyl group, an indenyl group and an azulenyl group.
A preferable example of a "$C_{6-10}$ aryl group" includes a phenyl group.
Herein, a "heteroatom" refers to a nitrogen atom, an oxygen atom or a sulfur atom.
Herein, a "5-10-membered heteroaryl group" refers to an aromatic cyclic group having 5-10 atoms forming the ring and 1-5 heteroatoms included in the atoms forming the ring, specific examples including a furyl group, a thienyl group, a pyrrolyl group, an imidazolyl group, a triazolyl group, a tetrazolyl group, a thiazolyl group, a pyrazolyl group, an oxazolyl group, an isoxazolyl group, an isothiazolyl group, a furazanyl group, a thiadiazolyl group, an oxadiazolyl group, a pyridyl group, a pyrazinyl group, a pyridazinyl group, a pyrimidinyl group, a triazinyl group, a purinyl group, a pteridinyl group, a quinolyl group, an isoquinolyl group, a naphthiridinyl group, a quinoxalinyl group, a cinnolinyl group, a quinazolinyl group, a phthalazinyl group, an imidazopyridyl group, an imidazothiazolyl group, an imidazoxazolyl group, a benzothiazolyl group, a benzoxazolyl group, a benzimidazolyl group, an indolyl group, an isoindolyl group, an indazolyl group, a pyrrolopyridyl group, a thienopyridyl group, a furopyridyl group, a benzothiadiazolyl group, a benzoxadiazolyl group, a pyridopyrimidinyl group, a benzofuryl group, a benzothienyl group and a thienofuryl group.
Preferable examples of a "5-10-membered heteroaryl group" include a furyl group, a thienyl group, a pyrrolyl group, an imidazolyl group, a thiazolyl group, a pyrazolyl group, an oxazolyl group, an isoxazolyl group, an isothiazolyl group, a pyridyl group and a pyrimidinyl group.
Herein, a "3-10-membered nonaromatic heterocyclic group":

(1) has 3-10 atoms forming the ring;
(2) has 1-2 heteroatoms included in the atoms forming the ring;
(3) may include 1-2 double bonds in the ring;
(4) may include 1-3 carbonyl groups, sulfinyl groups or sulfonyl groups in the ring; and
(5) is a nonaromatic monocyclic or bicyclic group where when a nitrogen atom is included in the atoms forming the ring, the nitrogen atom may have a chemical bond. Specific examples include an aziridinyl group, an azetidinyl group, a pyrrolidinyl group, a piperidinyl group, an azepanyl group, an azocanyl group, a piperazinyl group, a diazepanyl group, a diazocanyl group, a diazabicyclo[2. 2. 1]heptyl group, a morpholinyl group, a thiomorpholinyl group, a 1,1-dioxothiomorpholinyl group, an oxiranyl group, an oxetanyl group, a tetrahydrofuryl group, a dioxoranyl group, a tetrahydropyranyl group, a dioxanyl group, a tetrahydrothienyl group, a tetrahydrothiopyranyl group, an oxazolidinyl group and a thiazolidinyl group.

Preferable examples of a "3-10-membered nonaromatic heterocyclic group" include an aziridinyl group, an azetidinyl group, a pyrrolidinyl group, a piperidinyl group, an azepanyl group, a piperazinyl group, a diazepanyl group, a morpholinyl group, a thiomorpholinyl group, a 1,1-dioxothiomorpholinyl group, a tetrahydrofuryl group and a tetrahydropyranyl group.
Herein, a "$C_{1-6}$ alkoxy group" refers to a group in which an oxygen atom is bound to the terminal of a "$C_{1-6}$ alkyl group" defined above, specific examples including a methoxy group, an ethoxy group, a 1-propoxy group (n-propoxy group), a 2-propoxy group (i-propoxy group), a 2-methyl-1-propoxy group (i-butoxy group), a 2-methyl-2-propoxy group (t-butoxy group), a 1-butoxy group (n-butoxy group), a 2-butoxy group (s-butoxy group), a 1-pentyloxy group, a 2-pentyloxy group, a 3-pentyloxy group, a 2-methyl-1-butoxy group, a 3-methyl-1-butoxy group, a 2-methyl-2-butoxy group, a 3-methyl-2-butoxy group, a 2,2-dimethyl-1-propoxy group, a 1-hexyloxy group, a 2-hexyloxy group, a 3-hexyloxy group,

a 2-methyl-1-pentyloxy group, a 3-methyl-1-pentyloxy group, a 4-methyl-1-pentyloxy group, a 2-methyl-2-pentyloxy group, a 3-methyl-2-pentyloxy group, a 4-methyl-2-pentyloxy group, a 2-methyl-3-pentyloxy group, a 3-methyl-3-pentyloxy group, a 2,3-dimethyl-1-butoxy group, a 3,3-dimethyl-1-butoxy group, a 2,2-dimethyl-1-butoxy group, a 2-ethyl-1-butoxy group, a 3,3-dimethyl-2-butoxy group and a 2,3-dimethyl-2-butoxy group.

Preferable examples of a "$C_{1-6}$ alkoxy group" include a methoxy group, an ethoxy group, a 1-propoxy group, a 2-propoxy group, a 2-methyl-1-propoxy group, a 2-methyl-2-propoxy group, a 1-butoxy group and a 2-butoxy group.

Herein, a "$C_{1-6}$ alkylthio group" refers to a group in which a sulfur atom is bound to the terminal of a "$C_{1-6}$ alkyl group" defined above, specific examples including a methylthio group, an ethylthio group, a 1-propylthio group (n-propylthio group), a 2-propylthio group (i-propylthio group), a 2-methyl-1-propylthio group (i-butylthio group), a 2-methyl-2-propylthio group (t-butylthio group), a 1-butylthio group (n-butylthio group), a 2-butylthio group (s-butylthio group), a 1-pentylthio group, a 2-pentylthio group, a 3-pentylthio group, a 2-methyl-1-butylthio group, a 3-methyl-1-butylthio group, a 2-methyl-2-butylthio group, a 3-methyl-2-butylthio group, a 2,2-dimethyl-1-propylthio group, a 1-hexylthio group, a 2-hexylthio group, a 3-hexylthio group, a 2-methyl-1-pentylthio group, a 3-methyl-1-pentylthio group, a 4-methyl-1-pentylthio group, a 2-methyl-2-pentylthio group, a 3-methyl-2-pentylthio group, a 4-methyl-2-pentylthio group, a 2-methyl-3-pentylthio group, a 3-methyl-3-pentylthio group, a 2,3-dimethyl-1-butylthio group, a 3,3-dimethyl-1-butylthio group, a 2,2-dimethyl-1-butylthio group, a 2-ethyl-1-butylthio group, a 3,3-dimethyl-2-butylthio group and a 2,3-dimethyl-2-butylthio group.

Preferable examples of a "$C_{1-6}$ alkylthio group" include a methylthio group, an ethylthio group, a 1-propylthio group (n-propylthio group), a 2-propylthio group (i-propylthio group), a 2-methyl-1-propylthio group (i-butylthio group), a 2-methyl-2-propylthio group (t-butylthio group), a 1-butylthio group (n-butylthio group) and a 2-butylthio group (s-butylthio group).

Herein, a "$C_{3-8}$ cycloalkoxy group" refers to a group in which an oxygen atom is bound to the terminal of a "$C_{3-8}$ cycloalkyl group" defined above, specific examples including a cyclopropoxy group, a cyclobutoxy group, a cyclopentyloxy group, a cyclohexyloxy group, a cycloheptyloxy group, a cyclooctyloxy group, a bicyclo[2. 1. 0]pentyloxy group, a bicyclo [3. 1. 0]hexyloxy group, a bicyclo[2. 1. 1]hexyloxy group, a bicyclo[4. 1. 0]heptyloxy group, a bicyclo[2. 2. 1]heptyloxy group (norbornyloxy group), a bicyclo[3. 3. 0]octyloxy group, a bicyclo[3. 2. 1]octyloxy group and a bicyclo[2. 2. 2]octyloxy group.

Preferable examples of a "$C_{3-8}$ cycloalkoxy group" include a cyclopropoxy group, a cyclobutoxy group and a cyclopentyloxy group.

Herein, a "mono-$C_{1-6}$ alkylamino group" refers to a group in which a hydrogen atom in an amino group is substituted with a "$C_{1-6}$ alkyl group" defined above, specific examples including a methylamino group, an ethylamino group, a 1-propylamino group (n-propylamino group), a 2-propylamino group (i-propylamino group), a 2-methyl-1-propylamino group (i-butylamino group), a 2-methyl-2-propylamino group (t-butylamino group), a 1-butylamino group (n-butylamino group), a 2-butylamino group (s-butylamino group), a 1-pentylamino group, a 2-pentylamino group, a 3-pentylamino group, a 2-methyl-1-butylamino group, a 3-methyl-1-butylamino group, a 2-methyl-2-butylamino group, a 3-methyl-2-butylamino group, a 2,2-dimethyl-1-propylamino group, a 1-hexylamino group, a 2-hexylamino group, a 3-hexylamino group, a 2-methyl-1-pentylamino group, a 3-methyl-1-pentylamino group, a 4-methyl-1-pentylamino group, a 2-methyl-2-pentylamino group, a 3-methyl-2-pentylamino group, a 4-methyl-2-pentylamino group, a 2-methyl-3-pentylamino group, a 3-methyl-3-pentylamino group, a 2,3-dimethyl-1-butylamino group, a 3,3-dimethyl-1-butylamino group, a 2,2-dimethyl-1-butylamino group, a 2-ethyl-1-butylamino group, a 3,3-dimethyl-2-butylamino group and a 2,3-dimethyl-2-butylamino group.

Herein, a "di-$C_{1-6}$ alkylamino group" refers to a group in which two hydrogen atoms in an amino group are substituted with an identical or different "$C_{1-6}$ alkyl group" defined above, specific examples including a N,N-dimethylamino group, a N,N-diethylamino group, a N,N-di-n-propylamino group, a N,N-di-i-propylamino group, a N,N-di-n-butylamino group, a N,N-di-i-butylamino group, a N,N-di-s-butylamino group, a N,N-di-t-butylamino group, a N-ethyl-N-methylamino group, a N-n-propyl-N-methylamino group, a N-i-propyl-N-methylamino group, a N-n-butyl-N-methylamino group, a N-i-butyl-N-methylamino group, a N-s-butyl-N-methylamino group and a N-t-butyl-N-methylamino group.

Herein, a "$C_{2-7}$ acyl group" refers to a carbonyl group bound with a "$C_{1-6}$ alkyl group" defined above, specific examples including an acetyl group, a propionyl group, an isopropionyl group, a butyryl group, an isobutyryl group, a valeryl group, an isovaleryl group and a pivaloyl group.

Herein, a "$C_{2-7}$ alkoxycarbonyl group" refers to a carbonyl group bound with a "$C_{1-6}$ alkoxy group" defined above, specific examples including a methoxycarbonyl group, an ethoxycarbonyl group, a 1-propyloxycarbonyl group, a 2-propyloxycarbonyl group and a 2-methyl-2-propoxy group.

Herein, "that may have a substituent" means "that may have one or more substituents in any combination at substitutable positions", and specific examples of the substituent include a halogen atom, a hydroxyl group, a thiol group, a nitro group, a cyano group, a formyl group, a carboxyl group, an amino group, a silyl group, a methanesulfonyl group, a $C_{1-6}$ alkyl group, a $C_{2-6}$ alkenyl group, a $C_{2-6}$ alkynyl group, a $C_{3-8}$ cycloalkyl group, a $C_{6-10}$ aryl group, a 5-10-membered heteroaryl group, a 3-10-membered nonaromatic heterocyclic group, a $C_{1-6}$ alkoxy group, a $C_{1-6}$ alkylthio group, a $C_{3-8}$

cycloalkoxy group, a mono-$C_{1-6}$ alkylamino group, a di-$C_{1-6}$ alkylamino group, a $C_{2-7}$ acyl group and a $C_{2-7}$ alkoxycarbonyl group. In this case, the $C_{1-6}$ alkyl group, the $C_{2-6}$ alkenyl group, the $C_{2-6}$ alkynyl group, the $C_{3-8}$ cycloalkyl group, the $C_{6-10}$ aryl group, the 5-10-membered heteroaryl group, the 3-10-membered nonaromatic heterocyclic group, the $C_{1-6}$ alkoxy group, the $C_{1-6}$ alkylthio group, the $C_{3-8}$ cycloalkoxy group, the mono-$C_{1-6}$ alkylamino group, the di-$C_{1-6}$ alkylamino group, the $C_{2-7}$ acyl group and the $C_{2-7}$ alkoxycarbonyl group may each independently have 1-3 groups selected from the group consisting of the following substituent groups.

<Substituent groups>

A halogen atom, a hydroxyl group, a thiol group, a nitro group, a cyano group, a $C_{1-6}$ alkyl group, a $C_{3-8}$ cycloalkyl group, a $C_{2-6}$ alkenyl group, a $C_{2-6}$ alkynyl group, a $C_{6-10}$ aryl group, a 5-10-membered heteroaryl group, a 3-10-membered nonaromatic heterocyclic group, a $C_{1-6}$ alkoxy group and a $C_{1-6}$ alkylthio group.

(A) Compound of the Invention

According to the present invention, a compound represented by General Formula (I) is as follows.

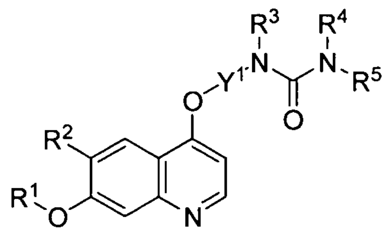

(I)

(i) $R^1$

$R^1$ represents a group represented by Formula $-V^1-V^2-V^3$ (wherein, $V^1$ represents an optionally substituted $C_{1-6}$ alkylene group; $V^2$ represents a single bond, an oxygen atom, a sulfur atom, a carbonyl group, a sulfinyl group, a sulfonyl group, a group represented by Formula $-CONR^6-$, a group represented by Formula $-SO_2NR^6-$, a group represented by Formula $-NR^6SO_2-$, a group represented by Formula $-NR^6CO-$ or a group represented by Formula $-NR^6-$ (wherein, $R^6$ represents a hydrogen atom, an optionally substituted $C_{1-6}$ alkyl group or an optionally substituted $C_{3-8}$ cycloalkyl group); $V^3$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group or an optionally substituted 3-10-membered nonaromatic heterocyclic group).

A preferable example of $R^1$ includes a $C_{1-6}$ alkyl group provided that $R^1$ may have a substituent selected from a 3-10-membered nonaromatic heterocyclic group, a hydroxyl group, a $C_{1-6}$ alkoxy group, an amino group, a mono-$C_{1-6}$ alkylamino group and a di-$C_{1-6}$ alkylamino group which may have a $C_{1-6}$ alkyl group.

More preferable examples of $R^1$ include a methyl group and a group represented by any one of the following formulae

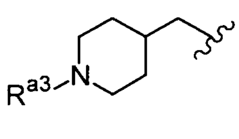

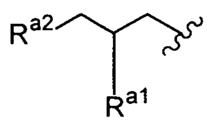

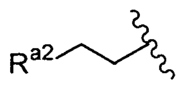

(wherein, $R^{a3}$ represents a methyl group; $R^{a1}$ represents a hydrogen atom or a hydroxyl group; $R^{a2}$ represents a methoxy group, an ethoxy group, a 1-pyrrolidinyl group, a 1-piperidinyl group, a 4-morpholinyl group, a dimethylamino group or a diethylamino group).

Still more preferable examples of $R^1$ include a methyl group and a 2-methoxyethyl group.

(ii) $R^2$

$R^2$ represents a cyano group, an optionally substituted $C_{1-6}$ alkoxy group, a carboxyl group, an optionally substituted $C_{2-7}$ alkoxycarbonyl group or a group represented by Formula $-CONV^{a11}V^{a12}$ (wherein, $V^{a11}$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group or an optionally substituted 3-10-membered nonaromatic

heterocyclic group; $V^{a12}$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group, an optionally substituted 3-10-membered nonaromatic heterocyclic group, a hydroxyl group, an optionally substituted $C_{1-6}$ alkoxy group or an optionally substituted $C_{3-8}$ cycloalkoxy group).

Preferable examples of $R^2$ include a cyano group or a group represented by Formula $-CONV^{a11}V^{a12}$ (wherein, $V^{a11}$ and $V^{a12}$ have the same meaning as defined above).

More preferable examples of $R^2$ include a cyano group or a group represented by Formula $-CONHV^{a16}$ (wherein, $V^{a16}$ represents a hydrogen atom, a $C_{1-6}$ alkyl group, a $C_{3-8}$ cycloalkyl group, a $C_{1-6}$ alkoxy group or a $C_{3-8}$ cycloalkoxy group, provided that $V^{a16}$ may have a substituent selected from a halogen atom, a cyano group, a hydroxyl group and a $C_{1-6}$ alkoxy group).

A still more preferable example of $R^2$ includes a group represented by Formula - $CONHV^{a17}$ (wherein, $V^{a17}$ represents a hydrogen atom, a $C_{1-6}$ alkyl group or a $C_{1-6}$ alkoxy group).

The most preferable example of $R^2$ includes a group represented by Formula - $CONHV^{a18}$ (wherein, $V^{a18}$ represents a hydrogen atom, a methyl group or a methoxy group).

(iii) $Y^1$

$Y^1$ represents a group represented by the following formula

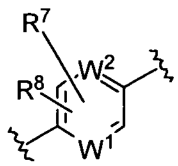

or

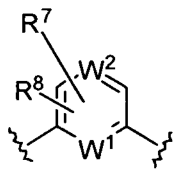

(wherein, $R^7$ and $R^8$ each independently represent a hydrogen atom, a halogen atom, a cyano group, a nitro group, an amino group, an optionally substituted $C_{1-6}$ alkyl group, an optionally substituted $C_{3-8}$ cycloalkyl group, an optionally substituted $C_{1-6}$ alkoxy group, an optionally substituted $C_{1-6}$ alkylthio group, a formyl group, an optionally substituted $C_{2-7}$ acyl group, an optionally substituted $C_{2-7}$ alkoxycarbonyl group or a group represented by Formula $-CONV^{d1}V^{d2}$ (wherein, $V^{d1}$ and $V^{d2}$ each independently represent a hydrogen atom or an optionally substituted $C_{1-6}$ alkyl group);

$W^1$ and $W^2$ each independently represent an optionally substituted carbon atom or nitrogen atom).

A preferable example of $Y^1$ includes a group represented by the following formula

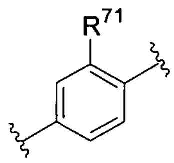

(wherein, $R^{71}$ represents a hydrogen atom or a halogen atom).

(iv) $R^3$ and $R^4$

$R^3$ and $R^4$ each independently represent a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{2-7}$ acyl group or an optionally substituted $C_{2-7}$ alkoxycarbonyl group.

A preferable example of $R^3$ and $R^4$ includes a hydrogen atom.

(v) $R^5$

$R^5$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group or an optionally substituted 3-10-membered nonaromatic heterocyclic group.

Preferable examples of $R^5$ include a hydrogen atom, a $C_{1-6}$ alkyl group, a $C_{3-8}$ cycloalkyl group and a $C_{6-10}$ aryl group (provided that $R^5$ may have a substituent selected from a halogen atom and a methanesulfonyl group).

More preferable examples of $R^5$ include a methyl group, an ethyl group and a cyclopropyl group.

Moreover, preferable examples of the compound represented by General Formula (I) include:

N-(4-(6-cyano-7-(2-methoxyethoxy)-4-quinolyl)oxy-2-fluorophenyl)-N'-(4-fluorophenyl)urea;
N-(2-chloro-4-((6-cyano-7-((1-methyl-4-piperidyl)methoxy)-4-quinolyl)oxy)phenyl)-N'-cyclopropylurea;
N-(4-((6-cyano-7-(((2R)-3-(diethylamino)-2-hydroxypropyl)oxy)-4-quinolyl)oxy)phenyl)-N'-(4-fluorophenyl)urea;
N-(4-((6-cyano-7-(((2R)-2-hydroxy-3-(1-pyrrolidino)propyl)oxy)-4-quinolyl)oxy)phenyl)-N'-(4-fluorophenyl)urea;
4-(3-chloro-4-(cyclopropylaminocarbonyl)aminophenoxy)-7-methoxy-6-quinolinecarboxamide;
4-(3-chloro-4-(cyclopropylaminocarbonyl)aminophenoxy)-7-(2-methoxyethoxy)-6-quinolinecarboxamide;
N6-cyclopropyl-4-(3-chloro-4-(((cyclopropylamino)carbonyl)amino)phenoxy)-7-methoxy-6-quinolinecarboxamide;
N6-(2-methoxyethyl)-4-(3-chloro-4-(((cyclopropylamino)carbonyl)amino)phenoxy)-7-methoxy-6-quinolinecarboxamide;
N6-(2-fluoroethyl)-4-(3-chloro-4-(((cyclopropylamino)carbonyl)amino)phenoxy)-7-methoxy-6-quinolinecarboxamide;
N6-methoxy-4-(3-chloro-4-(((cyclopropylamino)carbonyl)amino)phenoxy)-7-methoxy-6-quinolinecarboxamide;
N6-methyl-4-(3-chloro-4-(((cyclopropylamino)carbonyl)amino)phenoxy)-7-methoxy-6-quinolinecarboxamide;
N6-ethyl-4-(3-chloro-4-(((cyclopropylamino)carbonyl)amino)phenoxy)-7-methoxy-6-quinolinecarboxamide;
4-(3-fluoro-4-(cyclopropylaminocarbonyl)aminophenoxy)-7-(2-methoxyethoxy)-6-quinolinecarboxamide;
4-(3-chloro-4-(cyclopropylaminocarbonyl)aminophenoxy)-7-(2-hydroxyethoxy)-6-quinolinecarboxamide;
4-(3-chloro-4-(cyclopropylaminocarbonyl)aminophenoxy)-7-((2S)-2,3-dihydroxypropyl)oxy-6-quinolinecarboxamide;
4-(3-chloro-4-(methylaminocarbonyl)aminophenoxy)-7-methoxy-6-quinolinecarboxamide;
4-(3-chloro-4-(ethylaminocarbonyl)aminophenoxy)-7-methoxy-6-quinolinecarboxamide;
N6-methoxy-4-(3-chloro-4-(((ethylamino)carbonyl)amino)phenoxy)-7-methoxy-6-quinolinecarboxamide;
4-(3-chloro-4-(cyclopropylaminocarbonyl)aminophenoxy)-7-(2-ethoxyethoxy)-6-quinolinecarboxamide;
4-(4-((cyclopropylamino)carbonyl)aminophenoxy)-7-(2-methoxyethoxy)-6-quinolinecarboxamide;
N-(2-fluoro-4-((6-carbamoyl-7-methoxy-4-quinolyl)oxy)phenyl)-N'-cyclopropylurea;
N6-(2-hydroxyethyl)-4-(3-chloro-4-(((cyclopropylamino)carbonyl)amino)phenoxy)-7-methoxy-6-quinolinecarboxamide;
4-(3-chloro-4-(1-propylaminocarbonyl)aminophenoxy)-7-methoxy-6-quinolinecarboxamide;
4-(3-chloro-4-(cis-2-fluoro-cyclopropylaminocarbonyl)aminophenoxy)-7-methoxy-6-quinolinecarboxamide;
N6-methyl-4-(3-chloro-4-(((cyclopropylamino)carbonyl)amino)phenoxy)-7-(2-methoxyethoxy)-6-quinolinecarboxamide;
N6-methyl-4-(3-chloro-4-(((ethylamino)carbonyl)amino)phenoxy)-7-methoxy-6-quinolinecarboxamide;
4-(3-chloro-4-(cyclopropylaminocarbonyl)aminophenoxy)-7-(2-(4-morpholino)ethoxy)-6-quinolinecarboxamide;
4-(3-chloro-4-(2-fluoroethylaminocarbonyl)aminophenoxy)-7-methoxy-6-quinolinecarboxamide;
N6-((2R)tetrahydro-2-furanylmethyl)-4-(3-chloro-4-(((methylamino)carbonyl)amino)phenoxy)-7-methoxy-6-quinolinecarboxamide;
4-(3-fluoro-4-(ethylaminocarbonyl)aminophenoxy)-7-methoxy-6-quinolinecarboxamide;
4-(3-chloro-4-(((cyclopropylamino)carbonyl)amino)phenoxy)-7-((2R)-2-hydroxy-3-(1-pyrrolidino)propoxy)-6-quinolinecarboxamide;
N6-methyl-4-(3-chloro-4-(((methylamino)carbonyl)amino)phenoxy)-7-((2R)-3-diethylamino-2-hydroxypropoxy)-6-quinolinecarboxamide;
N6-methyl-4-(3-chloro-4-(((ethylamino)carbonyl)amino)phenoxy)-7-((2R)-3-diethylamino-2-hydroxypropoxy)-6-quinolinecarboxamide;
N6-methyl-4-(3-chloro-4-(((methylamino)carbonyl)amino)phenoxy)-7-((2R)-2-hydroxy-3-(1-pyrrolidino)propoxy)-6-quinolinecarboxamide;
N6-methyl-4-(3-chloro-4-(((ethylamino)carbonyl)amino)phenoxy)-7-((2R)-2-hydroxy-3-(1-pyrrolidino)propoxy)-6-quinolinecarboxamide;
N6-methyl-4-(3-chloro-4-(((methylamino)carbonyl)amino)phenoxy)-7-((1-methyl-4-piperidyl)methoxy)-6-quinolinecarboxamide;
N6-methyl-4-(3-chloro-4-(((ethylamino)carbonyl)amino)phenoxy)-7-((1-methyl-4-piperidyl)methoxy)-6-quinolinecarboxamide;
N-(4-(6-cyano-7-(2-methoxyethoxy)-4-quinolyl)oxy-2-fluorophenyl)-N'-cyclopropylurea;
N-(4-(6-cyano-7-(3-(4-morpholino)propoxy)-4-quinolyl)oxyphenyl)-N'-(3-(methylsulfonyl)phenyl)urea;
4-(4-((cyclopropylamino)carbonyl)aminophenoxy)-7-methoxy-6-quinolinecarboxamide;
4-(3-fluoro-4-((2-fluoroethylamino)carbonyl)aminophenoxy)-7-methoxy-6-quinolinecarboxamide;
N6-(2-ethoxyethyl)-4-(3-chloro-4-(((methylamino)carbonyl)amino)phenoxy)-7-methoxy-6-quinolinecarboxamide;
4-(4-(3-ethylureido)-3-fluoro-phenoxy)-7-methoxyquinoline-6-carboxylic acid (2-cyanoethyl)amide; and

N-(4-(6-(2-cyanoethyl)carbamoyl-7-methoxy-4-quinolyl)oxy-2-fluorophenyl)-N'-cyclopropylurea.

More preferable examples of the compound represented by General Formula (I) include:

4-(3-chloro-4-(cyclopropylaminocarbonyl)aminophenoxy)-7-methoxy-6-quinolinecarboxamide;
4-(3-chloro-4-(ethylaminocarbonyl)aminophenoxy)-7-methoxy-6-quinolinecarboxamide;
N6-methoxy-4-(3-chloro-4-(((cyclopropylamino)carbonyl)amino)phenoxy)-7-methoxy-6-quinolinecarboxamide;
4-(3-chloro-4-(methylaminocarbonyl)aminophenoxy)-7-methoxy-6-quinolinecarboxamide; and
N6-methoxy-4-(3-chloro-4-(((ethylamino)carbonyl)amino)phenoxy)-7-methoxy-6-quinolinecarboxamide.

A still more preferable example of the compound represented by General Formula (I) includes 4-(3-chloro-4-(cyclopropylaminocarbonyl)aminophenoxy)-7-methoxy-6-quinolinecarboxamide (see Formula (II)).
The most preferable example of the compound represented by General Formula (I), a pharmacologically acceptable salt thereof or a solvate thereof includes methanesulfonate of 4-(3-chloro-4-(cyclopropylaminocarbonyl)aminophenoxy)-7-methoxy-6-quinolinecarboxamide.

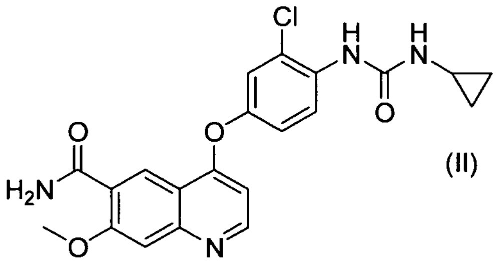

(II)

A compound represented by General formula (I) may be produced by a known method such as methods described in International Publication Nos. 02/32872 (WO02/32872) and 2005/063713 (WO2005/063713).

(B) Gemcitabine

(+)-2'-deoxy-2',2'-difluorocytidene or 2',2'-difluorodeoxycytidine (hereinafter, also referred to as "gemcitabine", see US Patent Nos. 5464826 (US5464826) and 4526988 (US4526988), Heinemann, V., et al., Cancer Research, 48, 4024-4031, 1988, and Hertel LW., et al., Cancer Research, 50, 4417-4422, 1990) (see Formula (III)) ($C_9H_{11}F_2N_3O_4$) (CAS95058-81-4)).

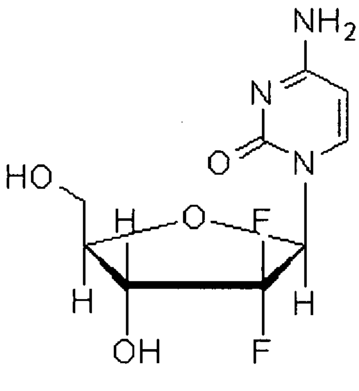

(III)

Gemcitabine may be produced according to a known method such as methods described in the respective references.
Gemcitabine is also available by purchasing Gemzar (registered trademark) from Eli Lilly & Co..

(C) Erlotinib

According to the present invention, an EGF receptor kinase inhibitor may be used in combination with a compound represented by Formula (I). Examples of EGF receptor kinase inhibitors include erlotinib and derivatives thereof. Erlotinib refers to 4-(3-ethynylphenylamino)-6,7-bis(2-methoxyethoxy)-quinazoline, which is represented by the structural formula below.

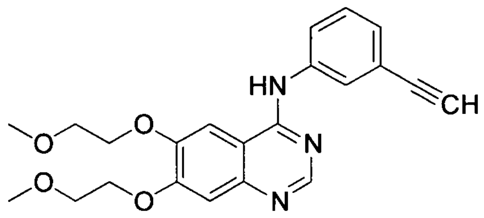

Examples of erlotinib derivatives include compounds mentioned in International Publication No. WO96/30347.
Erlotinib and derivatives thereof may be produced according to a known method, for example, by either of the methods described in International Publication No. WO96/30347 and Japanese Patent Nos. JP3088018 and JP3420549.
Moreover, erlotinib is available by purchasing Tarceva (registered trademark) from Genentech.
According to the present invention, the compound represented by General Formula (I), and/or gemcitabine and/or erlotinib may form a pharmacologically acceptable salt with acid or base. The compound represented by General Formula (I), and/or gemcitabine and/or erlotinib according to the invention also comprise such pharmacologically acceptable salts. Examples of salts formed with acids include inorganic acid salts such as hydrochloride salts, hydrobromate salts, sulfate salts and phosphate salts, and organic acid salts such as formic acid, acetic acid, lactic acid, succinic acid, fumaric acid, maleic acid, citric acid, tartaric acid, stearic acid, benzoic acid, methanesulfonic acid, benzenesulfonic acid, p-toluenesulfonic acid and trifluoroacetic acid. Examples of salts formed with bases include alkali metal salts such as sodium salt and potassium salt, alkaline earth metal salts such as calcium salt and magnesium salt, organic base salts such as trimethylamine, triethylamine, pyridine, picoline, dicyclohexylamine, N, N'-dibenzyl ethylenediamine, arginine and lysine and ammonium salts. A preferable example of gemcitabine includes gemcitabine hydrochloride. A preferable example of erlotinib includes erlotinib hydrochloride.
Furthermore, according to the present invention, the compound represented by General Formula (I), and/or gemcitabine and/or erlotinib comprise, if any, a solvate or an optical isomer thereof. Examples of solvates include hydrates and nonhydrates, preferably hydrates. Examples of solvents include water, alcohols (for example, methanol, ethanol and n-propanol) and dimethylformamide.
Moreover, according to the present invention, the compound represented by General Formula (I) may be crystalline or amorphous. If a crystalline polymorph is present, it may exist as one type of any crystalline or mixture thereof.
According to the present invention, the compound, and/or gemcitabine and/or erlotinib also comprises compounds that generate the compound represented by General Formula (I), and/or gemcitabine and/or erlotinib by undergoing metabolism such as oxidation, reduction and hydrolysis *in vivo.*

2. Pharmaceutical Composition, Kit and Method for Treating Cancer

The present invention relates to a pharmaceutical composition, a kit, a method for treating cancer and the like, **characterized in that** a compound of the invention, a pharmacologically acceptable salt thereof or a solvate thereof is combined with gemcitabine or erlotinib, a pharmacologically acceptable salt thereof or a solvate thereof.
According to the present invention, the term "combination" refers to a combination of compounds for combination use, and includes both modes where separate substances are used in combination upon administration or where they are provided as a mixture (compounding agent). Accordingly, administrations of the compound of the invention, a pharmacologically acceptable salt thereof or a solvate thereof and gemcitabine or erlotinib, a pharmacologically acceptable salt thereof or a solvate thereof are not limited to exactly the same timing. As long as the compound of the invention, a pharmacologically acceptable salt thereof or a solvate thereof and gemcitabine or erlotinib, a pharmacologically acceptable salt thereof or a solvate thereof are administered in a single administration schedule, such administrations may be referred to as "use in combination".
The form of a formulation included in a kit of the invention is not limited as long as it comprises a compound of the invention, a pharmacologically acceptable salt thereof or a solvate thereof, and/or gemcitabine and/or erlotinib, a pharmacologically acceptable salt thereof or a solvate thereof. A pharmaceutical composition and/or a kit of the invention is useful as a pharmaceutical composition and/or a kit for treating cancer.

A pharmaceutical composition and/or a kit of the invention may be used as a therapeutic agent for cancer.

According to the present invention, a therapeutic agent for cancer comprises those that contain an anti-tumor drug, a drug for improving prognosis of cancer, a drug for preventing cancer recurrence, and an antimetastatic drug or the like.

The effect of cancer treatment can be confirmed by observation of X-ray pictures, CT or the like, histopathologic diagnosis by biopsy, tumor marker value or the like.

A pharmaceutical composition and/or a kit of the invention may be administered to mammals (e.g., human, rat, rabbit, sheep, pig, cattle, cat, dog or monkey).

Examples of the types of cancers targeted by the therapeutic agent for cancer include, but not limited to, brain tumors (including hypophysial adenoma and glioma), head and neck cancer, cervical cancer, jaw cancer, maxillary cancer, submandibular gland cancer, oral cancers (including tongue cancer, mouth floor cancer, gingival cancer, buccal mucosa cancer and hard palate cancer), salivary gland cancer, sublingual gland cancer, parotid gland cancer, nasal cavity cancer, paranasal sinus cancers (including maxillary sinus cancer, frontal sinus cancer, ethmoid sinus cancer and sphenoid sinus cancer), pharyngeal cancers (including supraglottic cancer, glottic cancer and subglottic cancer), esophageal cancer, lung cancers (including bronchogenic cancer, non-small cell lung cancers (including lung adenocarcinoma, squamous lung cancer and large cell lung cancer), small cell lung cancers (including oat cell (lymphoid) and intermediate cell types) and mixed small/large cell lung cancers), breast cancer, pancreas cancers (including pancreatic duct cancer), gastric cancers (including scirrhous gastric cancer and undifferentiated gastric cancer), biliary tract cancers (including bile duct cancer and gallbladder cancer), small intestinal or duodenal cancer, colorectal cancers (colon cancer, rectal cancer, cecal cancer, sigmoid colon cancer, ascending colon cancer, transverse colon cancer and descending colon cancer), bladder cancer, renal cancers (including renal cell cancer), hepatic cancers (including hepatocellular cancer and intrahepatic bile duct cancer), prostate cancer, uterine cancers (including uterine cervix cancer and uterine body cancer), ovarian cancer, thyroid gland cancer, pharyngeal cancers (including nasopharyngeal cancer, oropharyngeal cancer and hypopharyngeal cancer), sarcomas (e.g., osteosarcoma, chondrosarcoma, Kaposi's sarcoma, myosarcoma, angiosarcoma, fibrosarcoma, etc.), malignant lymphomas (including Hodgkin's lymphoma and non-Hodgkin's lymphoma), leukemias (e.g., chronic myelocytic leukemia (CML), acute myelocytic leukemia (AML), chronic lymphocytic leukemia (CLL), acute lymphocytic leukemia (ALL), lymphoma, multiple myeloma (MM) and myelodysplastic syndrome), skin cancers (basal cell cancer, squamous cell cancer, malignant melanoma, mycosis fungoides, Sezary's syndrome, solar keratosis, Bowen's disease and Paget's disease) and melanoma.

The pharmaceutical composition and/or the kit of the invention may be administered orally or parenterally. Upon use of the pharmaceutical composition and/or the kit of the invention, the given dose of the compound of the invention, a pharmacologically acceptable salt thereof or a solvate thereof differs depending on the degree of the symptom, age, sex, weight and sensitivity difference of the patient, administration mode, administration period, administration interval, nature, prescription and type of the pharmaceutical formation and the type of the active ingredient. Usually, but without limitation, the dose is 0.1-1000 mg/day, preferably 0.5-100 mg/day and more preferably 1-30 mg/day for an adult (weight 60 kg), which may be administered usually once to three times a day.

Gemcitabine and erlotinib, a pharmacologically acceptable salt thereof or a solvate thereof may be administered according to known clinical practice. The given dose and dose schedule may be varied according to a specific case or all symptoms of the patient's disease. The dose may appropriately be reduced according to age, symptoms or incidence of side effects. Upon use of the pharmaceutical composition and/or the kit of the invention, gemcitabine or erlotinib, a pharmacologically acceptable salt thereof or a solvate thereof usually may be administered for 10-6000 mg/day, preferably 50-4000 mg/day and more preferably 50-2000 mg/day for an adult, which may be administered usually once to three times a day. The given dose needs to be reduced if undue toxicity occurs in the patient. The given dose and dose schedule may be altered when one or more additional chemotherapeutic agents are used in addition to the combination therapy of the invention. The dose schedule may be determined by the physician in charge of the treatment of the specific patient.

According to guidance, gemcitabine is usually intravenously injected for 1,000 mg/body surface area ($m^2$) of an adult per dose by spending 30 minutes according to physician's instruction. This is performed once a week for three weeks in a row followed by cessation during the fourth week. Administration may be repeated by repeating this cycle. In the case of pancreatic cancer, a recommended dose of gemcitabine is 1,000 $mg/m^2$ given by 30 minutes of intravenous injection. This may be repeated once a week for seven weeks in a row, optionally followed by a week of cessation. Subsequent cycles may consist of four week schedule where injection takes place once a week for three weeks in a row. The dose and the number of applications are usually varied according to general symptoms of the patient and severity of adverse effects, particularly adverse effects on hematopoietic system, hepatic system and renal system.

The amount of the compound of the invention used is not particularly limited, and differs depending on the individual combination with gemcitabine or erlotinib, a pharmacologically acceptable salt thereof or a solvate thereof. For example, the amount of the compound of the invention is about 0.01-100 times (weight ratio), more preferably about 0.1-10 times (weight ratio) of the amount of gemcitabine or erlotinib, a pharmacologically acceptable salt thereof or a

solvate thereof.

The pharmaceutical composition of the invention may be made into a solid oral formation, an injection or the like.

Furthermore, the compound of the invention, a pharmacologically acceptable salt thereof or a solvate thereof and gemcitabine or erlotinib, a pharmacologically acceptable salt thereof or a solvate thereof included in the kit of the invention may be made into a solid oral formation, an injection or the like, respectively.

In order to prepare a solid oral formation, the principal agent may be added with an excipient, and if necessary, a binder, a disintegrant, a lubricant, a colorant, a flavoring agent or the like, and then made into a tablet, a coated tablet, granule, subtle granule, powder, a capsule or the like according to a conventional method.

Examples of excipients used include lactose, cornstarch, sucrose, glucose, sorbit, crystalline cellulose and silicon dioxide; examples of binders used include polyvinyl alcohol, ethyl cellulose methyl cellulose, gum arabic, hydroxypropyl cellulose and hydroxypropylmethyl cellulose; examples of lubricants include magnesium stearate, talc and silica; examples of colorants include those that are allowed to be added to pharmaceutical preparations; examples of flavoring agents include cocoa powder, menthol, aromatic acid, peppermint oil, camphor and cinnamon powder. Of course, if necessary, these tablets and granule may be coated appropriately with sugar coating, gelatin coating or else.

When an injection is to be prepared, if necessary, the principal agent may be added with a pH adjuster, a buffer, a suspending agent, a solubilizing aid, a stabilizer, an isotonizing agent, a preservative or the like, and may be made into an injectable form for an intravenous, subcutaneous or intramuscular injection by a conventional technique. In this case, if necessary, it may be prepared into a lyophilized form by a conventional technique.

Examples of suspending agents may include methyl cellulose, Polysorbate 80, hydroxyethyl cellulose, gum arabic, powdered tragacanth, sodium carboxy methyl cellulose and polyoxyethylene sorbitan monolaurate.

Examples of solubilizing aids may include polyoxyethylene hydrogenated castor oil, Polysorbate 80, nicotine acid amide, polyoxyethylene sorbitan monolaurate, macrogol, and ethyl ester of castor oil fatty acid.

Examples of stabilizers may include sodium sulfite and sodium metasulfite; and examples of preservatives may include methyl paraoxybenzoate, ethyl paraoxybenzoate, sorbic acid, phenol, cresol and chlorocresol.

In the kit of the invention, a formulation containing the compound of the invention, a pharmacologically acceptable salt thereof or a solvate thereof may be mixed with a formulation containing gemcitabine or erlotinib, a pharmacologically acceptable salt thereof or a solvate thereof, or they may be kept separately and packed together. The order of administrations of the above formulations is not particularly limited, and they may be administered simultaneously or one after the other.

In addition to the compound of the invention, a pharmacologically acceptable salt thereof or a solvate thereof and gemcitabine or erlotinib, a pharmacologically acceptable salt thereof or a solvate thereof, the pharmaceutical composition and/or the kit of the invention can also comprise a packaging container, an instruction, a package insert or the like. The packaging container, the instruction, the package insert or the like may be printed with description of a combination for using the substances in combination, and description of usage and dosage for using separate substances in combination upon administration or for use of them as a mixture. The usage and dosage may be described by referring to the related description above.

The kit of the invention may comprise: (a) at least one selected from the group consisting of a packaging container, an instruction and a package insert describing combination use of the compound of the invention, a pharmacologically acceptable salt thereof or a solvate thereof with gemcitabine or erlotinib, a pharmacologically acceptable salt thereof or a solvate thereof; and (b) a pharmaceutical composition comprising the compound of the invention. This kit is useful for treating cancer. The pharmaceutical composition comprising the compound of the invention is useful for treating cancer. The packaging container, the instruction, the package insert of the like may be printed with description for using the compounds in combination, and description of usage and dosage for using separate substances in combination upon administration or for use of them as a mixture. The usage and dosage may be described by referring to the related description above.

The present invention also comprises use of a compound of the invention for producing a pharmaceutical composition in combination with gemcitabine or erlotinib, a pharmacologically acceptable salt thereof or a solvate thereof. According to the use of the invention, the pharmaceutical composition is useful for treating cancer.

The present invention also comprises a compound of the invention, a pharmacologically acceptable salt thereof or a solvate thereof for treating or preventing cancer in combination with gemcitabine or erlotinib, a pharmacologically acceptable salt thereof or a solvate thereof. According to the method for preventing or treating cancer of the invention, the route and the method for administering the compound of the invention, a pharmacologically acceptable salt thereof or a solvate thereof and gemcitabine or erlotinib, a pharmacologically acceptable salt thereof or a solvate thereof are not particularly limited but reference may be made to the description of the pharmaceutical composition of the invention above.

The present invention also comprises a method for preventing or treating cancer comprising simultaneously or separately administering a compound of the invention, a pharmacologically acceptable salt thereof or a solvate thereof and gemcitabine or erlotinib, a pharmacologically acceptable salt thereof or a solvate thereof to a patient. According to

the method of the invention for preventing or treating cancer, the route and the method for administering the compound of the invention, a pharmacologically acceptable salt thereof or a solvate thereof and gemcitabine or erlotinib, a pharmacologically acceptable salt thereof or a solvate thereof are not particularly limited but reference may be made to the description of the pharmaceutical composition of the invention above.

The present invention also comprises a pharmaceutical composition comprising a compound of the invention which is simultaneously or separately administered with gemcitabine or erlotinib, a pharmacologically acceptable salt thereof or a solvate thereof to a patient. For the pharmaceutical composition of the invention, the route and the method for administering the compound of the invention, a pharmacologically acceptable salt thereof or a solvate thereof and gemcitabine or erlotinib, a pharmacologically acceptable salt thereof or a solvate thereof are not particularly limited but reference may be made to the description of the pharmaceutical composition of the invention above.

EXAMPLES

Hereinafter, the present invention will be illustrated by way of specific examples, although the invention should not be limited thereto.

[Example 1] Combination use of the compound of the invention and gemcitabine hydrochloride in subcutaneous transplanted (xenograft) models *(in vivo)* of human pancreatic cancer cell line (AsPC-1)

Human pancreatic cancer cell line AsPC-1 (purchased from ATCC) was cultured in RPMI1640 (containing 10% FBS) in a 5% carbon dioxide gas incubator at 37°C to about 80% confluence, and then the cells were collected with trypsin-EDTA. A 5 x $10^7$ cells/mL suspension was prepared with a phosphate buffer, and each 0.1 mL of the resulting cell suspension was subcutaneously transplanted to a nude mouse at the side of its body. Eleven days after the transplantation, 4-(3-chloro-4-(cyclopropylaminocarbonyl)aminophenoxy)-7-methoxy-6-quinolinecarboxamide (3, 10 or 30 mg/kg, once a day for four weeks, oral) and gemcitabine hydrochloride (purchased from Eli Lilly Japan) (200 mg/kg, four times every three days, intravenous) were administered alone or in combination. The major and minor axes of tumors were measured with Digimatic caliper (Mitsutoyo Corporation), and tumor volumes and relative tumor volumes were calculated according to the following formulae:

$$\text{Tumor Volume (TV)} = \text{Major axis of tumor (mm)} \times (\text{Minor axis of tumor})^2 (\text{mm}^2)/2$$

$$\text{Relative Tumor Volume (RTV)} = \text{Tumor volume on measurement day/Tumor volume on the first administration day.}$$

As a result, 4-(3-chloro-4-(cyclopropylaminocarbonyl)aminophenoxy)-7-methoxy-6-quinolinecarboxamide (Compound A) showed an additive effect when used in combination with gemcitabine hydrochloride, and their combination use showed a superior anti-tumor effect as compared with those obtained with 4-(3-chloro-4-(cyclopropylaminocarbonyl) aminophenoxy)-7-methoxy-6-quinolinecarboxamide or gemcitabine hydrochloride alone (Tables 1, 2 and 3, and Figures 1, 2 and 3).

In addition, combination use of 4-(3-chloro-4-(cyclopropylaminocarbonyl)aminophenoxy)-7-methoxy-6-quinolinecarboxamide and gemcitabine hydrochloride also showed a remarkable anti-tumor effect that cannot be seen with gemcitabine hydrochloride alone (Tables 1, 2 and 3, and Figures 1, 2 and 3).

Table 1

| Administered compound | Relative tumor volume on Day 29 Average ± standard deviation | Two-way ANOVA |
|---|---|---|
| Control (untreated) | 4.91 ± 1.32 | |
| Compound A 3 mg/kg | 2.10 ± 0.64 | |
| Gemcitabine hydrochloride 200 mg/kg | 3.41 ± 0.41 | |

(continued)

| Administered compound | Relative tumor volume on Day 29 Average ± standard deviation | Two-way ANOVA |
|---|---|---|
| Compound A 3 mg/kg + Gemcitabine hydrochloride 200 mg/kg | 1.66 ± 0.62 | p=0.826 Additive effect |

Table 2

| Administered compound | Relative tumor volume on Day 29 Average ± standard deviation | Two-way ANOVA |
|---|---|---|
| Control (untreated) | 4.91 ± 1.32 | |
| Compound A 10 mg/kg | 1.79 ± 0.56 | |
| Gemcitabine hydrochloride 200 mg/kg | 3.41 ± 0.41 | |
| Compound A 10 mg/kg + Gemcitabine hydrochloride 200 mg/kg | 0.97 ± 0.20 | p=0.276 Additive effect |

Table 3

| Administered compound | Relative tumor volume on Day 29 Average ± standard deviation | Two-way ANOVA |
|---|---|---|
| Control (untreated) | 4.91 ± 1.32 | |
| Compound A 30 mg/kg | 0.88 ± 0.27 | |
| Gemcitabine hydrochloride 200 mg/kg | 3.41 ± 0.41 | |
| Compound A 30 mg/kg + Gemcitabine hydrochloride 200 mg/kg | 0.63 ± 0.19 | p=0.996 Additive effect |

Tables 1, 2 and 3 show anti-tumor effects obtained by the use of 4-(3-chloro-4-(cyclopropylaminocarbonyl) aminophenoxy)-7-methoxy-6-quinolinecarboxamide (Compound A in Tables 1, 2 and 3) alone, the use of gemcitabine hydrochloride alone and the combination use of 4-(3-chloro-4-(cyclopropylaminocarbonyl)aminophenoxy)-7-methoxy-6-quinolinecarboxamide and gemcitabine hydrochloride in subcutaneous transplanted (xenograft) models (AsPC-1). The first day of administration was considered Day 1.

According to the obtained results, the combination of 4-(3-chloro-4-(cyclopropylaminocarbonyl)aminophenoxy)-7-methoxy-6-quinolinecarboxamide and gemcitabine hydrochloride can provide a pharmaceutical composition and a kit that show a remarkable anti-tumor activity, which may be used for treating cancer.

[Example 2] Combination use of the compound of the invention and erlotinib hydrochloride in subcutaneous transplanted (xenograft) models (*in vivo*) of human pancreatic cancer cell line (AsPC-1)

Human pancreatic cancer cell line AsPC-1 (purchased from ATCC) was cultured in RPMI1640 (containing 10% FBS) in a 5% carbon dioxide gas incubator at 37°C to about 80% confluence, and then the cells were collected with trypsin-EDTA. A 5 x $10^7$ cells/mL suspension was prepared with a phosphate buffer, and each 0.1 mL of the resulting cell suspension was subcutaneously transplanted to a nude mouse at the side of its body. Ten days after the transplantation, 4-(3-chloro-4-(cyclopropylaminocarbonyl)aminophenoxy)-7-methoxy-6-quinolinecarboxamide (10 mg/kg, once a day for four weeks) and erlotinib hydrochloride (50 mg/kg, once a day for four weeks) were orally administered alone or in combination.

Erlotinib hydrochloride was synthesized by referring to the production method described in WO96/30347.

The major and minor axes of tumors were measured with Digimatic caliper (Mitsutoyo Corporation), and tumor volumes and relative tumor volumes were calculated according to the following formulae:

$$\text{Tumor Volume (TV)} = \text{Major axis of tumor (mm)} \times (\text{Minor axis of tumor})^2 (\text{mm}^2)/2$$

$$\text{Relative Tumor Volume (RTV)} = \text{Tumor volume on measurement day/Tumor volume on the first administration day.}$$

As a result, 4-(3-chloro-4-(cyclopropylaminocarbonyl)aminophenoxy)-7-methoxy-6-quinolinecarboxamide (Compound A) showed an additive effect when used in combination with erlotinib hydrochloride, and their combination use showed a superior anti-tumor effect as compared with those obtained with 4-(3-chloro-4-(cyclopropylaminocarbonyl) aminophenoxy)-7-methoxy-6-quinolinecarboxamide or erlotinib hydrochloride alone (Table 4 and Figure 4). In addition, combination use of 4-(3-chloro-4-(cyclopropylaminocarbonyl)aminophenoxy)-7-methoxy-6-quinolinecarboxamide and erlotinib hydrochloride also showed a remarkable anti-tumor effect that cannot be seen with erlotinib hydrochloride alone (Table 4 and Figure 4).

Table 4

| Administered compound | Relative tumor volume on Day 29 Average ± standard deviation | Two-way ANOVA |
|---|---|---|
| Control (untreated) | 5.46 ± 0.60 | |
| Compound A 10 mg/kg | 1.84 ± 1.31 | |
| Erlotinib hydrochloride 50 mg/kg | 4.54 ± 0.18 | |
| Compound A 10 mg/kg + Erlotinib hydrochloride 50 mg/kg | 1.04 ± 0.42 | p=0.552 Additive effect |

Table 4 shows anti-tumor effects obtained by the use of 4-(3-chloro-4-(cyclopropylaminocarbonyl)aminophenoxy)-7-methoxy-6-quinolinecarboxamide (Compound A in Table 4) alone, the use of erlotinib hydrochloride alone and the combination use of 4-(3-chloro-4-(cyclopropylaminocarbonyl)aminophenoxy)-7-methoxy-6-quinolinecarboxamide and erlotinib hydrochloride in subcutaneous transplanted (xenograft) models (AsPC-1). The first day of administration was considered Day 1.

According to the obtained results, the combination of 4-(3-chloro-4-(cyclopropylaminocarbonyl)aminophenoxy)-7-methoxy-6-quinolinecarboxamide and erlotinib hydrochloride can provide a pharmaceutical composition and a kit that show a remarkable anti-tumor activity, which may be used for treating cancer.

[Reference Example]

Hereinafter, a method for producing a formulation of one of the compounds represented by General Formula (I), i.e., 4-(3-chloro-4-(cyclopropylaminocarbonyl)aminophenoxy)-7-methoxy-6-quinolinecarboxamide, will be described as a reference example.

(Production of pharmaceutical composition)

(1) 1 mg tablet

24g of crystal (C) of methanesulfonate of 4-(3-chloro-4-(cyclopropylaminocarbonyl)aminophenoxy)-7-methoxy-6-quinolinecarboxamide (hereinafter, also referred to as "crystal (C)", which was produced according to the method described in Example 7 of W02005/063713) and 192g of light anhydrous silicic acid (antigelling agent sold under the trade name of AEROSIL (Resistered Trademark) 200, Nippon Aerosil) were mixed with 20L Super Mixer, and then 1236g of D-mannitol (excipient, Towa-Kasei Co., Ltd.), 720g of crystalline cellulose (excipient sold under the trade name of Avicel PH101, Asahi Kasei Corporation) and 72g of hydroxypropylcellulose (binder sold under the trade name of HPC-L, Nippon Soda Co., Ltd.) were further added and mixed together. Subsequently, a suitable amount of anhydrous ethanol was added to obtain a granulated body containing crystal (C). This granulated body was dried in a rack dryer (60°C),

and then size-regulated using PowerMILL to obtain granules. Together with the granules, 120g of croscarmellose sodium (disintegrant sold under the trade name of Ac-Di-Sol, FMC International Inc.) and 36g of sodium stearyl fumarate (lubricant, JRS Pharma LP) were placed and mixed together in a 20L tumbler mixer, and molded with a tablet machine to obtain tablets with a total mass of 100 mg per tablet. Furthermore, the tablets were coated using aqueous 10% Opadry yellow (OPADRY 03F42069 YELLOW, Colorcon Japan) solution as a coating solution with a tablet coating machine, thereby obtaining coated tablets with a total mass of 105 mg per tablet.

(2) 10 mg tablet

Sixty grams of crystal (C) and 192g of light anhydrous silicic acid (antigelling agent sold under the trade name of AEROSIL (Resistered Trademark) 200, Nippon Aerosil) were mixed with 20L Super Mixer, and then 1200g of D-mannitol (excipient, Towa-Kasei Co., Ltd.), 720g of crystalline cellulose (excipient sold under the trade name of Avicel PH101, Asahi Kasei Corporation) and 72g of hydroxypropylcellulose (binder sold under the trade name of HPC-L, Nippon Soda Co., Ltd.) were further added and mixed together. Subsequently, a suitable amount of anhydrous ethanol was added to obtain a granulated body containing crystal (C). This granulated body was dried in a rack dryer (60°C), and then size-regulated using PowerMILL to obtain granules. Together with the granules, 120g of croscarmellose sodium (disintegrant sold under the trade name of Ac-Di-Sol, FMC International Inc.) and 36g of sodium stearyl fumarate (lubricant, JRS Pharma LP) were placed and mixed together in a 20L tumbler mixer, and molded with a tablet machine to obtain tablets with a total mass of 400 mg per tablet. Furthermore, the tablets were coated using aqueous 10% Opadry yellow (OPADRY 03F42069 YELLOW, Colorcon Japan) solution as a coating solution with a tablet coating machine, thereby obtaining coated tablets with a total mass of 411 mg per tablet.

(3) 100 mg tablet

31.4g of crystal (C) and 4g of light anhydrous silicic acid (antigelling agent sold under the trade name of AEROSIL (Resistered Trademark) 200, Nippon Aerosil) were mixed with 1L Super Mixer, and then 40.1g of anhydrous calcium hydrogen phosphate (excipient, Kyowa Chemical Industry Co., Ltd.), 10g of low substituted hydroxypropylcellulose (binder sold under the trade name of L-HPC (LH-21), Shin-Etsu Chemical Co., Ltd.) and 3g of hydroxypropylcellulose (binder sold under the trade name of HPC-L, Nippon Soda Co., Ltd.) were further added and mixed together. Subsequently, a suitable amount of anhydrous ethanol was added to obtain a granulated body containing crystal (C). This granulated body was dried in a rack dryer (60°C), and then granulated using PowerMILL to obtain granules. Together with the granules, 10g of croscarmellose sodium (disintegrant sold under the trade name of Ac-Di-Sol, FMC International Inc.) and 1.5g of sodium stearyl fumarate (lubricant, JRS Pharma LP) were mixed and molded with a tablet machine to obtain tablets with a total mass of 400 mg per tablet.

INDUSTRIAL APPLICABILITY

According to the present invention, there is provided a pharmaceutical composition and/or a kit comprising a compound represented by General Formula (I), a pharmacologically acceptable salt thereof or a solvate thereof in combination with gemcitabine or erlotinib, which can be used for the treatment of cancer.

## Claims

**1.** A pharmaceutical composition comprising a combination of:

(i) a compound represented by General Formula (I) below, a pharmacologically acceptable salt thereof or a solvate thereof; and
(ii) gemcitabine or erlotinib, a pharmacologically acceptable salt thereof or a solvate thereof:

General Formula (I)

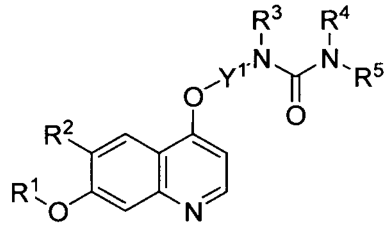

(I)

[wherein, $R^1$ represents a group represented by Formula $-V^1-V^2-V^3$ (wherein, $V^1$ represents an optionally substituted $C_{1-6}$ alkylene group; $V^2$ represents a single bond, an oxygen atom, a sulfur atom, a carbonyl group, a sulfinyl group, a sulfonyl group, a group represented by Formula $-CONR^6-$, a group represented by Formula $-SO_2NR^6-$, a group represented by Formula $-NR^6SO_2-$, a group represented by Formula $-NR^6CO-$ or a group represented by Formula $-NR^6-$ (wherein, $R^6$ represents a hydrogen atom, an optionally substituted $C_{1-6}$ alkyl group or an optionally substituted $C_{3-8}$ cycloalkyl group); $V^3$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group or an optionally substituted 3-10-membered nonaromatic heterocyclic group);

$R^2$ represents a cyano group, an optionally substituted $C_{1-6}$ alkoxy group, a carboxyl group, an optionally substituted $C_{2-7}$ alkoxycarbonyl group or a group represented by Formula $-CONV^{a11}V^{a12}$ (wherein, $V^{a11}$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group or an optionally substituted 3-10-membered nonaromatic heterocyclic group; $V^{a12}$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group, an optionally substituted 3-10-membered nonaromatic heterocyclic group, a hydroxyl group, an optionally substituted $C_{1-6}$ alkoxy group or an optionally substituted $C_{3-8}$ cycloalkoxy group);

$Y^1$ represents a group represented by either one of the following formulae

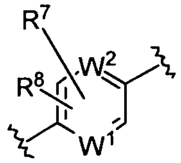

or

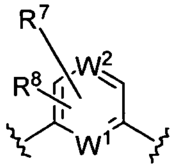

(wherein, $R^7$ and $R^8$ each independently represent a hydrogen atom, a halogen atom, a cyano group, a nitro group, an amino group, an optionally substituted $C_{1-6}$ alkyl group, an optionally substituted $C_{3-8}$ cycloalkyl group, an optionally substituted $C_{1-6}$ alkoxy group, an optionally substituted $C_{1-6}$ alkylthio group, a formyl group, an optionally substituted $C_{2-7}$ acyl group, an optionally substituted $C_{2-7}$ alkoxycarbonyl group or a group represented by Formula $-CONV^{d1}V^{d2}$ (wherein, $V^{d1}$ and $V^{d2}$ each independently represent a hydrogen atom or an optionally substituted $C_{1-6}$ alkyl group);

$W^1$ and $W^2$ each independently represent an optionally substituted carbon atom or nitrogen atom);

$R^3$ and $R^4$ each independently represent a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{2-7}$ acyl group or an optionally substituted $C_{2-7}$ alkoxycarbonyl group; and

$R^5$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group or an optionally substituted 3-10-membered nonaromatic heterocyclic group].

**2.** The pharmaceutical composition according to Claim 1, wherein $R^1$ is a $C_{1-6}$ alkyl group (provided that $R^1$ may have a substituent selected from a 3-10-membered nonaromatic heterocyclic group, a hydroxyl group, a $C_{1-6}$ alkoxy group, an amino group, a mono-$C_{1-6}$ alkylamino group and a di-$C_{1-6}$ alkylamino group which may have a $C_{1-6}$ alkyl group).

**3.** The pharmaceutical composition according to Claim 1, wherein $R^1$ is a methyl group or a group represented by any one of the following formulae

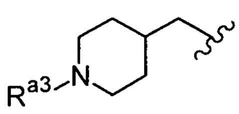
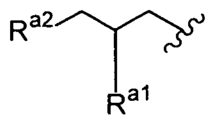
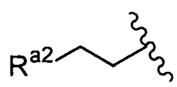

(wherein, $R^{a3}$ represents a methyl group; $R^{a1}$ represents a hydrogen atom or a hydroxyl group; $R^{a2}$ represents a methoxy group, an ethoxy group, a 1-pyrrolidinyl group, a 1-piperidinyl group, a 4-morpholinyl group, a dimethylamino group or a diethylamino group).

**4.** The pharmaceutical composition according to Claim 1, wherein $R^1$ is a methyl group or a 2-methoxyethyl group.

**5.** The pharmaceutical composition according to Claim 1, wherein $R^2$ is a cyano group or a group represented by Formula $-CONV^{a11}V^{a12}$ (wherein, $V^{a11}$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group or an optionally substituted 3-10-membered nonaromatic heterocyclic group; $V^{a12}$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group, an optionally substituted 3-10-membered nonaromatic heterocyclic group, a hydroxyl group, an optionally substituted $C_{1-6}$ alkoxy group or an optionally substituted $C_{3-8}$ cycloalkoxy group).

**6.** The pharmaceutical composition according to Claim 1, wherein $R^2$ is a cyano group or a group represented by Formula $-CONHV^{a16}$ (wherein, $V^{a16}$ represents a hydrogen atom, a $C_{1-6}$ alkyl group, a $C_{3-8}$ cycloalkyl group, a $C_{1-6}$ alkoxy group or a $C_{3-8}$ cycloalkoxy group, provided that $V^{a16}$ may have a substituent selected from a halogen atom, a cyano group, a hydroxyl group and a $C_{1-6}$ alkoxy group).

**7.** The pharmaceutical composition according to Claim 1, wherein $R^2$ is a group represented by Formula $-CONHV^{a17}$ (wherein, $V^{a17}$ represents a hydrogen atom, a $C_{1-6}$ alkyl group or a $C_{1-6}$ alkoxy group).

**8.** The pharmaceutical composition according to Claim 1, wherein $R^2$ is a group represented by Formula $-CONHV^{a18}$ (wherein, $V^{a18}$ represents a hydrogen atom, a methyl group or a methoxy group).

**9.** The pharmaceutical composition according to Claim 1, wherein $Y^1$ is a group represented by the following formula

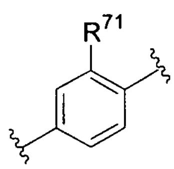

(wherein, $R^{71}$ represents a hydrogen atom or a halogen atom).

**10.** The pharmaceutical composition according to Claim 1, wherein $R^3$ and $R^4$ are hydrogen atoms.

**11.** The pharmaceutical composition according to Claim 1, wherein $R^5$ is a hydrogen atom, a $C_{1-6}$ alkyl group, a $C_{3-8}$ cycloalkyl group or a $C_{6-10}$ aryl group (provided that $R^5$ may have a substituent selected from a halogen atom and

a methanesulfonyl group).

**12.** The pharmaceutical composition according to Claim 1, wherein $R^5$ is a methyl group, an ethyl group or a cyclopropyl group.

**13.** The pharmaceutical composition according to Claim 1, wherein the compound represented by General Formula (I), a pharmacologically acceptable salt thereof or a solvate thereof is at least one compound selected from the group consisting of:

N-(4-(6-cyano-7-(2-methoxyethoxy)-4-quinolyl)oxy-2-fluorophenyl)-N'-(4-fluorophenyl)urea;
N-(2-chloro-4-((6-cyano-7-((1-methyl-4-piperidyl)methoxy)-4-quinolyl)oxy)phenyl)-N'-cyclopropylurea;
N-(4-((6- cyano- 7-(((2R)- 3-(diethylamino)- 2- hydroxypropyl) oxy)- 4- quinolyl) oxy) phenyl)-N'-(4- fluorophenyl) urea;
N-(4-((6- cyano- 7-(((2R)- 2- hydroxy- 3-(1- pyrrolidino) propyl) oxy)- 4- quinolyl) oxy) phenyl)-N'-(4- fluorophenyl) urea;
4-(3-chloro-4-(cyclopropylaminocarbonyl)aminophenoxy)-7-methoxy-6-quinolinecarboxamide;
4-(3-chloro-4-(cyclopropylaminocarbonyl)aminophenoxy)-7-(2-methoxyethoxy)-6-quinolinecarboxamide;
N6-cyclopropyl-4-(3-chloro-4-(((cyclopropylamino)carbonyl)amino)phenoxy)-7-methoxy-6-quinolinecarboxamide;
N6-(2-methoxyethyl)-4-(3-chloro-4-(((cyclopropylamino)carbonyl)amino)phenoxy)-7-methoxy-6-quinolinecarboxamide;
N6-(2-fluoroethyl)-4-(3-chloro-4-(((cyclopropylamino)carbonyl)amino)phenoxy)-7-methoxy-6-quinolinecarboxamide;
N6-methoxy-4-(3-chloro-4-(((cyclopropylamino)carbonyl)amino)phenoxy)-7-methoxy-6-quinolinecarboxamide;
N6-methyl-4-(3-chloro-4-(((cyclopropylamino)carbonyl)amino)phenoxy)-7-methoxy-6-quinolinecarboxamide;
N6-ethyl-4-(3-chloro-4-(((cyclopropylamino)carbonyl)amino)phenoxy)-7-methoxy-6-quinolinecarboxamide;
4-(3-fluoro-4-(cyclopropylaminocarbonyl)aminophenoxy)-7-(2-methoxyethoxy)-6-quinolinecarboxamide;
4-(3-chloro-4-(cyclopropylaminocarbonyl)aminophenoxy)-7-(2-hydroxyethoxy)-6-quinolinecarboxamide;
4-(3-chloro-4-(cyclopropylaminocarbonyl)aminophenoxy)-7-((2S)-2,3-dihydroxypropyl)oxy-6-quinolinecarboxamide;
4-(3-chloro-4-(methylaminocarbonyl)aminophenoxy)-7-methoxy-6-quinolinecarboxamide;
4-(3-chloro-4-(ethylaminocarbonyl)aminophenoxy)-7-methoxy-6-quinolinecarboxamide;
N6-methoxy-4-(3-chloro-4-(((ethylamino)carbonyl)amino)phenoxy)-7-methoxy-6-quinolinecarboxamide;
4-(3-chloro-4-(cyclopropylaminocarbonyl)aminophenoxy)-7-(2-ethoxyethoxy)-6-quinolinecarboxamide;
4-(4-((cyclopropylamino)carbonyl)aminophenoxy)-7-(2-methoxyethoxy)-6-quinolinecarboxamide;
N-(2-fluoro-4-((6-carbamoyl-7-methoxy-4-quinolyl)oxy)phenyl)-N'-cyclopropylurea;
N6-(2-hydroxyethyl)-4-(3-chloro-4-(((cyclopropylamino)carbonyl)amino)phenoxy)-7-methoxy-6-quinolinecarboxamide;
4-(3-chloro-4-(1-propylaminocarbonyl)aminophenoxy)-7-methoxy-6-quinolinecarboxamide;
4-(3-chloro-4-(cis-2-fluoro-cyclopropylaminocarbonyl)aminophenoxy)-7-methoxy-6-quinolinecarboxamide;
N6-methyl-4-(3-chloro-4-(((cyclopropylamino)carbonyl)amino)phenoxy)-7-(2-methoxyethoxy)-6-quinolinecarboxamide;
N6-methyl-4-(3-chloro-4-(((ethylamino)carbonyl)amino)phenoxy)-7-methoxy-6-quinolinecarboxamide;
4-(3-chloro-4-(cyclopropylaminocarbonyl)aminophenoxy)-7-(2-(4-morpholino)ethoxy)-6-quinolinecarboxamide;
4-(3-chloro-4-(2-fluoroethylaminocarbonyl)aminophenoxy)-7-methoxy-6-quinolinecarboxamide;
N6-((2R) tetrahydro-2-furanylmethyl)-4-(3-chloro-4-(((methylamino)carbonyl)amino)phenoxy)-7-methoxy-6-quinolinecarboxamide;
4-(3-fluoro-4-(ethylaminocarbonyl)aminophenoxy)-7-methoxy-6-quinolinecarboxamide;
4-(3-chloro-4-(((cyclopropylamino)carbonyl)amino)phenoxy)-7-((2R)-2-hydroxy-3-(1-pyrrolidino)propoxy)-6-quinolinecarboxamide;
N6-methyl-4-(3-chloro-4-(((methylamino)carbonyl)amino)phenoxy)-7-((2R)-3-diethylamino-2-hydroxypropoxy)-6-quinolinecarboxamide;
N6-methyl-4-(3-chloro-4-(((ethylamino)carbonyl)amino)phenoxy)-7-((2R)-3-diethylamino-2-hydroxypropoxy)-6-quinolinecarboxamide;
N6-methyl-4-(3-chloro-4-(((methylamino)carbonyl)amino)phenoxy)-7-((2R)-2-hydroxy-3-(1-pyrrolidino)propoxy)-6-quinolinecarboxamide;

N6-methyl-4-(3-chloro-4-(((ethylamino) carbonyl) amino) phenoxy)-7-((2R)-2-hydroxy-3-(1-pyrrolidino) propoxy)-6-quinolinecarboxamide;
N6-methyl-4-(3-chloro-4-(((methylamino)carbonyl)amino)phenoxy)-7-((1-methyl-4-piperidyl)methoxy)-6-quinolinecarboxamide;
N6-methyl-4-(3-chloro-4-(((ethylamino)carbonyl)amino)phenoxy)-7-((1-methyl-4-piperidyl)methoxy)-6-quinolinecarboxamide;
N-(4-(6-cyano-7-(2-methoxyethoxy)-4-quinolyl)oxy-2-fluorophenyl)-N'-cyclopropylurea;
N-(4-(6-cyano-7-(3-(4-morpholino)propoxy)-4-quinolyl)oxyphenyl)-N'-(3-(methylsulfonyl)phenyl)urea;
4-(4-((cyclopropylamino)carbonyl)aminophenoxy)-7-methoxy-6-quinolinecarboxamide;
4-(3-fluoro-4-((2-fluoroethylamino)carbonyl)aminophenoxy)-7-methoxy-6-quinolinecarboxamide;
N6-(2-ethoxyethyl)-4-(3-chloro-4-(((methylamino)carbonyl)amino)phenoxy)-7-methoxy-6-quinolinecarboxamide;
4-(4-(3-ethylureido)-3-fluoro-phenoxy)-7-methoxyquinoline-6-carboxylic acid (2-cyanoethyl)amide; and
N-(4-(6-(2-cyanoethyl)carbamoyl-7-methoxy-4-quinolyl)oxy-2-fluorophenyl)-N'-cyclopropylurea,

a pharmacologically acceptable salt thereof or a solvate thereof.

**14.** The pharmaceutical composition according to Claim 1, wherein the compound represented by General Formula (I), a pharmacologically acceptable salt thereof or a solvate thereof is at least one compound selected from the group consisting of:

4-(3-chloro-4-(cyclopropylaminocarbonyl)aminophenoxy)-7-methoxy-6-quinolinecarboxamide;
4-(3-chloro-4-(ethylaminocarbonyl)aminophenoxy)-7-methoxy-6-quinolinecarboxamide;
N6-methoxy-4-(3-chloro-4-(((cyclopropylamino)carbonyl)amino)phenoxy)-7-methoxy-6-quinolinecarboxamide;
4-(3-chloro-4-(methylaminocarbonyl)aminophenoxy)-7-methoxy-6-quinolinecarboxamide; and
N6-methoxy-4-(3-chloro-4-(((ethylamino)carbonyl)amino)phenoxy)-7-methoxy-6-quinolinecarboxamide,

a pharmacologically acceptable salt thereof or a solvate thereof.

**15.** The pharmaceutical composition according to Claim 1, wherein the compound represented by General Formula (I), a pharmacologically acceptable salt thereof or a solvate thereof is 4-(3-chloro-4-(cyclopropylaminocarbonyl)aminophenoxy)-7-methoxy-6-quinolinecarboxamide, a pharmacologically acceptable salt thereof or a solvate thereof.

**16.** The pharmaceutical composition according to Claim 1, wherein the compound represented by General Formula (I), a pharmacologically acceptable salt thereof or a solvate thereof is methanesulfonate of 4-(3-chloro-4-(cyclopropylaminocarbonyl)aminophenoxy)-7-methoxy-6-quinolinecarboxamide.

**17.** The pharmaceutical composition according to Claim 1, wherein gemcitabine, a pharmacologically acceptable salt thereof or a solvate thereof is gemcitabine hydrochloride.

**18.** The pharmaceutical composition according to Claim 1, wherein erlotinib, a pharmacologically acceptable salt thereof or a solvate thereof is erlotinib hydrochloride.

**19.** The pharmaceutical composition according to any one of Claims 1-18, wherein it is a pharmaceutical composition for treating cancer.

**20.** A kit comprising:

(a) at least one selected from the group consisting of a packaging container, an instruction and a package insert describing combination use of a compound represented by General Formula (I), a pharmacologically acceptable salt thereof or a solvate thereof with gemcitabine or erlotinib, a pharmacologically acceptable salt thereof or a solvate thereof; and
(b) a pharmaceutical composition comprising the compound represented by General Formula (I) below, a pharmacologically acceptable salt thereof or a solvate thereof:

General Formula (I)

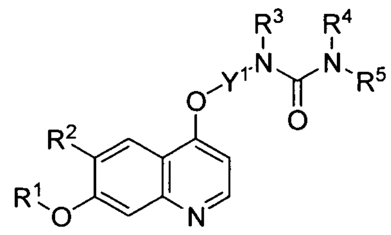

(I)

[wherein, $R^1$ represents a group represented by Formula $-V^1-V^2-V^3$ (wherein, $V^1$ represents an optionally substituted $C_{1-6}$ alkylene group; $V^2$ represents a single bond, an oxygen atom, a sulfur atom, a carbonyl group, a sulfinyl group, a sulfonyl group, a group represented by Formula $-CONR^6-$, a group represented by Formula $-SO_2NR^6-$, a group represented by Formula $-NR^6SO_2-$, a group represented by Formula $-NR^6CO-$ or a group represented by Formula $-NR^6-$ (wherein, $R^6$ represents a hydrogen atom, an optionally substituted $C_{1-6}$ alkyl group or an optionally substituted $C_{3-8}$ cycloalkyl group); $V^3$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group or an optionally substituted 3-10-membered nonaromatic heterocyclic group);

$R^2$ represents a cyano group, an optionally substituted $C_{1-6}$ alkoxy group, a carboxyl group, an optionally substituted $C_{2-7}$ alkoxycarbonyl group or a group represented by Formula $-CONV^{a11}V^{a12}$ (wherein, $V^{a11}$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group or an optionally substituted 3-10-membered nonaromatic heterocyclic group; $V^{a12}$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group, an optionally substituted 3-10-membered nonaromatic heterocyclic group, a hydroxyl group, an optionally substituted $C_{1-6}$ alkoxy group or an optionally substituted $C_{3-8}$ cycloalkoxy group);

$Y^1$ represents a group represented by either one of the following formulae

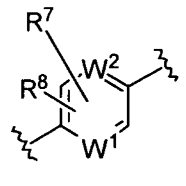

or

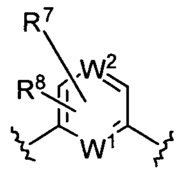

(wherein, $R^7$ and $R^8$ each independently represent a hydrogen atom, a halogen atom, a cyano group, a nitro group, an amino group, an optionally substituted $C_{1-6}$ alkyl group, an optionally substituted $C_{3-8}$ cycloalkyl group, an optionally substituted $C_{1-6}$ alkoxy group, an optionally substituted $C_{1-6}$ alkylthio group, a formyl group, an optionally substituted $C_{2-7}$ acyl group, an optionally substituted $C_{2-7}$ alkoxycarbonyl group or a group represented by Formula $-CONV^{d1}V^{d2}$ (wherein, $V^{d1}$ and $V^{d2}$ each independently represent a hydrogen atom or an optionally substituted $C_{1-6}$ alkyl group);

$W^1$ and $W^2$ each independently represent an optionally substituted carbon atom or nitrogen atom);

$R^3$ and $R^4$ each independently represent a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{2-7}$ acyl group or an optionally substituted $C_{2-7}$ alkoxycarbonyl group; and

$R^5$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group or an optionally substituted 3-10-membered nonaromatic heterocyclic group].

**21.** The kit according to Claim 19, wherein $R^1$ is a $C_{1-6}$ alkyl group (provided that $R^1$ may have a substituent selected from a 3-10-membered nonaromatic heterocyclic group, a hydroxyl group, a $C_{1-6}$ alkoxy group, an amino group, a mono-$C_{1-6}$ alkylamino group and a di-$C_{1-6}$ alkylamino group which may have a $C_{1-6}$ alkyl group).

**22.** The kit according to Claim 20, wherein $R^1$ is a methyl group or a group represented by any one of the following formulae

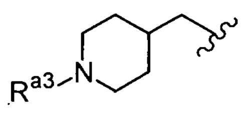 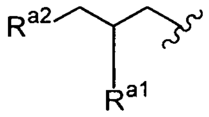 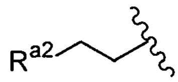

(wherein, $R^{a3}$ represents a methyl group; $R^{a1}$ represents a hydrogen atom or a hydroxyl group; $R^{a2}$ represents a methoxy group, an ethoxy group, a 1-pyrrolidinyl group, a 1-piperidinyl group, a 4-morpholinyl group, a dimethylamino group or a diethylamino group).

**23.** The kit according to Claim 20, wherein $R^1$ is a methyl group or a 2-methoxyethyl group.

**24.** The kit according to Claim 20, wherein $R^2$ is a cyano group or a group represented by Formula -$CONV^{a11}V^{a12}$ (wherein, $V^{a11}$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group or an optionally substituted 3-10-membered nonaromatic heterocyclic group; $V^{a12}$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group, an optionally substituted 3-10-membered nonaromatic heterocyclic group, a hydroxyl group, an optionally substituted $C_{1-6}$ alkoxy group or an optionally substituted $C_{3-8}$ cycloalkoxy group).

**25.** The kit according to Claim 20, wherein $R^2$ is a cyano group or a group represented by Formula -$CONHV^{a16}$ (wherein, $V^{a16}$ represents a hydrogen atom, a $C_{1-6}$ alkyl group, a $C_{3-8}$ cycloalkyl group, a $C_{1-6}$ alkoxy group or a $C_{3-8}$ cycloalkoxy group, provided that $V^{a16}$ may have a substituent selected from a halogen atom, a cyano group, a hydroxyl group and a $C_{1-6}$ alkoxy group).

**26.** The kit according to Claim 20, wherein $R^2$ is a group represented by Formula - $CONHV^{a17}$ (wherein, $V^{a17}$ represents a hydrogen atom, a $C_{1-6}$ alkyl group or a $C_{1-6}$ alkoxy group).

**27.** The kit according to Claim 20, wherein $R^2$ is a group represented by Formula - $CONHV^{a18}$ (wherein, $V^{a18}$ represents a hydrogen atom, a methyl group or a methoxy group).

**28.** The kit according to Claim 20, wherein $Y^1$ is a group represented by the following formula

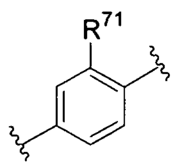

(wherein, $R^{71}$ represents a hydrogen atom or a halogen atom).

**29.** The kit according to Claim 20, wherein $R^3$ and $R^4$ are hydrogen atoms.

**30.** The kit according to Claim 20, wherein $R^5$ is a hydrogen atom, a $C_{1-6}$ alkyl group, a $C_{3-8}$ cycloalkyl group or a $C_{6-10}$ aryl group (provided that $R^5$ may have a substituent selected from a halogen atom and a methanesulfonyl group).

**31.** The kit according to Claim 20, wherein $R^5$ is a methyl group, an ethyl group or a cyclopropyl group.

**32.** The kit according to Claim 20, wherein the compound represented by General Formula (I), a pharmacologically acceptable salt thereof or a solvate thereof is at least one compound selected from the group consisting of:

N-(4-(6-cyano-7-(2-methoxyethoxy)-4-quinolyl)oxy-2-fluorophenyl)-N'-(4-fluorophenyl)urea;
N-(2-chloro-4-((6-cyano-7-((1-methyl-4-piperidyl)methoxy)-4-quinolyl)oxy)phenyl)-N'-cyclopropylurea;
N-(4-((6- cyano- 7-(((2R)- 3-(diethylamino)- 2- hydroxypropyl) oxy)- 4- quinolyl) oxy) phenyl)-N'-(4- fluorophenyl) urea;
N-(4-((6- cyano- 7-(((2R)- 2- hydroxy- 3-(1- pyrrolidino) propyl) oxy)- 4- quinolyl) oxy) phenyl)-N'-(4- fluorophenyl) urea;
4-(3-chloro-4-(cyclopropylaminocarbonyl)aminophenoxy)-7-methoxy-6-quinolinecarboxamide;
4-(3-chloro-4-(cyclopropylaminocarbonyl)aminophenoxy)-7-(2-methoxyethoxy)-6-quinolinecarboxamide;
N6-cyclopropyl-4-(3-chloro-4-(((cyclopropylamino)carbonyl)amino)phenoxy)-7-methoxy-6-quinolinecarboxamide;
N6-(2-methoxyethyl)-4-(3-chloro-4-(((cyclopropylamino)carbonyl)amino)phenoxy)-7-methoxy-6-quinolinecarboxamide;
N6-(2-fluoroethyl)-4-(3-chloro-4-(((cyclopropylamino)carbonyl)amino)phenoxy)-7-methoxy-6-quinolinecarboxamide;
N6-methoxy-4-(3-chloro-4-(((cyclopropylamino)carbonyl)amino)phenoxy)-7-methoxy-6-quinolinecarboxamide;
N6-methyl-4-(3-chloro-4-(((cyclopropylamino)carbonyl)amino)phenoxy)-7-methoxy-6-quinolinecarboxamide;
N6-ethyl-4-(3-chloro-4-(((cyclopropylamino)carbonyl)amino)phenoxy)-7-methoxy-6-quinolinecarboxamide;
4-(3-fluoro-4-(cyclopropylaminocarbonyl)aminophenoxy)-7-(2-methoxyethoxy)-6-quinolinecarboxamide;
4-(3-chloro-4-(cyclopropylaminocarbonyl)aminophenoxy)-7-(2-hydroxyethoxy)-6-quinolinecarboxamide;
4-(3-chloro-4-(cyclopropylaminocarbonyl)aminophenoxy)-7-((2S)-2,3-dihydroxypropyl)oxy-6-quinolinecarboxamide;
4-(3-chloro-4-(methylaminocarbonyl)aminophenoxy)-7-methoxy-6-quinolinecarboxamide;
4-(3-chloro-4-(ethylaminocarbonyl)aminophenoxy)-7-methoxy-6-quinolinecarboxamide;
N6-methoxy-4-(3-chloro-4-(((ethylamino)carbonyl)amino)phenoxy)-7-methoxy-6-quinolinecarboxamide;
4-(3-chloro-4-(cyclopropylaminocarbonyl)aminophenoxy)-7-(2-ethoxyethoxy)-6-quinolinecarboxamide;
4-(4-((cyclopropylamino)carbonyl)aminophenoxy)-7-(2-methoxyethoxy)-6-quinolinecarboxamide;
N-(2-fluoro-4-((6-carbamoyl-7-methoxy-4-quinolyl)oxy)phenyl)-N'-cyclopropylurea;
N6-(2-hydroxyethyl)-4-(3-chloro-4-(((cyclopropylamino)carbonyl)amino)phenoxy)-7-methoxy-6-quinolinecarboxamide;
4-(3-chloro-4-(1-propylaminocarbonyl)aminophenoxy)-7-methoxy-6-quinolinecarboxamide;
4-(3-chloro-4-(cis-2-fluoro-cyclopropylaminocarbonyl)aminophenoxy)-7-methoxy-6-quinolinecarboxamide;
N6-methyl-4-(3-chloro-4-(((cyclopropylamino)carbonyl)amino)phenoxy)-7-(2-methoxyethoxy)-6-quinolinecarboxamide;
N6-methyl-4-(3-chloro-4-(((ethylamino)carbonyl)amino)phenoxy)-7-methoxy-6-quinolinecarboxamide;
4-(3- chloro- 4-(cyclopropylaminocarbonyl) aminophenoxy)- 7-(2-(4- morpholino) ethoxy)- 6- quinolinecarboxamide;
4-(3-chloro-4-(2-fluoroethylaminocarbonyl)aminophenoxy)-7-methoxy-6-quinolinecarboxamide;
N6-((2R) tetrahydro- 2- furanylmethyl)- 4-(3- chloro- 4-(((methylamino) carbonyl) amino) phenoxy)- 7- methoxy- 6-quinolinecarboxamide;
4-(3-fluoro-4-(ethylaminocarbonyl)aminophenoxy)-7-methoxy-6-quinolinecarboxamide;
4-(3-chloro-4-(((cyclopropylamino)carbonyl)amino)phenoxy)-7-((2R)-2-hydroxy-3-(1-pyrrolidino)propoxy)-6-quinolinecarboxamide;
N6- methyl- 4-(3- chloro- 4-(((methylamino) carbonyl) amino) phenoxy)- 7-((2R)- 3- diethylamino- 2- hydroxypropoxy)-6-quinolinecarboxamide;
N6-methyl-4-(3-chloro-4-(((ethylamino)carbonyl)amino)phenoxy)-7-((2R)-3-diethylamino-2-hydroxypropoxy)-6-quinolinecarboxamide;
N6-methyl-4-(3-chloro-4-(((methylamino)carbonyl)amino)phenoxy)-7-((2R)-2-hydroxy-3-(1-pyrrolidino)propoxy)-6-quinolinecarboxamide;
N6- methyl- 4-(3- chloro- 4-(((ethylamino) carbonyl) amino) phenoxy)- 7-((2R)- 2- hydroxy- 3-(1- pyrrolidino) propoxy)-6-quinolinecarboxamide;
N6-methyl-4-(3-chloro-4-(((methylamino)carbonyl)amino)phenoxy)-7-((1-methyl-4-piperidyl)methoxy)-6-quinolinecarboxamide;

N6-methyl-4-(3-chloro-4-(((ethylamino)carbonyl)amino)phenoxy)-7-((1-methyl-4-piperidyl)methoxy)-6-quinolinecarboxamide;
N-(4-(6-cyano-7-(2-methoxyethoxy)-4-quinolyl)oxy-2-fluorophenyl)-N'-cyclopropylurea;
N-(4-(6-cyano-7-(3-(4-morpholino)propoxy)-4-quinolyl)oxyphenyl)-N'-(3-(methylsulfonyl)phenyl)urea;
4-(4-((cyclopropylamino)carbonyl)aminophenoxy)-7-methoxy-6-quinolinecarboxamide;
4-(3-fluoro-4-((2-fluoroethylamino)carbonyl)aminophenoxy)-7-methoxy-6-quinolinecarboxamide;
N6-(2-ethoxyethyl)-4-(3-chloro-4-(((methylamino)carbonyl)amino)phenoxy)-7-methoxy-6-quinolinecarboxamide;
4-(4-(3-ethylureido)-3-fluoro-phenoxy)-7-methoxyquinoline-6-carboxylic acid (2-cyanoethyl)amide; and
N-(4-(6-(2-cyanoethyl)carbamoyl-7-methoxy-4-quinolyl)oxy-2-fluorophenyl)-N'-cyclopropylurea,

a pharmacologically acceptable salt thereof or a solvate thereof.

**33.** The kit according to Claim 20, wherein the compound represented by General Formula (I), a pharmacologically acceptable salt thereof or a solvate thereof is at least one compound selected from the group consisting of:

4-(3-chloro-4-(cyclopropylaminocarbonyl)aminophenoxy)-7-methoxy-6-quinolinecarboxamide;
4-(3-chloro-4-(ethylaminocarbonyl)aminophenoxy)-7-methoxy-6-quinolinecarboxamide;
N6-methoxy-4-(3-chloro-4-(((cyclopropylamino)carbonyl)amino)phenoxy)-7-methoxy-6-quinolinecarboxamide;
4-(3-chloro-4-(methylaminocarbonyl)aminophenoxy)-7-methoxy-6-quinolinecarboxamide; and
N6-methoxy-4-(3-chloro-4-(((ethylamino)carbonyl)amino)phenoxy)-7-methoxy-6-quinolinecarboxamide,

a pharmacologically acceptable salt thereof or a solvate thereof.

**34.** The kit according to Claim 20, wherein the compound represented by General Formula (I), a pharmacologically acceptable salt thereof or a solvate thereof is 4-(3-chloro-4-(cyclopropylaminocarbonyl)aminophenoxy)-7-methoxy-6-quinolinecarboxamide, a pharmacologically acceptable salt thereof or a solvate thereof.

**35.** The kit according to Claim 20, wherein the compound represented by General Formula (I), a pharmacologically acceptable salt thereof or a solvate thereof is methanesulfonate of 4-(3-chloro-4-(cyclopropylaminocarbonyl)aminophenoxy)-7-methoxy-6-quinolinecarboxamide.

**36.** The kit according to Claim 20, wherein gemcitabine, a pharmacologically acceptable salt thereof or a solvate thereof is gemcitabine hydrochloride.

**37.** The kit according to Claim 20, wherein erlotinib, a pharmacologically acceptable salt thereof or a solvate thereof is erlotinib hydrochloride.

**38.** The kit according to any one of Claims 20-37, wherein it is a kit for treating cancer.

**39.** A kit **characterized by** a set of:

(I) a formulation containing a compound represented by General Formula (I), a pharmacologically acceptable salt thereof or a solvate thereof; and
(II) a formulation containing gemcitabine or erlotinib, a pharmacologically acceptable salt thereof or a solvate thereof:

General Formula (I)

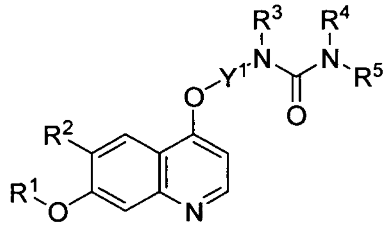

(I)

[wherein, $R^1$ represents a group represented by Formula $-V^1-V^2-V^3$ (wherein, $V^1$ represents an optionally substituted $C_{1-6}$ alkylene group; $V^2$ represents a single bond, an oxygen atom, a sulfur atom, a carbonyl group, a sulfinyl group, a sulfonyl group, a group represented by Formula $-CONR^6-$, a group represented by Formula $-SO_2NR^6-$, a group represented by Formula $-NR^6SO_2-$, a group represented by Formula $-NR^6CO-$ or a group represented by Formula $-NR^6-$ (wherein, $R^6$ represents a hydrogen atom, an optionally substituted $C_{1-6}$ alkyl group or an optionally substituted $C_{3-8}$ cycloalkyl group); $V^3$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group or an optionally substituted 3-10-membered nonaromatic heterocyclic group);

$R^2$ represents a cyano group, an optionally substituted $C_{1-6}$ alkoxy group, a carboxyl group, an optionally substituted $C_{2-7}$ alkoxycarbonyl group or a group represented by Formula $-CONV^{a11}V^{a12}$ (wherein, $V^{a11}$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group or an optionally substituted 3-10-membered nonaromatic heterocyclic group; $V^{a12}$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group, an optionally substituted 3-10-membered nonaromatic heterocyclic group, a hydroxyl group, an optionally substituted $C_{1-6}$ alkoxy group or an optionally substituted $C_{3-8}$ cycloalkoxy group);

$Y^1$ represents a group represented by either one of the following formulae

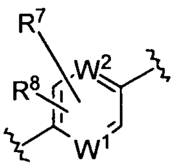

or

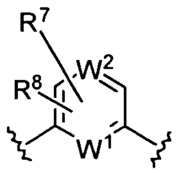

(wherein, $R^7$ and R each independently represent a hydrogen atom, a halogen atom, a cyano group, a nitro group, an amino group, an optionally substituted $C_{1-6}$ alkyl group, an optionally substituted $C_{3-8}$ cycloalkyl group, an optionally substituted $C_{1-6}$ alkoxy group, an optionally substituted $C_{1-6}$ alkylthio group, a formyl group, an optionally substituted $C_{2-7}$ acyl group, an optionally substituted $C_{2-7}$ alkoxycarbonyl group or a group represented by Formula $-CONV^{d1}V^{d2}$ (wherein, $V^{d1}$ and $V^{d2}$ each independently represent a hydrogen atom or an optionally substituted $C_{1-6}$ alkyl group);

$W^1$ and $W^2$ each independently represent an optionally substituted carbon atom or nitrogen atom);

$R^3$ and $R^4$ each independently represent a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{2-7}$ acyl group or an optionally substituted $C_{2-7}$ alkoxycarbonyl group; and

$R^5$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group or an optionally substituted 3-10-membered nonaromatic heterocyclic group].

**40.** The kit according to Claim 39, wherein $R^1$ is a $C_{1-6}$ alkyl group (provided that $R^1$ may have a substituent selected from a 3-10-membered nonaromatic heterocyclic group, a hydroxyl group, a $C_{1-6}$ alkoxy group, an amino group, a mono-$C_{1-6}$ alkylamino group and a di-$C_{1-6}$ alkylamino group which may have a $C_{1-6}$ alkyl group).

**41.** The kit according to Claim 39, wherein $R^1$ is a methyl group or a group represented by any one of the following formulae

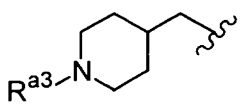 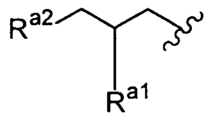 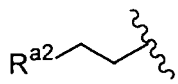

(wherein, $R^{a3}$ represents a methyl group; $R^{a1}$ represents a hydrogen atom or a hydroxyl group; $R^{a2}$ represents a methoxy group, an ethoxy group, a 1-pyrrolidinyl group, a 1-piperidinyl group, a 4-morpholinyl group, a dimethylamino group or a diethylamino group).

**42.** The kit according to Claim 39, wherein $R^1$ is a methyl group or a 2-methoxyethyl group.

**43.** The kit according to Claim 39, wherein $R^2$ is a cyano group or a group represented by Formula -CONV$^{a11}$V$^{a12}$ (wherein, $V^{a11}$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group or an optionally substituted 3-10-membered nonaromatic heterocyclic group; $V^{a12}$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group, an optionally substituted 3-10-membered nonaromatic heterocyclic group, a hydroxyl group, an optionally substituted $C_{1-6}$ alkoxy group or an optionally substituted $C_{3-8}$ cycloalkoxy group).

**44.** The kit according to Claim 39, wherein $R^2$ is a cyano group or a group represented by Formula -CONHV$^{a16}$ (wherein, $V^{a16}$ represents a hydrogen atom, a $C_{1-6}$ alkyl group, a $C_{3-8}$ cycloalkyl group, a $C_{1-6}$ alkoxy group or a $C_{3-8}$ cycloalkoxy group, provided that $V^{a16}$ may have a substituent selected from a halogen atom, a cyano group, a hydroxyl group and a $C_{1-6}$ alkoxy group).

**45.** The kit according to Claim 39, wherein $R^2$ is a group represented by Formula - CONHV$^{a17}$ (wherein, $V^{a17}$ represents a hydrogen atom, a $C_{1-6}$ alkyl group or a $C_{1-6}$ alkoxy group).

**46.** The kit according to Claim 39, wherein $R^2$ is a group represented by Formula - CONHV$^{a18}$ (wherein, $V^{a18}$ represents a hydrogen atom, a methyl group or a methoxy group).

**47.** The kit according to Claim 39, wherein $Y^1$ is a group represented by the following formula

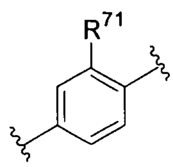

(wherein, $R^{71}$ represents a hydrogen atom or a halogen atom).

**48.** The kit according to Claim 39, wherein $R^3$ and $R^4$ are hydrogen atoms.

**49.** The kit according to Claim 39, wherein $R^5$ is a hydrogen atom, a $C_{1-6}$ alkyl group, a $C_{3-8}$ cycloalkyl group or a $C_{6-10}$ aryl group (provided that $R^5$ may have a substituent selected from a halogen atom and a methanesulfonyl group).

**50.** The kit according to Claim 39, wherein $R^5$ is a methyl group, an ethyl group or a cyclopropyl group.

**51.** The kit according to Claim 39, wherein the compound represented by General Formula (I), a pharmacologically acceptable salt thereof or a solvate thereof is at least one compound selected from the group consisting of:

N-(4-(6-cyano-7-(2-methoxyethoxy)-4-quinolyl)oxy-2-fluorophenyl)-N'-(4-fluorophenyl)urea;
N-(2-chloro-4-((6-cyano-7-((1-methyl-4-piperidyl)methoxy)-4-quinolyl)oxy)phenyl)-N'-cyclopropylurea;
N-(4-((6- cyano- 7-(((2R)- 3-(diethylamino)- 2- hydroxypropyl) oxy)- 4- quinolyl) oxy) phenyl)-N'-(4- fluorophenyl) urea;
N-(4-((6- cyano- 7-(((2R)- 2- hydroxy- 3-(1- pyrrolidino) propyl) oxy)- 4- quinolyl) oxy) phenyl)-N'-(4- fluorophenyl) urea;
4-(3-chloro-4-(cyclopropylaminocarbonyl)aminophenoxy)-7-methoxy-6-quinolinecarboxamide;
4-(3-chloro-4-(cyclopropylaminocarbonyl)aminophenoxy)-7-(2-methoxyethoxy)-6-quinolinecarboxamide;
N6-cyclopropyl-4-(3-chloro-4-(((cyclopropylamino)carbonyl)amino)phenoxy)-7-methoxy-6-quinolinecarboxamide;
N6-(2-methoxyethyl)-4-(3-chloro-4-(((cyclopropylamino)carbonyl)amino)phenoxy)-7-methoxy-6-quinolinecarboxamide;
N6-(2-fluoroethyl)-4-(3-chloro-4-(((cyclopropylamino)carbonyl)amino)phenoxy)-7-methoxy-6-quinolinecarboxamide;
N6-methoxy-4-(3-chloro-4-(((cyclopropylamino)carbonyl)amino)phenoxy)-7-methoxy-6-quinolinecarboxamide;
N6-methyl-4-(3-chloro-4-(((cyclopropylamino)carbonyl)amino)phenoxy)-7-methoxy-6-quinolinecarboxamide;
N6-ethyl-4-(3-chloro-4-(((cyclopropylamino)carbonyl)amino)phenoxy)-7-methoxy-6-quinolinecarboxamide;
4-(3-fluoro-4-(cyclopropylaminocarbonyl)aminophenoxy)-7-(2-methoxyethoxy)-6-quinolinecarboxamide;
4-(3-chloro-4-(cyclopropylaminocarbonyl)aminophenoxy)-7-(2-hydroxyethoxy)-6-quinolinecarboxamide;
4-(3-chloro-4-(cyclopropylaminocarbonyl)aminophenoxy)-7-((2S)-2,3-dihydroxypropyl)oxy-6-quinolinecarboxamide;
4-(3-chloro-4-(methylaminocarbonyl)aminophenoxy)-7-methoxy-6-quinolinecarboxamide;
4-(3-chloro-4-(ethylaminocarbonyl)aminophenoxy)-7-methoxy-6-quinolinecarboxamide;
N6-methoxy-4-(3-chloro-4-(((ethylamino)carbonyl)amino)phenoxy)-7-methoxy-6-quinolinecarboxamide;
4-(3-chloro-4-(cyclopropylaminocarbonyl)aminophenoxy)-7-(2-ethoxyethoxy)-6-quinolinecarboxamide;
4-(4-((cyclopropylamino)carbonyl)aminophenoxy)-7-(2-methoxyethoxy)-6-quinolinecarboxamide;
N-(2-fluoro-4-((6-carbamoyl-7-methoxy-4-quinolyl)oxy)phenyl)-N'-cyclopropylurea;
N6-(2-hydroxyethyl)-4-(3-chloro-4-(((cyclopropylamino)carbonyl)amino)phenoxy)-7-methoxy-6-quinolinecarboxamide;
4-(3-chloro-4-(1-propylaminocarbonyl)aminophenoxy)-7-methoxy-6-quinolinecarboxamide;
4-(3-chloro-4-(cis-2-fluoro-cyclopropylaminocarbonyl)aminophenoxy)-7-methoxy-6-quinolinecarboxamide;
N6-methyl-4-(3-chloro-4-(((cyclopropylamino)carbonyl)amino)phenoxy)-7-(2-methoxyethoxy)-6-quinolinecarboxamide;
N6-methyl-4-(3-chloro-4-(((ethylamino)carbonyl)amino)phenoxy)-7-methoxy-6-quinolinecarboxamide;
4-(3-chloro-4-(cyclopropylaminocarbonyl)aminophenoxy)-7-(2-(4-morpholino)ethoxy)-6-quinolinecarboxamide;
4-(3-chloro-4-(2-fluoroethylaminocarbonyl)aminophenoxy)-7-methoxy-6-quinolinecarboxamide;
N6-((2R) tetrahydro-2-furanylmethyl)-4-(3-chloro-4-(((methylamino)carbonyl)amino)phenoxy)-7-methoxy-6-quinolinecarboxamide;
4-(3-fluoro-4-(ethylaminocarbonyl)aminophenoxy)-7-methoxy-6-quinolinecarboxamide;
4-(3-chloro-4-(((cyclopropylamino)carbonyl)amino)phenoxy)-7-((2R)-2-hydroxy-3-(1-pyrrolidino)propoxy)-6-quinolinecarboxamide;
N6-methyl-4-(3-chloro-4-(((methylamino)carbonyl)amino)phenoxy)-7-((2R)-3-diethylamino-2-hydroxypropoxy)-6-quinolinecarboxamide;
N6-methyl-4-(3-chloro-4-(((ethylamino)carbonyl)amino)phenoxy)-7-((2R)-3-diethylamino-2-hydroxypropoxy)-6-quinolinecarboxamide;
N6-methyl-4-(3-chloro-4-(((methylamino)carbonyl)amino)phenoxy)-7-((2R)-2-hydroxy-3-(1-pyrrolidino)propoxy)-6-quinolinecarboxamide;
N6-methyl-4-(3-chloro-4-(((ethylamino)carbonyl)amino)phenoxy)-7-((2R)-2-hydroxy-3-(1-pyffolidino)propoxy)-6-quinolinecarboxamide;
N6-methyl-4-(3-chloro-4-(((methylamino)carbonyl)amino)phenoxy)-7-((1-methyl-4-piperidyl)methoxy)-6-quinolinecarboxamide;
N6-methyl-4-(3-chloro-4-(((ethylamino)carbonyl)amino)phenoxy)-7-((1-methyl-4-piperidyl)methoxy)-6-quinolinecarboxamide;

N-(4-(6-cyano-7-(2-methoxyethoxy)-4-quinolyl)oxy-2-fluorophenyl)-N'-cyclopropylurea;
N-(4-(6-cyano-7-(3-(4-morpholino)propoxy)-4-quinolyl)oxyphenyl)-N'-(3-(methylsulfonyl)phenyl)urea;
4-(4-((cyclopropylamino)carbonyl)aminophenoxy)-7-methoxy-6-quinolinecarboxamide;
4-(3-fluoro-4-((2-fluoroethylamino)carbonyl)aminophenoxy)-7-methoxy-6-quinolinecarboxamide;
N6-(2-ethoxyethyl)-4-(3-chloro-4-(((methylamino)carbonyl)amino)phenoxy)-7-methoxy-6-quinolinecarboxamide;
4-(4-(3-ethylureido)-3-fluoro-phenoxy)-7-methoxyquinoline-6-carboxylic acid (2-cyanoethyl)amide; and
N-(4-(6-(2-cyanoethyl)carbamoyl-7-methoxy-4-quinolyl)oxy-2-fluorophenyl)-N'-cyclopropylurea,

a pharmacologically acceptable salt thereof or a solvate thereof.

**52.** The kit according to Claim 39, wherein the compound represented by General Formula (I), a pharmacologically acceptable salt thereof or a solvate thereof is at least one compound selected from the group consisting of:

4-(3-chloro-4-(cyclopropylaminocarbonyl)aminophenoxy)-7-methoxy-6-quinolinecarboxamide;
4-(3-chloro-4-(ethylaminocarbonyl)aminophenoxy)-7-methoxy-6-quinolinecarboxamide;
N6-methoxy-4-(3-chloro-4-(((cyclopropylamino)carbonyl)amino)phenoxy)-7-methoxy-6-quinolinecarboxamide;
4-(3-chloro-4-(methylaminocarbonyl)aminophenoxy)-7-methoxy-6-quinolinecarboxamide; and
N6-methoxy-4-(3-chloro-4-(((ethylamino)carbonyl)amino)phenoxy)-7-methoxy-6-quinolinecarboxamide,

a pharmacologically acceptable salt thereof or a solvate thereof.

**53.** The kit according to Claim 39, wherein the compound represented by General Formula (I), a pharmacologically acceptable salt thereof or a solvate thereof is 4-(3-chloro-4-(cyclopropylaminocarbonyl)aminophenoxy)-7-methoxy-6-quinolinecarboxamide, a pharmacologically acceptable salt thereof or a solvate thereof.

**54.** The kit according to Claim 39, wherein the compound represented by General Formula (I), a pharmacologically acceptable salt thereof or a solvate thereof is methanesulfonate of 4-(3-chloro-4-(cyclopropylaminocarbonyl)aminophenoxy)-7-methoxy-6-quinolinecarboxamide.

**55.** The kit according to Claim 39, wherein gemcitabine, a pharmacologically acceptable salt thereof or a solvate thereof is gemcitabine hydrochloride.

**56.** The kit according to Claim 39, wherein erlotinib, a pharmacologically acceptable salt thereof or a solvate thereof is erlotinib hydrochloride.

**57.** The kit according to any one of Claims 39-56, wherein it is a kit for treating cancer.

**58.** A pharmaceutical composition comprising a compound represented by General Formula (I), a pharmacologically acceptable salt thereof or a solvate thereof that is simultaneously or separately administered to a patient with gemcitabine or erlotinib, a pharmacologically acceptable salt thereof or a solvate thereof:

General Formula (I)

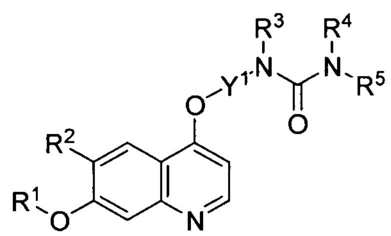

(I)

[wherein, $R^1$ represents a group represented by Formula $-V^1-V^2-V^3$ (wherein, $V^1$ represents an optionally substituted $C_{1-6}$ alkylene group; $V^2$ represents a single bond, an oxygen atom, a sulfur atom, a carbonyl group, a sulfinyl group, a sulfonyl group, a group represented by Formula $-CONR^6-$, a group represented by Formula $-SO_2NR^6-$, a

group represented by Formula -$NR^6SO_2$-, a group represented by Formula -$NR^6CO$- or a group represented by Formula -$NR^6$- (wherein, $R^6$ represents a hydrogen atom, an optionally substituted $C_{1-6}$ alkyl group or an optionally substituted $C_{3-8}$ cycloalkyl group); $V^3$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group or an optionally substituted 3-10-membered nonaromatic heterocyclic group);

$R^2$ represents a cyano group, an optionally substituted $C_{1-6}$ alkoxy group, a carboxyl group, an optionally substituted $C_{2-7}$ alkoxycarbonyl group or a group represented by Formula -$CONV^{a11}V^{a12}$ (wherein, $V^{a11}$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group or an optionally substituted 3-10-membered nonaromatic heterocyclic group; $V^{a12}$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group, an optionally substituted 3-10-membered nonaromatic heterocyclic group, a hydroxyl group, an optionally substituted $C_{1-6}$ alkoxy group or an optionally substituted $C_{3-8}$ cycloalkoxy group);

$Y^1$ represents a group represented by either one of the following formulae

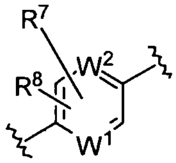

or

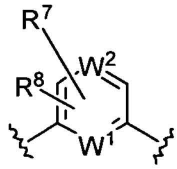

(wherein, $R^7$ and $R^8$ each independently represent a hydrogen atom, a halogen atom, a cyano group, a nitro group, an amino group, an optionally substituted $C_{1-6}$ alkyl group, an optionally substituted $C_{3-8}$ cycloalkyl group, an optionally substituted $C_{1-6}$ alkoxy group, an optionally substituted $C_{1-6}$ alkylthio group, a formyl group, an optionally substituted $C_{2-7}$ acyl group, an optionally substituted $C_{2-7}$ alkoxycarbonyl group or a group represented by Formula -$CONV^{d1}V^{d2}$ (wherein, $V^{d1}$ and $V^{d2}$ each independently represent a hydrogen atom or an optionally substituted $C_{1-6}$ alkyl group);

$W^1$ and $W^2$ each independently represent an optionally substituted carbon atom or nitrogen atom);

$R^3$ and $R^4$ each independently represent a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{2-7}$ acyl group or an optionally substituted $C_{2-7}$ alkoxycarbonyl group; and

$R^5$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group or an optionally substituted 3-10-membered nonaromatic heterocyclic group].

**59.** The pharmaceutical composition according to Claim 58, wherein $R^1$ is a $C_{1-6}$ alkyl group (provided that $R^1$ may have a substituent selected from a 3-10-membered nonaromatic heterocyclic group, a hydroxyl group, a $C_{1-6}$ alkoxy group, an amino group, a mono-$C_{1-6}$ alkylamino group and a di-$C_{1-6}$ alkylamino group which may have a $C_{1-6}$ alkyl group).

**60.** The therapeutic agent according to Claim 58, wherein $R^1$ is a methyl group or a group represented by any one of the following formulae

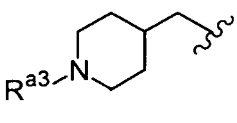

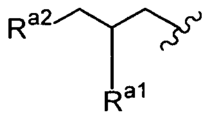

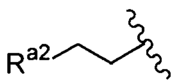

(wherein, $R^{a3}$ represents a methyl group; $R^{a1}$ represents a hydrogen atom or a hydroxyl group; $R^{a2}$ represents a methoxy group, an ethoxy group, a 1-pyrrolidinyl group, a 1-piperidinyl group, a 4-morpholinyl group, a dimethylamino group or a diethylamino group).

**61.** The pharmaceutical composition according to Claim 58, wherein $R^1$ is a methyl group or a 2-methoxyethyl group.

**62.** The pharmaceutical composition according to Claim 58, wherein $R^2$ is a cyano group or a group represented by Formula $-CONV^{a11}V^{a12}$ (wherein, $V^{a11}$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group or an optionally substituted 3-10-membered nonaromatic heterocyclic group; $V^{a12}$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group, an optionally substituted 3-10-membered nonaromatic heterocyclic group, a hydroxyl group, an optionally substituted $C_{1-6}$ alkoxy group or an optionally substituted $C_{3-8}$ cycloalkoxy group).

**63.** The pharmaceutical composition according to Claim 58, wherein $R^2$ is a cyano group or a group represented by Formula $-CONHV^{a16}$ (wherein, $V^{a16}$ represents a hydrogen atom, a $C_{1-6}$ alkyl group, a $C_{3-8}$ cycloalkyl group, a $C_{1-6}$ alkoxy group or a $C_{3-8}$ cycloalkoxy group, provided that $V^{a16}$ may have a substituent selected from a halogen atom, a cyano group, a hydroxyl group and a $C_{1-6}$ alkoxy group).

**64.** The pharmaceutical composition according to Claim 58, wherein $R^2$ is a group represented by Formula $-CONHV^{a17}$ (wherein, $V^{a17}$ represents a hydrogen atom, a $C_{1-6}$ alkyl group or a $C_{1-6}$ alkoxy group).

**65.** The pharmaceutical composition according to Claim 58, wherein $R^2$ is a group represented by Formula $-CONHV^{a18}$ (wherein, $V^{a18}$ represents a hydrogen atom, a methyl group or a methoxy group).

**66.** The pharmaceutical composition according to Claim 58, wherein $Y^1$ is a group represented by the following formula

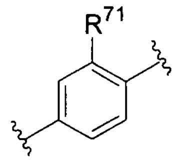

(wherein, $R^{71}$ represents a hydrogen atom or a halogen atom).

**67.** The pharmaceutical composition according to Claim 58, wherein $R^3$ and $R^4$ are hydrogen atoms.

**68.** The pharmaceutical composition according to Claim 58, wherein $R^5$ is a hydrogen atom, a $C_{1-6}$ alkyl group, a $C_{3-8}$ cycloalkyl group or a $C_{6-10}$ aryl group (provided that $R^5$ may have a substituent selected from a halogen atom and a methanesulfonyl group).

**69.** The pharmaceutical composition according to Claim 58, wherein $R^5$ is a methyl group, an ethyl group or a cyclopropyl group.

**70.** The pharmaceutical composition according to Claim 58, wherein the compound represented by General Formula (I), a pharmacologically acceptable salt thereof or a solvate thereof is at least one compound selected from the group consisting of:

N-(4-(6-cyano-7-(2-methoxyethoxy)-4-quinolyl)oxy-2-fluorophenyl)-N' -(4-fluorophenyl)urea;
N-(2-chloro-4-((6-cyano-7-((1-methyl-4-piperidyl)methoxy)-4-quinolyl)oxy)phenyl)-N'-cyclopropylurea;
N-(4-((6- cyano- 7-(((2R)- 3-(diethylamino)- 2- hydroxypropyl) oxy)- 4- quinolyl) oxy) phenyl)-N'-(4- fluorophenyl) urea;
N-(4-((6- cyano- 7-(((2R)- 2- hydroxy- 3-(1- pyrrolidino) propyl) oxy)- 4- quinolyl) oxy) phenyl)-N'-(4- fluorophenyl) urea;
4-(3-chloro-4-(cyclopropylaminocarbonyl)aminophenoxy)-7-methoxy-6-quinolinecarboxamide;
4-(3-chloro-4-(cyclopropylaminocarbonyl)aminophenoxy)-7-(2-methoxyethoxy)-6-quinolinecarboxamide;
N6-cyclopropyl-4-(3-chloro-4-(((cyclopropylamino)carbonyl)amino)phenoxy)-7-methoxy-6-quinolinecarboxamide;
N6-(2-methoxyethyl)-4-(3-chloro-4-(((cyclopropylamino)carbonyl)amino)phenoxy)-7-methoxy-6-quinolinecarboxamide;
N6-(2-fluoroethyl)-4-(3-chloro-4-(((cyclopropylamino)carbonyl)amino)phenoxy)-7-methoxy-6-quinolinecarboxamide;
N6-methoxy-4-(3-chloro-4-(((cyclopropylamino)carbonyl)amino)phenoxy)-7-methoxy-6-quinolinecarboxamide;
N6-methyl-4-(3-chloro-4-(((cyclopropylamino)carbonyl)amino)phenoxy)-7-methoxy-6-quinolinecarboxamide;
N6-ethyl-4-(3-chloro-4-(((cyclopropylamino)carbonyl)amino)phenoxy)-7-methoxy-6-quinolinecarboxamide;
4-(3-fluoro-4-(cyclopropylaminocarbonyl)aminophenoxy)-7-(2-methoxyethoxy)-6-quinolinecarboxamide;
4-(3-chloro-4-(cyclopropylaminocarbonyl)aminophenoxy)-7-(2-hydroxyethoxy)-6-quinolinecarboxamide;
4-(3-chloro-4-(cyclopropylaminocarbonyl)aminophenoxy)-7-((2S)-2,3-dihydroxypropyl)oxy-6-quinolinecarboxamide;
4-(3-chloro-4-(methylaminocarbonyl)aminophenoxy)-7-methoxy-6-quinolinecarboxamide;
4-(3-chloro-4-(ethylaminocarbonyl)aminophenoxy)-7-methoxy-6-quinolinecarboxamide;
N6-methoxy-4-(3-chloro-4-(((ethylamino)carbonyl)amino)phenoxy)-7-methoxy-6-quinolinecarboxamide;
4-(3-chloro-4-(cyclopropylaminocarbonyl)aminophenoxy)-7-(2-ethoxyethoxy)-6-quinolinecarboxamide;
4-(4-((cyclopropylamino)carbonyl)aminophenoxy)-7-(2-methoxyethoxy)-6-quinolinecarboxamide;
N-(2-fluoro-4-((6-carbamoyl-7-methoxy-4-quinolyl)oxy)phenyl)-N'-cyclopropylurea;
N6-(2-hydroxyethyl)-4-(3-chloro-4-(((cyclopropylamino)carbonyl)amino)phenoxy)-7-methoxy-6-quinolinecarboxamide;
4-(3-chloro-4-(1-propylaminocarbonyl)aminophenoxy)-7-methoxy-6-quinolinecarboxamide;
4-(3-chloro-4-(cis-2-fluoro-cyclopropylaminocarbonyl)aminophenoxy)-7-methoxy-6-quinolinecarboxamide;
N6-methyl-4-(3-chloro-4-(((cyclopropylamino)carbonyl)amino)phenoxy)-7-(2-methoxyethoxy)-6-quinolinecarboxamide;
N6-methyl-4-(3-chloro-4-(((ethylamino)carbonyl)amino)phenoxy)-7-methoxy-6-quinolinecarboxamide;
4-(3-chloro-4-(cyclopropylaminocarbonyl)aminophenoxy)-7-(2-(4-morpholino)ethoxy)-6-quinolinecarboxamide;
4-(3-chloro-4-(2-fluoroethylaminocarbonyl)aminophenoxy)-7-methoxy-6-quinolinecarboxamide;
N6-((2R)tetrahydro-2-furanylmethyl)-4-(3-chloro-4-(((methylamino)carbonyl)amino)phenoxy)-7-methoxy-6-quinolinecarboxamide;
4-(3-fluoro-4-(ethylaminocarbonyl)aminophenoxy)-7-methoxy-6-quinolinecarboxamide;
4-(3-chloro-4-(((cyclopropylamino)carbonyl)amino)phenoxy)-7-((2R)-2-hydroxy-3-(1-pyrrolidino)propoxy)-6-quinolinecarboxamide;
N6-methyl-4-(3-chloro-4-(((methylamino)carbonyl)amino)phenoxy)-7-((2R)-3-diethylamino-2-hydroxypropoxy)-6-quinolinecarboxamide;
N6-methyl-4-(3-chloro-4-(((ethylamino)carbonyl)amino)phenoxy)-7-((2R)-3-diethylamino-2-hydroxypropoxy)-6-quinolinecarboxamide;
N6-methyl-4-(3-chloro-4-(((methylamino)carbonyl)amino)phenoxy)-7-((2R)-2-hydroxy-3-(1-pyrrolidino)propoxy)-6-quinolinecarboxamide;
N6-methyl-4-(3-chloro-4-(((ethylamino)carbonyl)amino)phenoxy)-7-((2R)-2-hydroxy-3-(1-pyrrolidino)propoxy)-6-quinolinecarboxamide;
N6-methyl-4-(3-chloro-4-(((methylamino)carbonyl)amino)phenoxy)-7-((1-methyl-4-piperidyl)methoxy)-6-quinolinecarboxamide;
N6-methyl-4-(3-chloro-4-(((ethylamino)carbonyl)amino)phenoxy)-7-((1-methyl-4-piperidyl)methoxy)-6-quinolinecarboxamide;
N-(4-(6-cyano-7-(2-methoxyethoxy)-4-quinolyl)oxy-2-fluorophenyl)-N'-cyclopropylurea;
N-(4-(6-cyano-7-(3-(4-morpholino)propoxy)-4-quinolyl)oxyphenyl)-N'-(3-(methylsulfonyl)phenyl)urea;
4-(4-((cyclopropylamino)carbonyl)aminophenoxy)-7-methoxy-6-quinolinecarboxamide;
4-(3-fluoro-4-((2-fluoroethylamino)carbonyl)aminophenoxy)-7-methoxy-6-quinolinecarboxamide;
N6-(2-ethoxyethyl)-4-(3-chloro-4-(((methylamino)carbonyl)amino)phenoxy)-7-methoxy-6-quinolinecarboxamide;
4-(4-(3-ethylureido)-3-fluoro-phenoxy)-7-methoxyquinoline-6-carboxylic acid (2-cyanoethyl)amide; and
N-(4-(6-(2-cyanoethyl)carbamoyl-7-methoxy-4-quinolyl)oxy-2-fluorophenyl)-N'-cyclopropylurea,

a pharmacologically acceptable salt thereof or a solvate thereof.

**71.** The pharmaceutical composition according to Claim 58, wherein the compound represented by General Formula

(I), a pharmacologically acceptable salt thereof or a solvate thereof is at least one compound selected from the group consisting of:

4-(3-chloro-4-(cyclopropylaminocarbonyl)aminophenoxy)-7-methoxy-6-quinolinecarboxamide;
4-(3-chloro-4-(ethylaminocarbonyl)aminophenoxy)-7-methoxy-6-quinolinecarboxamide;
N6-methoxy-4-(3-chloro-4-(((cyclopropylamino)carbonyl)amino)phenoxy)-7-methoxy-6-quinolinecarboxamide;
4-(3-chloro-4-(methylaminocarbonyl)aminophenoxy)-7-methoxy-6-quinolinecarboxamide; and
N6-methoxy-4-(3-chloro-4-(((ethylamino)carbonyl)amino)phenoxy)-7-methoxy-6-quinolinecarboxamide,

a pharmacologically acceptable salt thereof or a solvate thereof.

**72.** The pharmaceutical composition according to Claim 58, wherein the compound represented by General Formula (I), a pharmacologically acceptable salt thereof or a solvate thereof is 4-(3-chloro-4-(cyclopropylaminocarbonyl) aminophenoxy)-7-methoxy-6-quinolinecarboxamide, a pharmacologically acceptable salt thereof or a solvate thereof.

**73.** The pharmaceutical composition according to Claim 58, wherein the compound represented by General Formula (I), a pharmacologically acceptable salt thereof or a solvate thereof is methanesulfonate of 4-(3-chloro-4-(cyclopropylaminocarbonyl)aminophenoxy)-7-methoxy-6-quinolinecarboxamide.

**74.** The pharmaceutical composition according to Claim 58, wherein gemcitabine, a pharmacologically acceptable salt thereof or a solvate thereof is gemcitabine hydrochloride.

**75.** The pharmaceutical composition according to Claim 58, wherein erlotinib, a pharmacologically acceptable salt thereof or a solvate thereof is erlotinib hydrochloride.

**76.** The pharmaceutical composition according to any one of Claims 58-75, wherein it is a pharmaceutical composition for treating cancer.

**77.** A method for treating cancer **characterized by** administering effective dosages of (i) a compound represented by General Formula (I), a pharmacologically acceptable salt thereof or a solvate thereof and (ii) gemcitabine or erlotinib, a pharmacologically acceptable salt thereof or a solvate thereof to a patient:

General Formula (I)

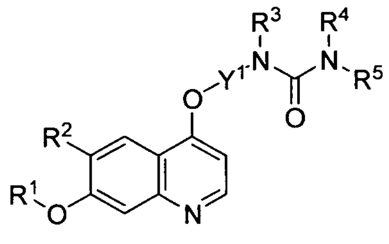

(I)

[wherein, $R^1$ represents a group represented by Formula $-V^1-V^2-V^3$ (wherein, $V^1$ represents an optionally substituted $C_{1-6}$ alkylene group; $V^2$ represents a single bond, an oxygen atom, a sulfur atom, a carbonyl group, a sulfinyl group, a sulfonyl group, a group represented by Formula $-CONR^6-$, a group represented by Formula $-SO_2NR6-$, a group represented by Formula $-NR^6SO_2-$, a group represented by Formula $-NR^6CO-$ or a group represented by Formula $-NR^6-$ (wherein, $R^6$ represents a hydrogen atom, an optionally substituted $C_{1-6}$ alkyl group or an optionally substituted $C_{3-8}$ cycloalkyl group); $V^3$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group or an optionally substituted 3-10-membered nonaromatic heterocyclic group);
$R^2$ represents a cyano group, an optionally substituted $C_{1-6}$ alkoxy group, a carboxyl group, an optionally substituted

$C_{2-7}$ alkoxycarbonyl group or a group represented by Formula -CONV$^{a11}$N$^{a12}$ (wherein, V$^{a11}$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group or an optionally substituted 3-10-membered nonaromatic heterocyclic group; V$^{a12}$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group, an optionally substituted 3-10-membered nonaromatic heterocyclic group, a hydroxyl group, an optionally substituted $C_{1-6}$ alkoxy group or an optionally substituted $C_{3-8}$ cycloalkoxy group);
Y$^1$ represents a group represented by either one of the following formulae

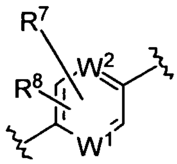

or

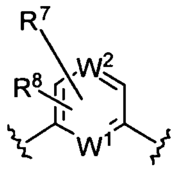

(wherein, R$^7$ and R$^8$ each independently represent a hydrogen atom, a halogen atom, a cyano group, a nitro group, an amino group, an optionally substituted $C_{1-6}$ alkyl group, an optionally substituted $C_{3-8}$ cycloalkyl group, an optionally substituted $C_{1-6}$ alkoxy group, an optionally substituted $C_{1-6}$ alkylthio group, a formyl group, an optionally substituted $C_{2-7}$ acyl group, an optionally substituted $C_{2-7}$ alkoxycarbonyl group or a group represented by Formula -CONV$^{d1}$V$^{d2}$ (wherein, V$^{d1}$ and V$^{d2}$ each independently represent a hydrogen atom or an optionally substituted $C_{1-6}$ alkyl group);
W$^1$ and W$^2$ each independently represent an optionally substituted carbon atom or nitrogen atom);
R$^3$ and R$^4$ each independently represent a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted
$C_{2-7}$ acyl group or an optionally substituted $C_{2-7}$ alkoxycarbonyl group; and
R$^5$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group or an optionally substituted 3-10-membered nonaromatic heterocyclic group].

**78.** The method for treating cancer according to Claim 77, wherein R$^1$ is a $C_{1-6}$ alkyl group (provided that R$^1$ may have a substituent selected from a 3-10-membered nonaromatic heterocyclic group, a hydroxyl group, a $C_{1-6}$ alkoxy group, an amino group, a mono-$C_{1-6}$ alkylamino group and a di-$C_{1-6}$ alkylamino group which may have a $C_{1-6}$ alkyl group).

**79.** The method for treating cancer according to Claim 77, wherein R$^1$ is a methyl group or a group represented by any one of the following formulae

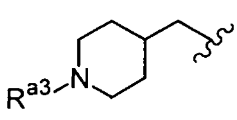

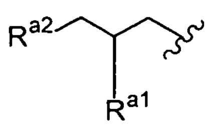

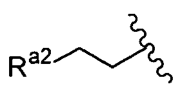

(wherein, R$^{a3}$ represents a methyl group; R$^{a1}$ represents a hydrogen atom or a hydroxyl group; R$^{a2}$ represents a methoxy group, an ethoxy group, a 1-pyrrolidinyl group, a 1-piperidinyl group, a 4-morpholinyl group, a dimethylamino group or a diethylamino group).

**80.** The method for treating cancer according to Claim 77, wherein R$^1$ is a methyl group or a 2-methoxyethyl group.

**81.** The method for treating cancer according to Claim 77, wherein R$^2$ is a cyano group or a group represented by

Formula -CONV$^{a11}$V$^{a12}$ (wherein, V$^{a11}$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group or an optionally substituted 3-10-membered nonaromatic heterocyclic group; V$^{a12}$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group, an optionally substituted 3-10-membered nonaromatic heterocyclic group, a hydroxyl group, an optionally substituted $C_{1-6}$ alkoxy group or an optionally substituted $C_{3-8}$ cycloalkoxy group).

**82.** The method for treating cancer according to Claim 77, wherein $R^2$ is a cyano group or a group represented by Formula -CONHV$^{a16}$ (wherein, V$^{a16}$ represents a hydrogen atom, a $C_{1-6}$ alkyl group, a $C_{3-8}$ cycloalkyl group, a $C_{1-6}$ alkoxy group or a $C_{3-8}$ cycloalkoxy group, provided that V$^{a16}$ may have a substituent selected from the a halogen atom, a cyano group, a hydroxyl group and a $C_{1-6}$ alkoxy group).

**83.** The method for treating cancer according to Claim 77, wherein $R^2$ is a group represented by Formula -CONHV$^{a17}$ (wherein, V$^{a17}$ represents a hydrogen atom, a $C_{1-6}$ alkyl group or a $C_{1-6}$ alkoxy group).

**84.** The method for treating cancer according to Claim 77, wherein $R^2$ is a group represented by Formula -CONHV$^{a18}$ (wherein, V$^{a18}$ represents a hydrogen atom, a methyl group or a methoxy group).

**85.** The method for treating cancer according to Claim 77, wherein $Y^1$ is a group represented by the following formula

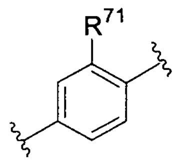

(wherein, $R^{71}$ represents a hydrogen atom or a halogen atom).

**86.** The method for treating cancer according to Claim 77, wherein $R^3$ and $R^4$ are hydrogen atoms.

**87.** The method for treating cancer according to Claim 77, wherein $R^5$ is a hydrogen atom, a $C_{1-6}$ alkyl group, a $C_{3-8}$ cycloalkyl group or a $C_{6-10}$ aryl group (provided that $R^5$ may have a substituent selected from a halogen atom and a methanesulfonyl group).

**88.** The method for treating cancer according to Claim 77, wherein $R^5$ is a methyl group, an ethyl group or a cyclopropyl group.

**89.** The method for treating cancer according to Claim 77, wherein the compound represented by General Formula (I), a pharmacologically acceptable salt thereof or a solvate thereof is at least one compound selected from the group consisting of:

N-(4-(6-cyano-7-(2-methoxyethoxy)-4-quinolyl)oxy-2-fluorophenyl)-N'-(4-fluorophenyl)urea;
N-(2-chloro-4-((6-cyano-7-((1-methyl-4-piperidyl)methoxy)-4-quinolyl)oxy)phenyl)-N'-cyclopropylurea;
N-(4-((6- cyano- 7-(((2R)- 3-(diethylamino)- 2- hydroxypropyl) oxy)- 4- quinolyl) oxy) phenyl)-N'-(4- fluorophenyl) urea;
N-(4-((6- cyano- 7-(((2R)- 2- hydroxy- 3-(1- pyrrolidino) propyl) oxy)- 4- quinolyl) oxy) phenyl)-N'-(4- fluorophenyl) urea;
4-(3-chloro-4-(cyclopropylaminocarbonyl)aminophenoxy)-7-methoxy-6-quinolinecarboxamide;
4-(3-chloro-4-(cyclopropylaminocarbonyl)aminophenoxy)-7-(2-methoxyethoxy)-6-quinolinecarboxamide;
N6-cyclopropyl-4-(3-chloro-4-(((cyclopropylamino)carbonyl)amino)phenoxy)-7-methoxy-6-quinolinecarboxamide;
N6-(2-methoxyethyl)-4-(3-chloro-4-(((cyclopropylamino)carbonyl)amino)phenoxy)-7-methoxy-6-quinolinecarboxamide;
N6-(2-fluoroethyl)-4-(3-chloro-4-(((cyclopropylamino)carbonyl)amino)phenoxy)-7-methoxy-6-quinolinecarboxamide;
N6-methoxy-4-(3-chloro-4-(((cyclopropylamino)carbonyl)amino)phenoxy)-7-methoxy-6-quinolinecarboxamide;
N6-methyl-4-(3-chloro-4-(((cyclopropylamino)carbonyl)amino)phenoxy)-7-methoxy-6-quinolinecarboxamide;
N6-ethyl-4-(3-chloro-4-(((cyclopropylamino)carbonyl)amino)phenoxy)-7-methoxy-6-quinolinecarboxamide;
4-(3-fluoro-4-(cyclopropylaminocarbonyl)aminophenoxy)-7-(2-methoxyethoxy)-6-quinolinecarboxamide;
4-(3-chloro-4-(cyclopropylaminocarbonyl)aminophenoxy)-7-(2-hydroxyethoxy)-6-quinolinecarboxamide;
4-(3-chloro-4-(cyclopropylaminocarbonyl)aminophenoxy)-7-((2S)-2,3-dihydroxypropyl)oxy-6-quinolinecarboxamide;
4-(3-chloro-4-(methylaminocarbonyl)aminophenoxy)-7-methoxy-6-quinolinecarboxamide;
4-(3-chloro-4-(ethylaminocarbonyl)aminophenoxy)-7-methoxy-6-quinolinecarboxamide;
N6-methoxy-4-(3-chloro-4-(((ethylamino)carbonyl)amino)phenoxy)-7-methoxy-6-quinolinecarboxamide;
4-(3-chloro-4-(cyclopropylaminocarbonyl)aminophenoxy)-7-(2-ethoxyethoxy)-6-quinolinecarboxamide;
4-(4-((cyclopropylamino)carbonyl)aminophenoxy)-7-(2-methoxyethoxy)-6-quinolinecarboxamide;
N-(2-fluoro-4-((6-carbamoyl-7-methoxy-4-quinolyl)oxy)phenyl)-N'-cyclopropylurea;
N6-(2-hydroxyethyl)-4-(3-chloro-4-(((cyclopropylamino)carbonyl)amino)phenoxy)-7-methoxy-6-quinolinecarboxamide;
4-(3-chloro-4-(1-propylaminocarbonyl)aminophenoxy)-7-methoxy-6-quinolinecarboxamide;
4-(3-chloro-4-(cis-2-fluoro-cyclopropylaminocarbonyl)aminophenoxy)-7-methoxy-6-quinolinecarboxamide;
N6-methyl-4-(3-chloro-4-(((cyclopropylamino)carbonyl)amino)phenoxy)-7-(2-methoxyethoxy)-6-quinolinecarboxamide;
N6-methyl-4-(3-chloro-4-(((ethylamino)carbonyl)amino)phenoxy)-7-methoxy-6-quinolinecarboxamide;
4-(3-chloro-4-(cyclopropylaminocarbonyl)aminophenoxy)-7-(2-(4-morpholino)ethoxy)-6-quinolinecarboxamide;
4-(3-chloro-4-(2-fluoroethylaminocarbonyl)aminophenoxy)-7-methoxy-6-quinolinecarboxamide;
N6-((2R)tetrahydro-2-furanylmethyl)-4-(3-chloro-4-(((methylamino)carbonyl)amino)phenoxy)-7-methoxy-6-quinolinecarboxamide;
4-(3-fluoro-4-(ethylaminocarbonyl)aminophenoxy)-7-methoxy-6-quinolinecarboxamide;
4-(3-chloro-4-(((cyclopropylamino)carbonyl)amino)phenoxy)-7-((2R)-2-hydroxy-3-(1-pyrrolidino)propoxy)-6-quinolinecarboxamide;
N6-methyl-4-(3-chloro-4-(((methylamino)carbonyl)amino)phenoxy)-7-((2R)-3-diethylamino-2-hydroxypropoxy)-6-quinolinecarboxamide;
N6-methyl-4-(3-chloro-4-(((ethylamino)carbonyl)amino)phenoxy)-7-((2R)-3-diethylamino-2-hydroxypropoxy)-6-quinolinecarboxamide;
N6-methyl-4-(3-chloro-4-(((methylamino)carbonyl)amino)phenoxy)-7-((2R)-2-hydroxy-3-(1-pyrrolidino)propoxy)-6-quinolinecarboxamide;
N6-methyl-4-(3-chloro-4-(((ethylamino)carbonyl)amino)phenoxy)-7-((2R)-2-hydroxy-3-(1-pyrrolidino)propoxy)-6-quinolinecarboxamide;
N6-methyl-4-(3-chloro-4-(((methylamino)carbonyl)amino)phenoxy)-7-((1-methyl-4-piperidyl)methoxy)-6-quinolinecarboxamide;
N6-methyl-4-(3-chloro-4-(((ethylamino)carbonyl)amino)phenoxy)-7-((1-methyl-4-piperidyl)methoxy)-6-quinolinecarboxamide;
N-(4-(6-cyano-7-(2-methoxyethoxy)-4-quinolyl)oxy-2-fluorophenyl)-N'-cyclopropylurea;
N-(4-(6-cyano-7-(3-(4-morpholino)propoxy)-4-quinolyl)oxyphenyl)-N'-(3-(methylsulfonyl)phenyl)urea;
4-(4-((cyclopropylamino)carbonyl)aminophenoxy)-7-methoxy-6-quinolinecarboxamide;
4-(3-fluoro-4-((2-fluoroethylamino)carbonyl)aminophenoxy)-7-methoxy-6-quinolinecarboxamide;
N6-(2-ethoxyethyl)-4-(3-chloro-4-(((methylamino)carbonyl)amino)phenoxy)-7-methoxy-6-quinolinecarboxamide;
4-(4-(3-ethylureido)-3-fluoro-phenoxy)-7-methoxyquinoline-6-carboxylic acid (2-cyanoethyl)amide; and
N-(4-(6-(2-cyanoethyl)carbamoyl-7-methoxy-4-quinolyl)oxy-2-fluorophenyl)-N'-cyclopropylurea,

a pharmacologically acceptable salt thereof or a solvate thereof.

**90.** The method for treating cancer according to Claim 77, wherein the compound represented by General Formula (I), a pharmacologically acceptable salt thereof or a solvate thereof is at least one compound selected from the group consisting of:

4-(3-chloro-4-(cyclopropylaminocarbonyl)aminophenoxy)-7-methoxy-6-quinolinecarboxamide;

4-(3-chloro-4-(ethylaminocarbonyl)aminophenoxy)-7-methoxy-6-quinolinecarboxamide;
N6-methoxy-4-(3-chloro-4-(((cyclopropylamino)carbonyl)amino)phenoxy)-7-methoxy-6-quinolinecarboxamide;
4-(3-chloro-4-(methylaminocarbonyl)aminophenoxy)-7-methoxy-6-quinolinecarboxamide; and
N6-methoxy-4-(3-chloro-4-(((ethylamino)carbonyl)amino)phenoxy)-7-methoxy-6-quinolinecarboxamide,

a pharmacologically acceptable salt thereof or a solvate thereof.

**91.** The method for treating cancer according to Claim 77, wherein the compound represented by General Formula (I), a pharmacologically acceptable salt thereof or a solvate thereof is 4-(3-chloro-4-(cyclopropylaminocarbonyl)aminophenoxy)-7-methoxy-6-quinolinecarboxamide, a pharmacologically acceptable salt thereof or a solvate thereof.

**92.** The method for treating cancer according to Claim 77, wherein the compound represented by General Formula (I), a pharmacologically acceptable salt thereof or a solvate thereof is methanesulfonate of 4-(3-chloro-4-(cyclopropylaminocarbonyl)aminophenoxy)-7-methoxy-6-quinolinecarboxamide.

**93.** The method for treating cancer according to Claim 77, wherein gemcitabine, a pharmacologically acceptable salt thereof or a solvate thereof is gemcitabine hydrochloride.

**94.** The method for treating cancer according to Claim 77, wherein erlotinib, a pharmacologically acceptable salt thereof or a solvate thereof is erlotinib hydrochloride.

**95.** The method for treating cancer according to any one of Claims 77-94, wherein the pharmaceutical composition is for treating cancer.

**96.** Use of a compound represented by General Formula (I), a pharmacologically acceptable salt thereof or a solvate thereof for producing a pharmaceutical composition in combination with gemcitabine or erlotinib, a pharmacologically acceptable salt thereof or a solvate thereof:

General Formula (I)

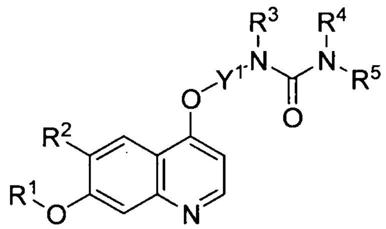

(I)

[wherein, $R^1$ represents a group represented by Formula $-V^1-V^2-V^3$ (wherein, $V^1$ represents an optionally substituted $C_{1-6}$ alkylene group; $V^2$ represents a single bond, an oxygen atom, a sulfur atom, a carbonyl group, a sulfinyl group, a sulfonyl group, a group represented by Formula $-CONR^6-$, a group represented by Formula $-SO_2NR^6-$, a group represented by Formula $-NR^6SO_2-$, a group represented by Formula $-NR^6CO-$ or a group represented by Formula $-NR^6-$ (wherein, $R^6$ represents a hydrogen atom, an optionally substituted $C_{1-6}$ alkyl group or an optionally substituted $C_{3-8}$ cycloalkyl group)); $V^3$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group or an optionally substituted 3-10-membered nonaromatic heterocyclic group);
$R^2$ represents a cyano group, an optionally substituted $C_{1-6}$ alkoxy group, a carboxyl group, an optionally substituted $C_{2-7}$ alkoxycarbonyl group or a group represented by Formula $-CONV^{a11}V^{a12}$ (wherein, $V^{a11}$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group or an optionally substituted 3-10-membered nonaromatic heterocyclic group; $V^{a12}$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group, an

optionally substituted 3-10-membered nonaromatic heterocyclic group, a hydroxyl group, an optionally substituted $C_{1-6}$ alkoxy group or an optionally substituted $C_{3-8}$ cycloalkoxy group);
$Y^1$ represents a group represented by either one of the following formulae

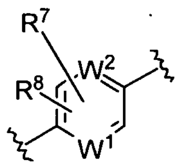

or

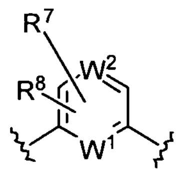

(wherein, $R^7$ and $R^8$ each independently represent a hydrogen atom, a halogen atom, a cyano group, a nitro group, an amino group, an optionally substituted $C_{1-6}$ alkyl group, an optionally substituted $C_{3-8}$ cycloalkyl group, an optionally substituted $C_{1-6}$ alkoxy group, an optionally substituted $C_{1-6}$ alkylthio group, a formyl group, an optionally substituted $C_{2-7}$ acyl group, an optionally substituted $C_{2-7}$ alkoxycarbonyl group or a group represented by Formula -CONV$^{d11}$V$^{d2}$ (wherein, $V^{d1}$ and $V^{d2}$ each independently represent a hydrogen atom or an optionally substituted $C_{1-6}$ alkyl group);
$W^1$ and $W^2$ each independently represent an optionally substituted carbon atom or nitrogen atom);
$R^3$ and $R^4$ each independently represent a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{2-7}$ acyl group or an optionally substituted $C_{2-7}$ alkoxycarbonyl group; and
$R^5$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group or an optionally substituted 3-10-membered nonaromatic heterocyclic group].

**97.** Use according to Claim 96, wherein $R^1$ is a $C_{1-6}$ alkyl group (provided that $R^1$ may have a substituent selected from a 3-10-membered nonaromatic heterocyclic group, a hydroxyl group, a $C_{1-6}$ alkoxy group, an amino group, a mono-$C_{1-6}$ alkylamino group and a di-$C_{1-6}$ alkylamino group which may have a $C_{1-6}$ alkyl group).

**98.** Use according to Claim 96, wherein $R^1$ is a methyl group or a group represented by any one of the following formulae

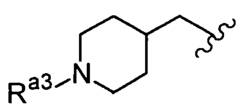

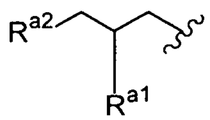

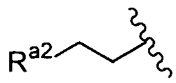

(wherein, $R^{a3}$ represents a methyl group; $R^{a1}$ represents a hydrogen atom or a hydroxyl group; $R^{a2}$ represents a methoxy group, an ethoxy group, a 1-pyrrolidinyl group, a 1-piperidinyl group, a 4-morpholinyl group, a dimethylamino group or a diethylamino group).

**99.** Use according to Claim 96, wherein $R^1$ is a methyl group or a 2-methoxyethyl group.

**100.** Use according to Claim 96, wherein $R^2$ is a cyano group or a group represented by Formula -CONV$^{a11}$V$^{a12}$ (wherein, $V^{a11}$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group or an optionally substituted 3-10-membered nonaromatic heterocyclic group; $V^{a12}$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group, an optionally substituted 3-10-membered nonaromatic heterocyclic group, a hydroxyl group, an optionally substituted $C_{1-6}$ alkoxy group or an optionally substituted $C_{3-8}$ cycloalkoxy group).

**101.** Use according to Claim 96, wherein $R^2$ is a cyano group or a group represented by Formula -CONHV$^{a16}$ (wherein, $V^{a16}$ represents a hydrogen atom, a $C_{1-6}$ alkyl group, a $C_{3-8}$ cycloalkyl group, a $C_{1-6}$ alkoxy group or a $C_{3-8}$ cycloalkoxy

group, provided that $V^{a16}$ may have a substituent selected from a halogen atom, a cyano group, a hydroxyl group and a $C_{1-6}$ alkoxy group).

**102.** Use according to Claim 96, wherein $R^2$ is a group represented by Formula-CONHV$^{a17}$ (wherein, $V^{a17}$ represents a hydrogen atom, a $C_{1-6}$ alkyl group or a $C_{1-6}$ alkoxy group).

**103.** Use according to Claim 96, wherein $R^2$ is a group represented by Formula-CONHV$^{a18}$ (wherein, $V^{a18}$ represents a hydrogen atom, a methyl group or a methoxy group).

**104.** Use according to Claim 96, wherein $Y^1$ is a group represented by the following formula

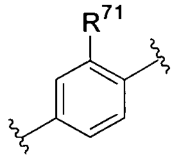

(wherein, $R^{71}$ represents a hydrogen atom or a halogen atom).

**105.** Use according to Claim 96, wherein $R^3$ and $R^4$ are hydrogen atoms.

**106.** Use according to Claim 96, wherein $R^5$ is a hydrogen atom, a $C_{1-6}$ alkyl group, a $C_{3-8}$ cycloalkyl group or a $C_{6-10}$ aryl group (provided that $R^5$ may have a substituent selected from a halogen atom and a methanesulfonyl group).

**107.** Use according to Claim 96, wherein $R^5$ is a methyl group, an ethyl group or a cyclopropyl group.

**108.** Use according to Claim 96, wherein the compound represented by General Formula (I), a pharmacologically acceptable salt thereof or a solvate thereof is at least one compound selected from the group consisting of:

N-(4-(6-cyano-7-(2-methoxyethoxy)-4-quinolyl)oxy-2-fluorophenyl)-N'-(4-fluorophenyl)urea;
N-(2-chloro-4-((6-cyano-7-((1-methyl-4-piperidyl)methoxy)-4-quinolyl)oxy)phenyl)-N'-cyclopropylurea;
N-(4-((6-cyano-7-(((2R)-3-(diethylamino)-2-hydroxypropyl)oxy)-4-quinolyl)oxy)phenyl)-N'-(4-fluorophenyl)urea;
N-(4-((6-cyano-7-(((2R)-2-hydroxy-3-(1-pyrrolidino)propyl)oxy)-4-quinolyl)oxy)phenyl)-N'-(4-fluorophenyl)urea;
4-(3-chloro-4-(cyclopropylaminocarbonyl)aminophenoxy)-7-methoxy-6-quinolinecarboxamide;
4-(3-chloro-4-(cyclopropylaminocarbonyl)aminophenoxy)-7-(2-methoxyethoxy)-6-quinolinecarboxamide;
N6-cyclopropyl-4-(3-chloro-4-(((cyclopropylamino)carbonyl)amino)phenoxy)-7-methoxy-6-quinolinecarboxamide;
N6-(2-methoxyethyl)-4-(3-chloro-4-(((cyclopropylamino)carbonyl)amino)phenoxy)-7-methoxy-6-quinolinecarboxamide;
N6-(2-fluoroethyl)-4-(3-chloro-4-(((cyclopropylamino)carbonyl)amino)phenoxy)-7-methoxy-6-quinolinecarboxamide;
N6-methoxy-4-(3-chloro-4-(((cyclopropylamino)carbonyl)amino)phenoxy)-7-methoxy-6-quinolinecarboxamide;
N6-methyl-4-(3-chloro-4-(((cyclopropylamino)carbonyl)amino)phenoxy)-7-methoxy-6-quinolinecarboxamide;
N6-ethyl-4-(3-chloro-4-(((cyclopropylamino)carbonyl)amino)phenoxy)-7-methoxy-6-quinolinecarboxamide;
4-(3-fluoro-4-(cyclopropylaminocarbonyl)aminophenoxy)-7-(2-methoxyethoxy)-6-quinolinecarboxamide;
4-(3-chloro-4-(cyclopropylaminocarbonyl)aminophenoxy)-7-(2-hydroxyethoxy)-6-quinolinecarboxamide;
4-(3-chloro-4-(cyclopropylaminocarbonyl)aminophenoxy)-7-((2S)-2,3-dihydroxypropyl)oxy-6-quinolinecarboxamide;
4-(3-chloro-4-(methylaminocarbonyl)aminophenoxy)-7-methoxy-6-quinolinecarboxamide;
4-(3-chloro-4-(ethylaminocarbonyl)aminophenoxy)-7-methoxy-6-quinolinecarboxamide;
N6-methoxy-4-(3-chloro-4-(((ethylamino)carbonyl)amino)phenoxy)-7-methoxy-6-quinolinecarboxamide;
4-(3-chloro-4-(cyclopropylaminocarbonyl)aminophenoxy)-7-(2-ethoxyethoxy)-6-quinolinecarboxamide;
4-(4-((cyclopropylamino)carbonyl)aminophenoxy)-7-(2-methoxyethoxy)-6-quinolinecarboxamide;

N-(2-fluoro-4-((6-carbamoyl-7-methoxy-4-quinolyl)oxy)phenyl)-N'-cyclopropylurea;
N6-(2-hydroxyethyl)-4-(3-chloro-4-(((cyclopropylamino)carbonyl)amino)phenoxy)-7-methoxy-6-quinolinecarboxamide;
4-(3-chloro-4-(1-propylaminocarbonyl)aminophenoxy)-7-methoxy-6-quinolinecarboxamide;
4-(3-chloro-4-(cis-2-fluoro-cyclopropylaminocarbonyl)aminophenoxy)-7-methoxy-6-quinolinecarboxamide;
N6-methyl-4-(3-chloro-4-(((cyclopropylamino)carbonyl)amino)phenoxy)-7-(2-methoxyethoxy)-6-quinolinecarboxamide;
N6-methyl-4-(3-chloro-4-(((ethylamino)carbonyl)amino)phenoxy)-7-methoxy-6-quinolinecarboxamide;
4-(3-chloro-4-(cyclopropylaminocarbonyl)aminophenoxy)-7-(2-(4-morpholino)ethoxy)-6-quinolinecarboxamide;
4-(3-chloro-4-(2-fluoroethylaminocarbonyl)aminophenoxy)-7-methoxy-6-quinolinecarboxamide;
N6-((2R)tetrahydro-2-furanylmethyl)-4-(3-chloro-4-(((methylamino)carbonyl)amino)phenoxy)-7-methoxy-6-quinolinecarboxamide;
4-(3-fluoro-4-(ethylaminocarbonyl)aminophenoxy)-7-methoxy-6-quinolinecarboxamide;
4-(3-chloro-4-(((cyclopropylamino)carbonyl)amino)phenoxy)-7-((2R)-2-hydroxy-3-(1-pyrrolidino)propoxy)-6-quinolinecarboxamide;
N6-methyl-4-(3-chloro-4-(((methylamino)carbonyl)amino)phenoxy)-7-((2R)-3-diethylamino-2-hydroxypropoxy)-6-quinolinecarboxamide;
N6-methyl-4-(3-chloro-4-(((ethylamino)carbonyl)amino)phenoxy)-7-((2R)-3-diethylamino-2-hydroxypropoxy)-6-quinolinecarboxamide;
N6-methyl-4-(3-chloro-4-(((methylamino)carbonyl)amino)phenoxy)-7-((2R)-2-hydroxy-3-(1-pyrrolidino)propoxy)-6-quinolinecarboxamide;
N6-methyl-4-(3-chloro-4-(((ethylamino)carbonyl)amino)phenoxy)-7-((2R)-2-hydroxy-3-(1-pyrrolidino)propoxy)-6-quinolinecarboxamide;
N6-methyl-4-(3-chloro-4-(((methylamino)carbonyl)amino)phenoxy)-7-((1-methyl-4-piperidyl)methoxy)-6-quinolinecarboxamide;
N6-methyl-4-(3-chloro-4-(((ethylamino)carbonyl)amino)phenoxy)-7-((1-methyl-4-piperidyl)methoxy)-6-quinolinecarboxamide;
N-(4-(6-cyano-7-(2-methoxyethoxy)-4-quinolyl)oxy-2-fluorophenyl)-N'-cyclopropylurea;
N-(4-(6-cyano-7-(3-(4-morpholino)propoxy)-4-quinolyl)oxyphenyl)-N'-(3-(methylsulfonyl)phenyl)urea;
4-(4-((cyclopropylamino)carbonyl)aminophenoxy)-7-methoxy-6-quinolinecarboxamide;
4-(3-fluoro-4-((2-fluoroethylamino)carbonyl)aminophenoxy)-7-methoxy-6-quinolinecarboxamide;
N6-(2-ethoxyethyl)-4-(3-chloro-4-(((methylamino)carbonyl)amino)phenoxy)-7-methoxy-6-quinolinecarboxamide;
4-(4-(3-ethylureido)-3-fluoro-phenoxy)-7-methoxyquinoline-6-carboxylic acid (2-cyanoethyl)amide; and
N-(4-(6-(2-cyanoethyl)carbamoyl-7-methoxy-4-quinolyl)oxy-2-fluorophenyl)-N'-cyclopropylurea,

a pharmacologically acceptable salt thereof or a solvate thereof.

**109.** Use according to Claim 96, wherein the compound represented by General Formula (I), a pharmacologically acceptable salt thereof or a solvate thereof is at least one compound selected from the group consisting of:

4-(3-chloro-4-(cyclopropylaminocarbonyl)aminophenoxy)-7-methoxy-6-quinolinecarboxamide;
4-(3-chloro-4-(ethylaminocarbonyl)aminophenoxy)-7-methoxy-6-quinolinecarboxamide;
N6-methoxy-4-(3-chloro-4-(((cyclopropylamino)carbonyl)amino)phenoxy)-7-methoxy-6-quinolinecarboxamide;
4-(3-chloro-4-(methylaminocarbonyl)aminophenoxy)-7-methoxy-6-quinolinecarboxamide; and
N6-methoxy-4-(3-chloro-4-(((ethylamino)carbonyl)amino)phenoxy)-7-methoxy-6-quinolinecarboxamide,

a pharmacologically acceptable salt thereof or a solvate thereof.

**110.** Use according to Claim 96, wherein the compound represented by General Formula (I), a pharmacologically acceptable salt thereof or a solvate thereof is 4-(3-chloro-4-(cyclopropylaminocarbonyl)aminophenoxy)-7-methoxy-6-quinolinecarboxamide, a pharmacologically acceptable salt thereof or a solvate thereof.

**111.** Use according to Claim 96, wherein the compound represented by General Formula (I), a pharmacologically acceptable salt thereof or a solvate thereof is methanesulfonate of 4-(3-chloro-4-(cyclopropylaminocarbonyl)aminophenoxy)-7-methoxy-6-quinolinecarboxamide.

**112.** Use according to Claim 96, wherein gemcitabine, a pharmacologically acceptable salt thereof or a solvate thereof is gemcitabine hydrochloride.

**113.** Use according to Claim 96, wherein erlotinib, a pharmacologically acceptable salt thereof or a solvate thereof is erlotinib hydrochloride.

**114.** Use according to any one of Claims 96-113, wherein the pharmaceutical composition is for treating cancer.

**115.** A compound represented by General Formula (I), a pharmacologically acceptable salt thereof or a solvate thereof for a pharmaceutical composition in combination with gemcitabine or erlotinib, a pharmacologically acceptable salt thereof or a solvate thereof:

General Formula (I)

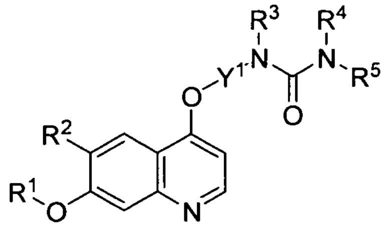

(I)

[wherein, $R^1$ represents a group represented by Formula $-V^1-V^2-V^3$ (wherein, $V^1$ represents an optionally substituted $C_{1-6}$ alkylene group; $V^2$ represents a single bond, an oxygen atom, a sulfur atom, a carbonyl group, a sulfinyl group, a sulfonyl group, a group represented by Formula $-CONR^6-$, a group represented by Formula $-SO_2NR^6-$, a group represented by Formula $-NR^6SO_2-$, a group represented by Formula $-NR^6CO-$ or a group represented by Formula $-NR^6-$ (wherein, $R^6$ represents a hydrogen atom, an optionally substituted $C_{1-6}$ alkyl group or an optionally substituted $C_{3-8}$ cycloalkyl group)); $V^3$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group or an optionally substituted 3-10-membered nonaromatic heterocyclic group);
$R^2$ represents a cyano group, an optionally substituted $C_{1-6}$ alkoxy group, a carboxyl group, an optionally substituted $C_{2-7}$ alkoxycarbonyl group or a group represented by Formula $-CONV^{a11}V^{a12}$ (wherein, $V^{a11}$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group or an optionally substituted 3-10-membered nonaromatic heterocyclic group; $V^{a12}$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group, an optionally substituted 3-10-membered nonaromatic heterocyclic group, a hydroxyl group, an optionally substituted $C_{1-6}$ alkoxy group or an optionally substituted $C_{3-8}$ cycloalkoxy group);
$Y^1$ represents a group represented by either one of the following formulae

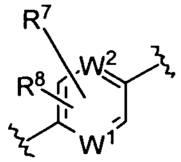

or

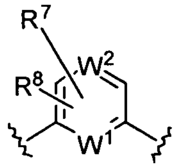

(wherein, $R^7$ and $R^8$ each independently represent a hydrogen atom, a halogen atom, a cyano group, a nitro group, an amino group, an optionally substituted $C_{1-6}$ alkyl group, an optionally substituted $C_{3-8}$ cycloalkyl group, an optionally substituted $C_{1-6}$ alkoxy group, an optionally substituted $C_{1-6}$ alkylthio group, a formyl group, an optionally substituted $C_{2-7}$ acyl group, an optionally substituted $C_{2-7}$ alkoxycarbonyl group or a group represented by Formula -CONV$^{d1}$V$^{d2}$ (wherein, $V^{d1}$ and $V^{d2}$ each independently represent a hydrogen atom or an optionally substituted $C_{1-6}$ alkyl group);
$W^1$ and $W^2$ each independently represent an optionally substituted carbon atom or nitrogen atom);
$R^3$ and $R^4$ each independently represent a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted
$C_{2-7}$ acyl group or an optionally substituted $C_{2-7}$ alkoxycarbonyl group; and
$R^5$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group or an optionally substituted 3-10-membered nonaromatic heterocyclic group].

**116.** The compound, a pharmacologically acceptable salt thereof or a solvate thereof according to Claim 115, wherein $R^1$ is a $C_{1-6}$ alkyl group (provided that $R^1$ may have a substituent selected from a 3-10-membered nonaromatic heterocyclic group, a hydroxyl group, a $C_{1-6}$ alkoxy group, an amino group, a mono-$C_{1-6}$ alkylamino group and a di-$C_{1-6}$ alkylamino group which may have a $C_{1-6}$ alkyl group).

**117.** The compound, a pharmacologically acceptable salt thereof or a solvate thereof according to Claim 115, wherein $R^1$ is a methyl group or a group represented by any one of the following formulae

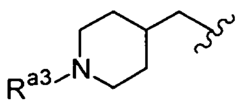
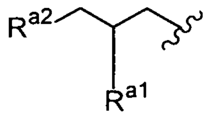
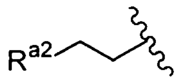

(wherein, $R^{a3}$ represents a methyl group; $R^{a1}$ represents a hydrogen atom or a hydroxyl group; $R^{a2}$ represents a methoxy group, an ethoxy group, a 1-pyrrolidinyl group, a 1-piperidinyl group, a 4-morpholinyl group, a dimethylamino group or a diethylamino group).

**118.** The compound, a pharmacologically acceptable salt thereof or a solvate thereof according to Claim 115, wherein $R^1$ is a methyl group or a 2-methoxyethyl group.

**119.** The compound, a pharmacologically acceptable salt thereof or a solvate thereof according to Claim 115, wherein $R^2$ is a cyano group or a group represented by Formula-CONV$^{a11}$V$^{a12}$ (wherein, $V^{a11}$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group or an optionally substituted 3-10-membered nonaromatic heterocyclic group; $V^{a12}$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group, an optionally substituted 3-10-membered nonaromatic heterocyclic group, a hydroxyl group, an optionally substituted $C_{1-6}$ alkoxy group or an optionally substituted $C_{3-8}$ cycloalkoxy group).

**120.** The compound, a pharmacologically acceptable salt thereof or a solvate thereof according to Claim 115, wherein $R^2$ is a cyano group or a group represented by Formula-CONHV$^{a16}$ (wherein, $V^{a16}$ represents a hydrogen atom, a $C_{1-6}$ alkyl group, a $C_{3-8}$ cycloalkyl group, a $C_{1-6}$ alkoxy group or a $C_{3-8}$ cycloalkoxy group, provided that $V^{a16}$ may have a substituent selected from a halogen atom, a cyano group, a hydroxyl group and a $C_{1-6}$ alkoxy group).

**121.** The compound, a pharmacologically acceptable salt thereof or a solvate thereof according to Claim 115, wherein $R^2$ is a group represented by Formula -CONHV$^{a17}$ (wherein, $V^{a17}$ represents a hydrogen atom, a $C_{1-6}$ alkyl group or a $C_{1-6}$ alkoxy group).

**122.** The compound, a pharmacologically acceptable salt thereof or a solvate thereof according to Claim 115, wherein $R^2$ is a group represented by Formula -CONHV$^{a18}$ (wherein, $V^{a18}$ represents a hydrogen atom, a methyl group or a methoxy group).

**123.** The compound, a pharmacologically acceptable salt thereof or a solvate thereof according to Claim 115, wherein $Y^1$ is a group represented by the following formula

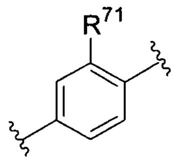

(wherein, $R^{71}$ represents a hydrogen atom or a halogen atom).

**124.** The compound, a pharmacologically acceptable salt thereof or a solvate thereof according to Claim 115, wherein $R^3$ and $R^4$ are hydrogen atoms.

**125.** The compound, a pharmacologically acceptable salt thereof or a solvate thereof according to Claim 115, wherein $R^5$ is a hydrogen atom, a $C_{1-6}$ alkyl group, a $C_{3-8}$ cycloalkyl group or a $C_{6-10}$ aryl group (provided that $R^5$ may have a substituent selected from a halogen atom and a methanesulfonyl group).

**126.** The compound, a pharmacologically acceptable salt thereof or a solvate thereof according to Claim 115, wherein $R^5$ is a methyl group, an ethyl group or a cyclopropyl group.

**127.** The compound, a pharmacologically acceptable salt thereof or a solvate thereof according to Claim 115, wherein the compound represented by General Formula (I), a pharmacologically acceptable salt thereof or a solvate thereof is at least one compound selected from the group consisting of:

N-(4-(6-cyano-7-(2-methoxyethoxy)-4-quinolyl)oxy-2-fluorophenyl)-N'-(4-fluorophenyl)urea;
N-(2-chloro-4-((6-cyano-7-((1-methyl-4-piperidyl)methoxy)-4-quinolyl)oxy)phenyl)-N'-cyclopropylurea;
N-(4-((6-cyano-7-(((2R)-3-(diethylamino)-2-hydroxypropyl)oxy)-4-quinolyl)oxy)phenyl)-N'-(4-fluorophenyl)urea;
N-(4-((6-cyano-7-(((2R)-2-hydroxy-3-(1-pyrrolidino)propyl)oxy)-4-quinolyl)oxy)phenyl)-N'-(4-fluorophenyl)urea;
4-(3-chloro-4-(cyclopropylaminocarbonyl)aminophenoxy)-7-methoxy-6-quinolinecarboxamide;
4-(3-chloro-4-(cyclopropylaminocarbonyl)aminophenoxy)-7-(2-methoxyethoxy)-6-quinolinecarboxamide;
N6-cyclopropyl-4-(3-chloro-4-(((cyclopropylamino)carbonyl)amino)phenoxy)-7-methoxy-6-quinolinecarboxamide;
N6-(2-methoxyethyl)-4-(3-chloro-4-(((cyclopropylamino)carbonyl)amino)phenoxy)-7-methoxy-6-quinolinecarboxamide;
N6-(2-fluoroethyl)-4-(3-chloro-4-(((cyclopropylamino)carbonyl)amino)phenoxy)-7-methoxy-6-quinolinecarboxamide;
N6-methoxy-4-(3-chloro-4-(((cyclopropylamino)carbonyl)amino)phenoxy)-7-methoxy-6-quinolinecarboxamide;
N6-methyl-4-(3-chloro-4-(((cyclopropylamino)carbonyl)amino)phenoxy)-7-methoxy-6-quinolinecarboxamide;
N6-ethyl-4-(3-chloro-4-(((cyclopropylamino)carbonyl)amino)phenoxy)-7-methoxy-6-quinolinecarboxamide;
4-(3-fluoro-4-(cyclopropylaminocarbonyl)aminophenoxy)-7-(2-methoxyethoxy)-6-quinolinecarboxamide;
4-(3-chloro-4-(cyclopropylaminocarbonyl)aminophenoxy)-7-(2-hydroxyethoxy)-6-quinolinecarboxamide;
4-(3-chloro-4-(cyclopropylaminocarbonyl)aminophenoxy)-7-((2S)-2,3-dihydroxypropyl)oxy-6-quinolinecarboxamide;
4-(3-chloro-4-(methylaminocarbonyl)aminophenoxy)-7-methoxy-6-quinolinecarboxamide;
4-(3-chloro-4-(ethylaminocarbonyl)aminophenoxy)-7-methoxy-6-quinolinecarboxamide;
N6-methoxy-4-(3-chloro-4-(((ethylamino)carbonyl)amino)phenoxy)-7-methoxy-6-quinolinecarboxamide;
4-(3-chloro-4-(cyclopropylaminocarbonyl)aminophenoxy)-7-(2-ethoxyethoxy)-6-quinolinecarboxamide;
4-(4-((cyclopropylamino)carbonyl)aminophenoxy)-7-(2-methoxyethoxy)-6-quinolinecarboxamide;

N-(2-fluoro-4-((6-carbamoyl-7-methoxy-4-quinolyl)oxy)phenyl)-N'-cyclopropylurea;
N6-(2-hydroxyethyl)-4-(3-chloro-4-(((cyclopropylamino)carbonyl)amino)phenoxy)-7-methoxy-6-quinolinecarboxamide;
4-(3-chloro-4-(1-propylaminocarbonyl)aminophenoxy)-7-methoxy-6-quinolinecarboxamide;
4-(3-chloro-4-(cis-2-fluoro-cyclopropylaminocarbonyl)aminophenoxy)-7-methoxy-6-quinolinecarboxamide;
N6-methyl-4-(3-chloro-4-(((cyclopropylamino)carbonyl)amino)phenoxy)-7-(2-methoxyethoxy)-6-quinolinecarboxamide;
N6-methyl-4-(3-chloro-4-(((ethylamino)carbonyl)amino)phenoxy)-7-methoxy-6-quinolinecarboxamide;
4-(3-chloro-4-(cyclopropylaminocarbonyl)aminophenoxy)-7-(2-(4-morpholino)ethoxy)-6-quinolinecarboxamide;
4-(3-chloro-4-(2-fluoroethylaminocarbonyl)aminophenoxy)-7-methoxy-6-quinolinecarboxamide;
N6-((2R)tetrahydro-2-furanylmethyl)-4-(3-chloro-4-(((methylamino)carbonyl)amino)phenoxy)-7-methoxy-6-quinolinecarboxamide;
4-(3-fluoro-4-(ethylaminocarbonyl)aminophenoxy)-7-methoxy-6-quinolinecarboxamide;
4-(3-chloro-4-(((cyclopropylamino)carbonyl)amino)phenoxy)-7-((2R)-2-hydroxy-3-(1-pyrrolidino)propoxy)-6-quinolinecarboxamide;
N6-methyl-4-(3-chloro-4-(((methylamino)carbonyl)amino)phenoxy)-7-((2R)-3-diethylamino-2-hydroxypropoxy)-6-quinolinecarboxamide;
N6-methyl-4-(3-chloro-4-(((ethylamino)carbonyl)amino)phenoxy)-7-((2R)-3-diethylamino-2-hydroxypropoxy)-6-quinolinecarboxamide;
N6-methyl-4-(3-chloro-4-(((methylamino)carbonyl)amino)phenoxy)-7-((2R)-2-hydroxy-3-(1-pyrrolidino)propoxy)-6-quinolinecarboxamide;
N6-methyl-4-(3-chloro-4-(((ethylamino)carbonyl)amino)phenoxy)-7-((2R)-2-hydroxy-3-(1-pyrrolidino)propoxy)-6-quinolinecarboxamide;
N6-methyl-4-(3-chloro-4-(((methylamino)carbonyl)amino)phenoxy)-7-((1-methyl-4-piperidyl)methoxy)-6-quinolinecarboxamide;
N6-methyl-4-(3-chloro-4-(((ethylamino)carbonyl)amino)phenoxy)-7-((1-methyl-4-piperidyl)methoxy)-6-quinolinecarboxamide;
N-(4-(6-cyano-7-(2-methoxyethoxy)-4-quinolyl)oxy-2-fluorophenyl)-N'-cyclopropylurea;
N-(4-(6-cyano-7-(3-(4-morpholino)propoxy)-4-quinolyl)oxyphenyl)-N'-(3-(methylsulfonyl)phenyl)urea;
4-(4-((cyclopropylamino)carbonyl)aminophenoxy)-7-methoxy-6-quinolinecarboxamide;
4-(3-fluoro-4-((2-fluoroethylamino)carbonyl)aminophenoxy)-7-methoxy-6-quinolinecarboxamide;
N6-(2-ethoxyethyl)-4-(3-chloro-4-(((methylamino)carbonyl)amino)phenoxy)-7-methoxy-6-quinolinecarboxamide;
4-(4-(3-ethylureido)-3-fluoro-phenoxy)-7-methoxyquinoline-6-carboxylic acid (2-cyanoethyl)amide; and
N-(4-(6-(2-cyanoethyl)carbamoyl-7-methoxy-4-quinolyl)oxy-2-fluorophenyl)-N'-cyclopropylurea,

a pharmacologically acceptable salt thereof or a solvate thereof.

**128.** The compound, a pharmacologically acceptable salt thereof or a solvate thereof according to Claim 115, wherein the compound represented by General Formula (I), a pharmacologically acceptable salt thereof or a solvate thereof is at least one compound selected from the group consisting of:

4-(3-chloro-4-(cyclopropylaminocarbonyl)aminophenoxy)-7-methoxy-6-quinolinecarboxamide;
4-(3-chloro-4-(ethylaminocarbonyl)aminophenoxy)-7-methoxy-6-quinolinecarboxamide;
N6-methoxy-4-(3-chloro-4-(((cyclopropylamino)carbonyl)amino)phenoxy)-7-methoxy-6-quinolinecarboxamide;
4-(3-chloro-4-(methylaminocarbonyl)aminophenoxy)-7-methoxy-6-quinolinecarboxamide; and
N6-methoxy-4-(3-chloro-4-(((ethylamino)carbonyl)amino)phenoxy)-7-methoxy-6-quinolinecarboxamide,

a pharmacologically acceptable salt thereof or a solvate thereof.

**129.** The compound, a pharmacologically acceptable salt thereof or a solvate thereof according to Claim 115, wherein the compound represented by General Formula (I), a pharmacologically acceptable salt thereof or a solvate thereof is 4-(3-chloro-4-(cyclopropylaminocarbonyl)aminophenoxy)-7-methoxy-6-quinolinecarboxamide, a pharmacologically acceptable salt thereof or a solvate thereof.

**130.** The compound, a pharmacologically acceptable salt thereof or a solvate thereof according to Claim 115, wherein

the compound represented by General Formula (I), a pharmacologically acceptable salt thereof or a solvate thereof is methanesulfonate of 4-(3-chloro-4-(cyclopropylaminocarbonyl)aminophenoxy)-7-methoxy-6-quinolinecarboxamide.

**131.** The compound, a pharmacologically acceptable salt thereof or a solvate thereof according to Claim 115, wherein gemcitabine, a pharmacologically acceptable salt thereof or a solvate thereof is gemcitabine hydrochloride.

**132.** The compound, a pharmacologically acceptable salt thereof or a solvate thereof according to Claim 115, wherein erlotinib, a pharmacologically acceptable salt thereof or a solvate thereof is erlotinib hydrochloride.

**133.** The compound, a pharmacologically acceptable salt thereof or a solvate thereof according to any one of Claims 115-132, wherein the pharmaceutical composition is for treating cancer.

**134.** A compound represented by General Formula (I), a pharmacologically acceptable salt thereof or a solvate thereof for treating or preventing cancer in combination with gemcitabine or erlotinib, a pharmacologically acceptable salt thereof or a solvate thereof:

General Formula (I)

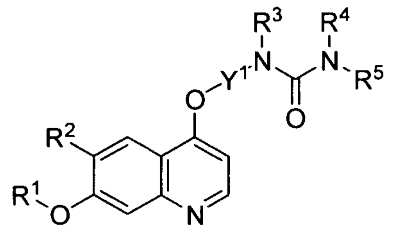

(I)

[wherein, $R^1$ represents a group represented by Formula $-V^1-V^2-V^3$ (wherein, $V^1$ represents an optionally substituted $C_{1-6}$ alkylene group; $V^2$ represents a single bond, an oxygen atom, a sulfur atom, a carbonyl group, a sulfinyl group, a sulfonyl group, a group represented by Formula $-CONR^6-$, a group represented by Formula $-SO_2NR^6-$, a group represented by Formula $-NR^6SO_2-$, a group represented by Formula $-NR^6CO-$ or a group represented by Formula $-NR^6-$ (wherein, $R^6$ represents a hydrogen atom, an optionally substituted $C_{1-6}$ alkyl group or an optionally substituted $C_{3-8}$ cycloalkyl group); $V^3$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group or an optionally substituted 3-10-membered nonaromatic heterocyclic group);
$R^2$ represents a cyano group, an optionally substituted $C_{1-6}$ alkoxy group, a carboxyl group, an optionally substituted $C_{2-7}$ alkoxycarbonyl group or a group represented by Formula $-CONV^{a11}V^{a12}$ (wherein, $V^{a11}$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group or an optionally substituted 3-10-membered nonaromatic heterocyclic group; $V^{a12}$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group, an optionally substituted 3-10-membered nonaromatic heterocyclic group, a hydroxyl group, an optionally substituted $C_{1-6}$ alkoxy group or an optionally substituted $C_{3-8}$ cycloalkoxy group);
$Y^1$ represents a group represented by either one of the following formulae

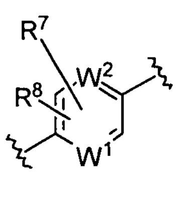

or

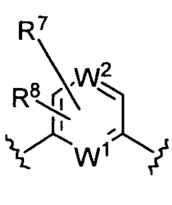

(wherein, $R^7$ and $R^8$ each independently represent a hydrogen atom, a halogen atom, a cyano group, a nitro group, an amino group, an optionally substituted $C_{1-6}$ alkyl group, an optionally substituted $C_{3-8}$ cycloalkyl group, an optionally substituted $C_{1-6}$ alkoxy group, an optionally substituted $C_{1-6}$ alkylthio group, a formyl group, an optionally substituted $C_{2-7}$ acyl group, an optionally substituted $C_{2-7}$ alkoxycarbonyl group or a group represented by Formula $-CONV^{d1}V^{d2}$ (wherein, $V^{d1}$ and $V^{d2}$ each independently represent a hydrogen atom or an optionally substituted $C_{1-6}$ alkyl group);
$W^1$ and $W^2$ each independently represent an optionally substituted carbon atom or nitrogen atom);
$R^3$ and $R^4$ each independently represent a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted
$C_{2-7}$ acyl group or an optionally substituted $C_{2-7}$ alkoxycarbonyl group; and
$R^5$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group or an optionally substituted 3-10-membered nonaromatic heterocyclic group].

**135.** The compound, a pharmacologically acceptable salt thereof or a solvate thereof according to Claim 134, wherein $R^1$ is a $C_{1-6}$ alkyl group (provided that $R^1$ may have a substituent selected from a 3-10-membered nonaromatic heterocyclic group, a hydroxyl group, a $C_{1-6}$ alkoxy group, an amino group, a mono-$C_{1-6}$ alkylamino group and a di-$C_{1-6}$ alkylamino group which may have a $C_{1-6}$ alkyl group).

**136.** The compound, a pharmacologically acceptable salt thereof or a solvate thereof according to Claim 134, wherein $R^1$ is a methyl group or a group represented by any one of the following formulae

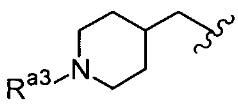
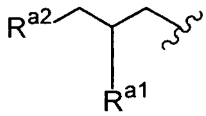
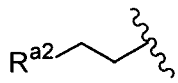

(wherein, $R^{a3}$ represents a methyl group; $R^{a1}$ represents a hydrogen atom or a hydroxyl group; $R^{a2}$ represents a methoxy group, an ethoxy group, a 1-pyrrolidinyl group, a 1-piperidinyl group, a 4-morpholinyl group, a dimethylamino group or a diethylamino group).

**137.** The compound, a pharmacologically acceptable salt thereof or a solvate thereof according to Claim 134, wherein $R^1$ is a methyl group or a 2-methoxyethyl group.

**138.** The compound, a pharmacologically acceptable salt thereof or a solvate thereof according to Claim 134, wherein $R^2$ is a cyano group or a group represented by Formula-$CONV^{a11}V^{a12}$ (wherein, $V^{a11}$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group or an optionally substituted 3-10-membered nonaromatic heterocyclic group; $V^{a12}$ represents a hydrogen atom, an optionally substituted $C_{1-6}$ alkyl group, an optionally substituted $C_{2-6}$ alkenyl group, an optionally substituted $C_{2-6}$ alkynyl group, an optionally substituted $C_{3-8}$ cycloalkyl group, an optionally substituted $C_{6-10}$ aryl group, an optionally substituted 5-10-membered heteroaryl group, an optionally substituted 3-10-membered nonaromatic heterocyclic group, a hydroxyl group, an optionally substituted $C_{1-6}$ alkoxy group or an optionally substituted $C_{3-8}$ cycloalkoxy group).

**139.** The compound, a pharmacologically acceptable salt thereof or a solvate thereof according to Claim 134, wherein $R^2$ is a cyano group or a group represented by Formula-$CONHV^{a16}$ (wherein, $V^{a16}$ represents a hydrogen atom, a $C_{1-6}$ alkyl group, a $C_{3-8}$ cycloalkyl group, a $C_{1-6}$ alkoxy group or a $C_{3-8}$ cycloalkoxy group, provided that $V^{a16}$ may have a substituent selected from a halogen atom, a cyano group, a hydroxyl group and a $C_{1-6}$ alkoxy group).

**140.** The compound, a pharmacologically acceptable salt thereof or a solvate thereof according to Claim 134, wherein $R^2$ is a group represented by Formula $-CONHV^{a17}$ (wherein, $V^{a17}$ represents a hydrogen atom, a $C_{1-6}$ alkyl group or a $C_{1-6}$ alkoxy group).

**141.** The compound, a pharmacologically acceptable salt thereof or a solvate thereof according to Claim 134, wherein

$R^2$ is a group represented by Formula -CONHV$^{a18}$ (wherein, V$^{a18}$ represents a hydrogen atom, a methyl group or a methoxy group).

**142.** The compound, a pharmacologically acceptable salt thereof or a solvate thereof according to Claim 134, wherein $Y^1$ is a group represented by the following formula

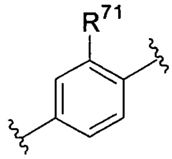

(wherein, $R^{71}$ represents a hydrogen atom or a halogen atom).

**143.** The compound, a pharmacologically acceptable salt thereof or a solvate thereof according to Claim 134, wherein $R^3$ and $R^4$ are hydrogen atoms.

**144.** The compound, a pharmacologically acceptable salt thereof or a solvate thereof according to Claim 134, wherein $R^5$ is a hydrogen atom, a $C_{1-6}$ alkyl group, a $C_{3-8}$ cycloalkyl group or a $C_{6-10}$ aryl group (provided that $R^5$ may have a substituent selected from a halogen atom and a methanesulfonyl group).

**145.** The compound, a pharmacologically acceptable salt thereof or a solvate thereof according to Claim 134, wherein $R^5$ is a methyl group, an ethyl group or a cyclopropyl group.

**146.** The compound, a pharmacologically acceptable salt thereof or a solvate thereof according to Claim 134, wherein the compound represented by General Formula (I), a pharmacologically acceptable salt thereof or a solvate thereof is at least one compound selected from the group consisting of:

N-(4-(6-cyano-7-(2-methoxyethoxy)-4-quinolyl)oxy-2-fluorophenyl)-N'-(4-fluorophenyl)urea;
N-(2-chloro-4-((6-cyano-7-((1-methyl-4-piperidyl)methoxy)-4-quinolyl)oxy)phenyl)-N'-cyclopropylurea;
N-(4-((6-cyano-7-(((2R)-3-(diethylamino)-2-hydroxypropyl)oxy)-4-quinolyl)oxy)phenyl)-N'-(4-fluorophenyl)urea;
N-(4-((6-cyano-7-(((2R)-2-hydroxy-3-(1-pyrrolidino)propyl)oxy)-4-quinolyl)oxy)phenyl)-N'-(4-fluorophenyl)urea;
4-(3-chloro-4-(cyclopropylaminocarbonyl)aminophenoxy)-7-methoxy-6-quinolinecarboxamide;
4-(3-chloro-4-(cyclopropylaminocarbonyl)aminophenoxy)-7-(2-methoxyethoxy)-6-quinolinecarboxamide;
N6-cyclopropyl-4-(3-chloro-4-(((cyclopropylamino)carbonyl)amino)phenoxy)-7-methoxy-6-quinolinecarboxamide;
N6-(2-methoxyethyl)-4-(3-chloro-4-(((cyclopropylamino)carbonyl)amino)phenoxy)-7-methoxy-6-quinolinecarboxamide;
N6-(2-fluoroethyl)-4-(3-chloro-4-(((cyclopropylamino)carbonyl)amino)phenoxy)-7-methoxy-6-quinolinecarboxamide;
N6-methoxy-4-(3-chloro-4-(((cyclopropylamino)carbonyl)amino)phenoxy)-7-methoxy-6-quinolinecarboxamide;
N6-methyl-4-(3-chloro-4-(((cyclopropylamino)carbonyl)amino)phenoxy)-7-methoxy-6-quinolinecarboxamide;
N6-ethyl-4-(3-chloro-4-(((cyclopropylamino)carbonyl)amino)phenoxy)-7-methoxy-6-quinolinecarboxamide;
4-(3-fluoro-4-(cyclopropylaminocarbonyl)aminophenoxy)-7-(2-methoxyethoxy)-6-quinolinecarboxamide;
4-(3-chloro-4-(cyclopropylaminocarbonyl)aminophenoxy)-7-(2-hydroxyethoxy)-6-quinolinecarboxamide;
4-(3-chloro-4-(cyclopropylaminocarbonyl)aminophenoxy)-7-((2S)-2,3-dihydroxypropyl)oxy-6-quinolinecarboxamide;
4-(3-chloro-4-(methylaminocarbonyl)aminophenoxy)-7-methoxy-6-quinolinecarboxamide;
4-(3-chloro-4-(ethylaminocarbonyl)aminophenoxy)-7-methoxy-6-quinolinecarboxamide;
N6-methoxy-4-(3-chloro-4-(((ethylamino)carbonyl)amino)phenoxy)-7-methoxy-6-quinolinecarboxamide;
4-(3-chloro-4-(cyclopropylaminocarbonyl)aminophenoxy)-7-(2-ethoxyethoxy)-6-quinolinecarboxamide;
4-(4-((cyclopropylamino)carbonyl)aminophenoxy)-7-(2-methoxyethoxy)-6-quinolinecarboxamide;

N-(2-fluoro-4-((6-carbamoyl-7-methoxy-4-quinolyl)oxy)phenyl)-N'-cyclopropylurea;
N6-(2-hydroxyethyl)-4-(3-chloro-4-(((cyclopropylamino)carbonyl)amino)phenoxy)-7-methoxy-6-quinolinecarboxamide;
4-(3-chloro-4-(1-propylaminocarbonyl)aminophenoxy)-7-methoxy-6-quinolinecarboxamide;
4-(3-chloro-4-(cis-2-fluoro-cyclopropylaminocarbonyl)aminophenoxy)-7-methoxy-6-quinolinecarboxamide;
N6-methyl-4-(3-chloro-4-(((cyclopropylamino)carbonyl)amino)phenoxy)-7-(2-methoxyethoxy)-6-quinolinecarboxamide;
N6-methyl-4-(3-chloro-4-(((ethylamino)carbonyl)amino)phenoxy)-7-methoxy-6-quinolinecarboxamide;
4-(3-chloro-4-(cyclopropylaminocarbonyl)aminophenoxy)-7-(2-(4-morpholino)ethoxy)-6-quinolinecarboxamide;
4-(3-chloro-4-(2-fluoroethylaminocarbonyl)aminophenoxy)-7-methoxy-6-quinolinecarboxamide;
N6-((2R)tetrahydro-2-furanylmethyl)-4-(3-chloro-4-(((methylamino)carbonyl)amino)phenoxy)-7-methoxy-6-quinolinecarboxamide;
4-(3-fluoro-4-(ethylaminocarbonyl)aminophenoxy)-7-methoxy-6-quinolinecarboxamide;
4-(3-chloro-4-(((cyclopropylamino)carbonyl)amino)phenoxy)-7-((2R)-2-hydroxy-3-(1-pyrrolidino)propoxy)-6-quinolinecarboxamide;
N6-methyl-4-(3-chloro-4-(((methylamino)carbonyl)amino)phenoxy)-7-((2R)-3-diethylamino-2-hydroxypropoxy)-6-quinolinecarboxamide;
N6-methyl-4-(3-chloro-4-(((ethylamino)carbonyl)amino)phenoxy)-7-((2R)-3-diethylamino-2-hydroxypropoxy)-6-quinolinecarboxamide;
N6-methyl-4-(3-chloro-4-(((methylamino)carbonyl)amino)phenoxy)-7-((2R)-2-hydroxy-3-(1-pyrrolidino)propoxy)-6-quinolinecarboxamide;
N6-methyl-4-(3-chloro-4-(((ethylamino)carbonyl)amino)phenoxy)-7-((2R)-2-hydroxy-3-(1-pyrrolidino)propoxy)-6-quinolinecarboxamide;
N6-methyl-4-(3-chloro-4-(((methylamino)carbonyl)amino)phenoxy)-7-((1-methyl-4-piperidyl)methoxy)-6-quinolinecarboxamide;
N6-methyl-4-(3-chloro-4-(((ethylamino)carbonyl)amino)phenoxy)-7-((1-methyl-4-piperidyl)methoxy)-6-quinolinecarboxamide;
N-(4-(6-cyano-7-(2-methoxyethoxy)-4-quinolyl)oxy-2-fluorophenyl)-N'-cyclopropylurea;
N-(4-(6-cyano-7-(3-(4-morpholino)propoxy)-4-quinolyl)oxyphenyl)-N'-(3-(methylsulfonyl)phenyl)urea;
4-(4-((cyclopropylamino)carbonyl)aminophenoxy)-7-methoxy-6-quinolinecarboxamide;
4-(3-fluoro-4-((2-fluoroethylamino)carbonyl)aminophenoxy)-7-methoxy-6-quinolinecarboxamide;
N6-(2-ethoxyethyl)-4-(3-chloro-4-(((methylamino)carbonyl)amino)phenoxy)-7-methoxy-6-quinolinecarboxamide;
4-(4-(3-ethylureido)-3-fluoro-phenoxy)-7-methoxyquinoline-6-carboxylic acid (2-cyanoethyl)amide; and
N-(4-(6-(2-cyanoethyl)carbamoyl-7-methoxy-4-quinolyl)oxy-2-fluorophenyl)-N'-cyclopropylurea,

a pharmacologically acceptable salt thereof or a solvate thereof.

**147.** The compound, a pharmacologically acceptable salt thereof or a solvate thereof according to Claim 134, wherein the compound represented by General Formula (I), a pharmacologically acceptable salt thereof or a solvate thereof is at least one compound selected from the group consisting of:

4-(3-chloro-4-(cyclopropylaminocarbonyl)aminophenoxy)-7-methoxy-6-quinolinecarboxamide;
4-(3-chloro-4-(ethylaminocarbonyl)aminophenoxy)-7-methoxy-6-quinolinecarboxamide;
N6-methoxy-4-(3-chloro-4-(((cyclopropylamino)carbonyl)amino)phenoxy)-7-methoxy-6-quinolinecarboxamide;
4-(3-chloro-4-(methylaminocarbonyl)aminophenoxy)-7-methoxy-6-quinolinecarboxamide; and
N6-methoxy-4-(3-chloro-4-(((ethylamino)carbonyl)amino)phenoxy)-7-methoxy-6-quinolinecarboxamide,

a pharmacologically acceptable salt thereof or a solvate thereof.

**148.** The compound, a pharmacologically acceptable salt thereof or a solvate thereof according to Claim 134, wherein the compound represented by General Formula (I), a pharmacologically acceptable salt thereof or a solvate thereof is 4-(3-chloro-4-(cyclopropylaminocarbonyl)aminophenoxy)-7-methoxy-6-quinolinecarboxamide, a pharmacologically acceptable salt thereof or a solvate thereof.

**149.** The compound, a pharmacologically acceptable salt thereof or a solvate thereof according to Claim 134, wherein

the compound represented by General Formula (I), a pharmacologically acceptable salt thereof or a solvate thereof is methanesulfonate of 4-(3-chloro-4-(cyclopropylaminocarbonyl)aminophenoxy)-7-methoxy-6-quinolinecarboxamide.

**150.** The compound, a pharmacologically acceptable salt thereof or a solvate thereof according to Claim 134, wherein gemcitabine, a pharmacologically acceptable salt thereof or a solvate thereof is gemcitabine hydrochloride.

**151.** The compound, a pharmacologically acceptable salt thereof or a solvate thereof according to Claim 134, wherein erlotinib, a pharmacologically acceptable salt thereof or a solvate thereof is erlotinib hydrochloride.

**152.** The compound, a pharmacologically acceptable salt thereof or a solvate thereof according to any one of Claims 134-151, wherein the pharmaceutical composition is for treating cancer.

Figure 1

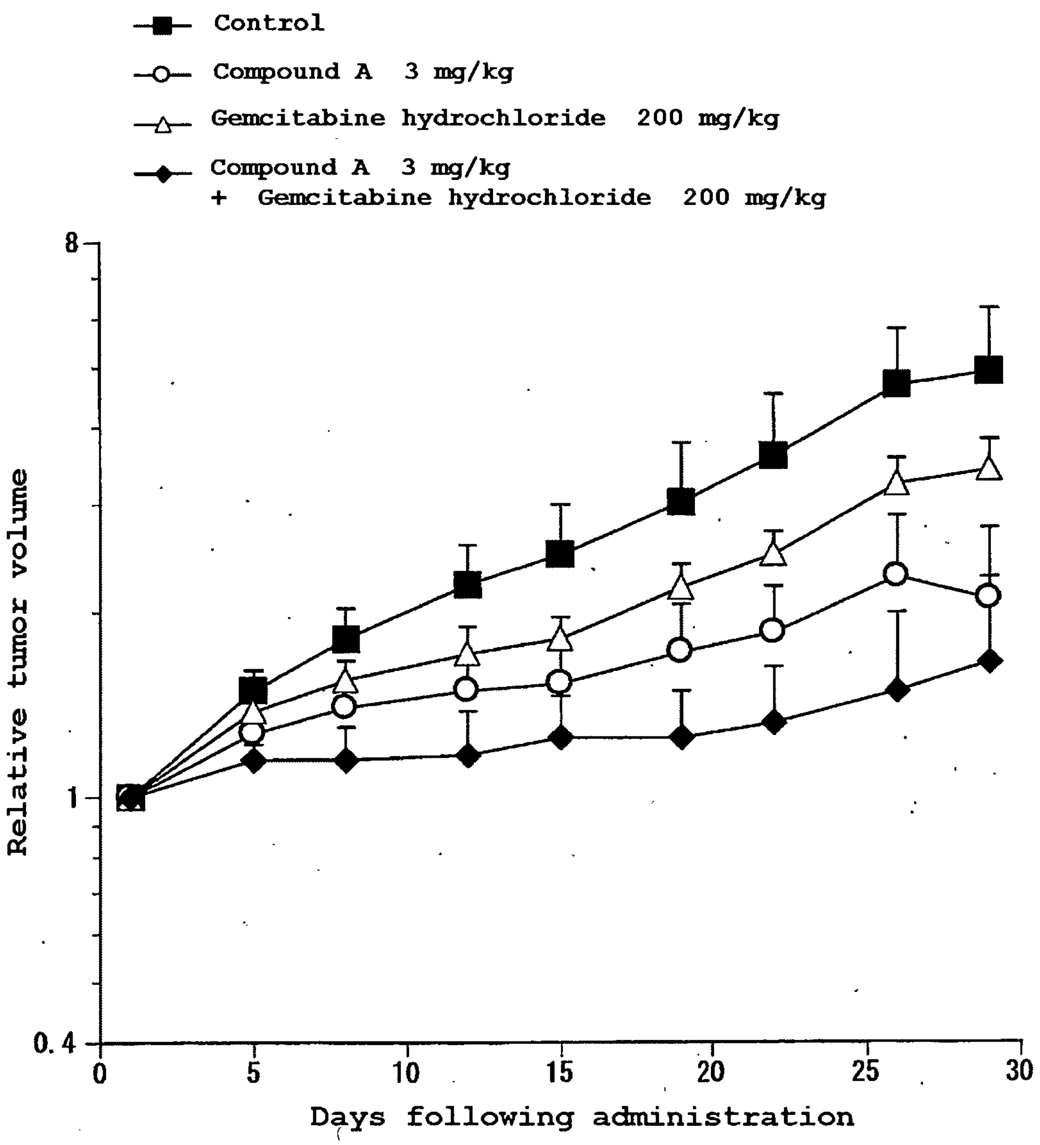
Control
Compound A 3 mg/kg
Gemcitabine hydrochloride 200 mg/kg
Compound A 3 mg/kg
+ Gemcitabine hydrochloride 200 mg/kg
8
1
0.4
Relative tumor volume
0
5
10
15
20
25
30
Days following administration

Figure 2

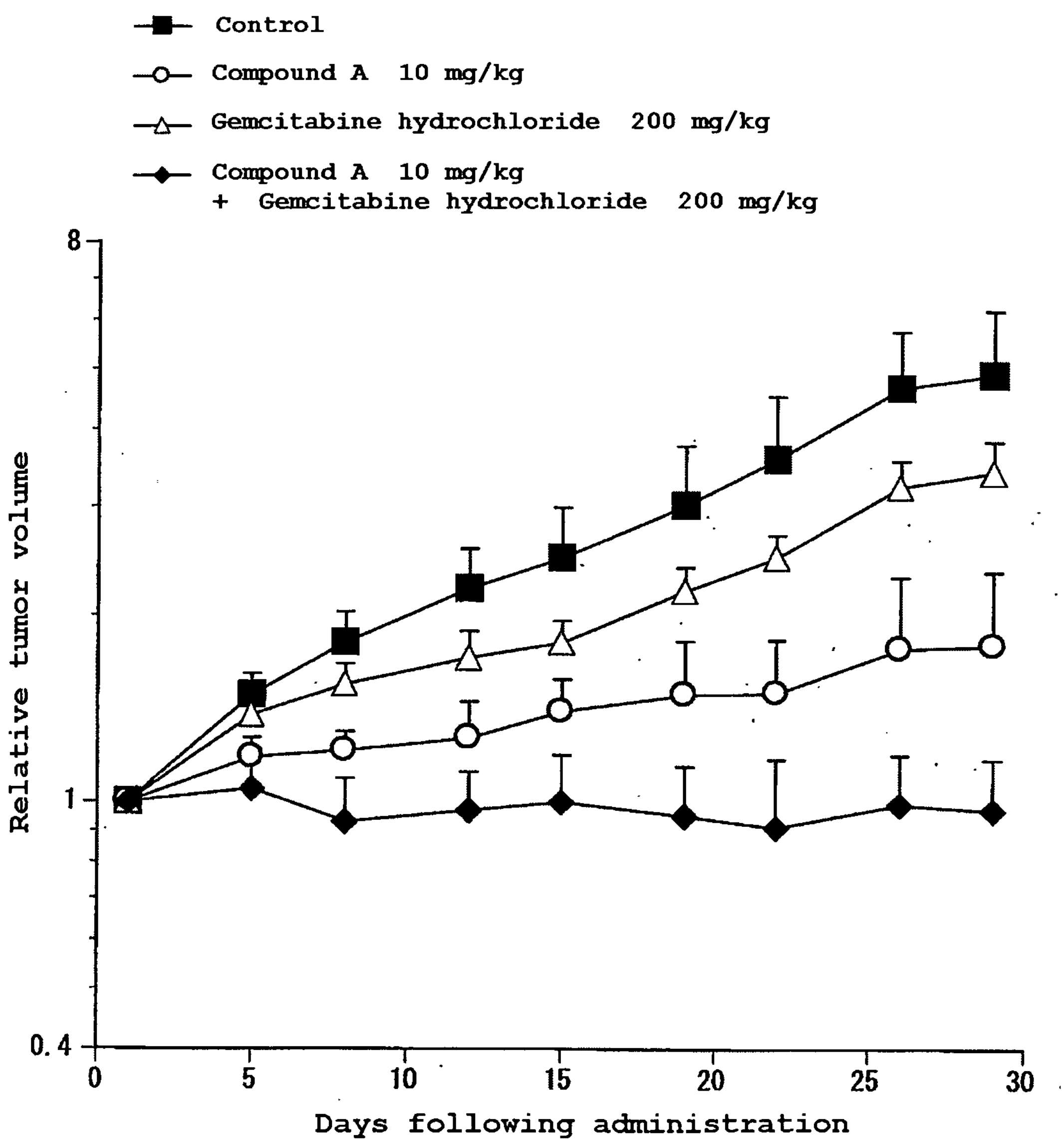
Control
Compound A 10 mg/kg
Gemcitabine hydrochloride 200 mg/kg
Compound A 10 mg/kg
+ Gemcitabine hydrochloride 200 mg/kg
8
1
0.4
Relative tumor volume
0
5
10
15
20
25
30
Days following administration

Figure 3

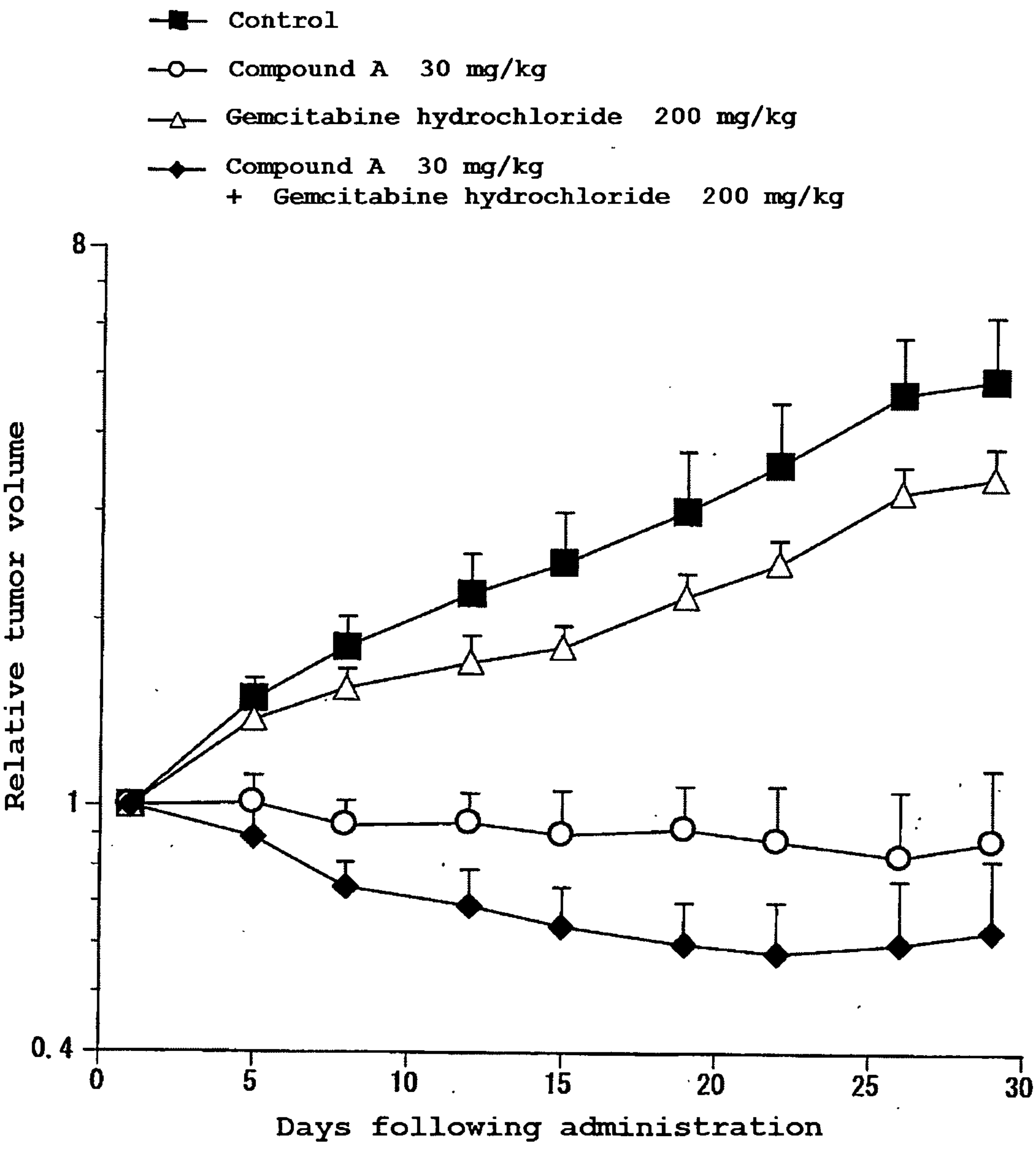
Control
Compound A 30 mg/kg
Gemcitabine hydrochloride 200 mg/kg
Compound A 30 mg/kg
+ Gemcitabine hydrochloride 200 mg/kg
8
1
0.4
Relative tumor volume
0
5
10
15
20
25
30
Days following administration

Figure 4

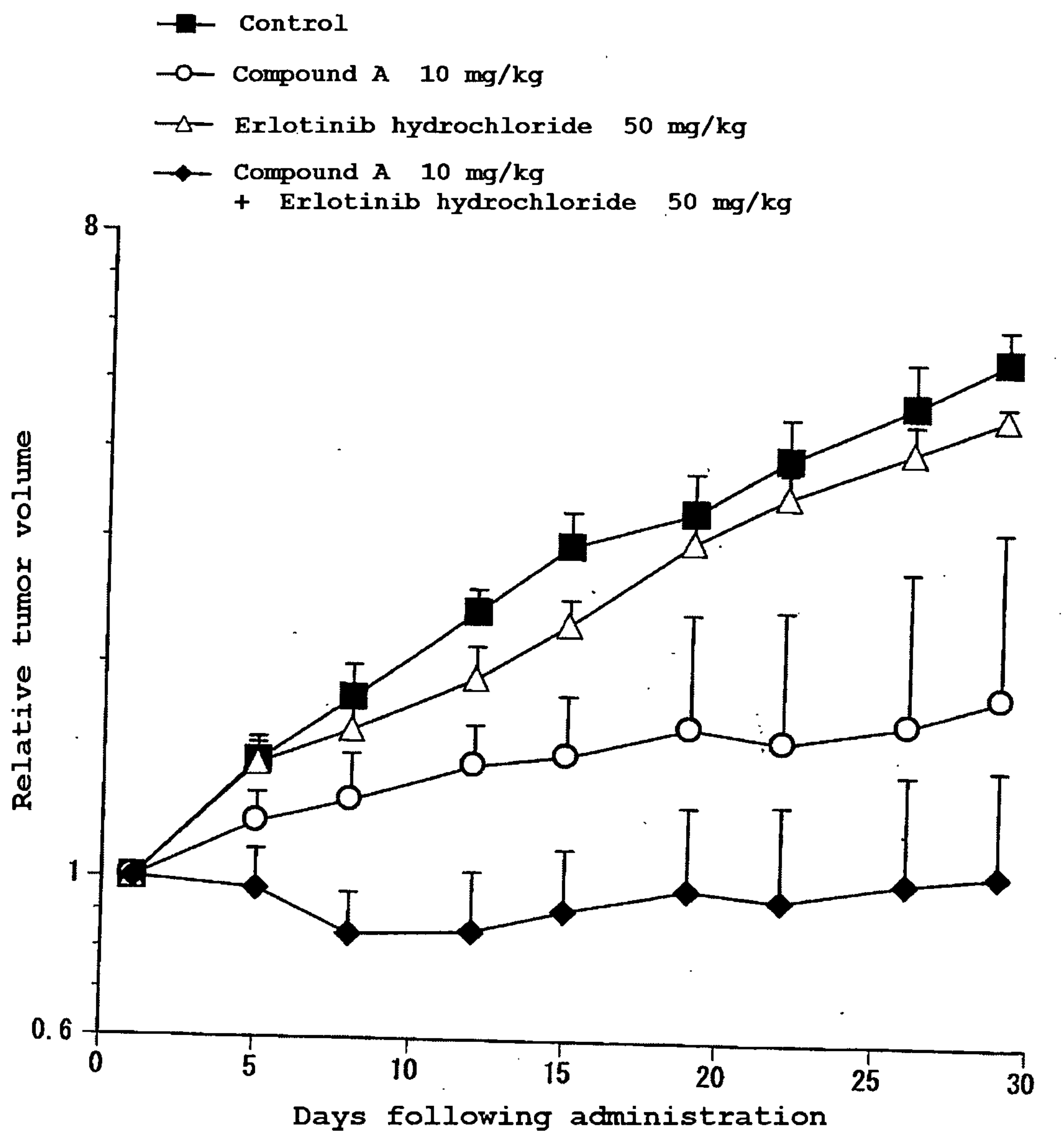
Control
Compound A 10 mg/kg
Erlotinib hydrochloride 50 mg/kg
Compound A 10 mg/kg
+ Erlotinib hydrochloride 50 mg/kg
8
1
0.6
Relative tumor volume
0
5
10
15
20
25
30
Days following administration

**INTERNATIONAL SEARCH REPORT**

International application No.
PCT/JP2008/051024

A. CLASSIFICATION OF SUBJECT MATTER
*A61K31/47*(2006.01)i, *A61K31/517*(2006.01)i, *A61K31/7068*(2006.01)i, *A61P35/00*(2006.01)i, *C07D215/48*(2006.01)i

According to International Patent Classification (IPC) or to both national classification and IPC

B. FIELDS SEARCHED

Minimum documentation searched (classification system followed by classification symbols)
A61K31/47, A61K31/517, A61K31/7068, A61P35/00, C07D215/48

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)
BIOSIS(STN), CAplus(STN), EMBASE(STN), MEDLINE(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br><br>Y | WO 2002/032872 A1 (Eisai Co., Ltd.), 25 April, 2002 (25.04.02), Full text; particularly, Claims; example 368; pharmacological test examples<br>& JP 2005-272474 A & US 2004/0053908 A1<br>& EP 1415987 A1 & AU 9598601 A<br>& NO 20031731 A & CA 2426461 A<br>& CN 1478078 A | 20-38,58-76, 115-152<br>1-19,39-57, 96-114 |
| X<br><br>Y | WO 2005/063713 A1 (Eisai Co., Ltd.), 14 July, 2005 (14.07.05), Full text; particularly, Claims; examples<br>& EP 1698623 A1 & NO 20063383 A<br>& CN 1890220 A & US 2007/0078159 A1 | 20-38,58-76, 115-152<br>1-19,39-57, 96-114 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 24 March, 2008 (24.03.08) | 01 April, 2008 (01.04.08) |
| Name and mailing address of the ISA/<br>Japanese Patent Office<br><br>Facsimile No. | Authorized officer<br><br><br>Telephone No. |

Form PCT/ISA/210 (second sheet) (April 2007)

**INTERNATIONAL SEARCH REPORT**

International application No.
PCT/JP2008/051024

C (Continuation). DOCUMENTS CONSIDERED TO BE RELEVANT

| Category* | Citation of document, with indication, where appropriate, of the relevant passages | Relevant to claim No. |
|---|---|---|
| Y | WO 2005/117887 A1 (F.HOFFMANN-LA ROCHE AG.), 15 December, 2005 (15.12.05), Full text; particularly, Claims 1 to 3, 23, 24 & US 2005/0272688 A1 & EP 1755608 A1 & CA 2567836 A & NO 20066056 A & CN 1960732 A | 1-19,39-57, 96-114 |
| Y | WO 2006/090931 A1 (Eisai Co., Ltd.), 31 August, 2006 (31.08.06), Full text; particularly, Claims; examples (Family: none) | 1-19,39-57, 96-114 |
| Y | WO 2006/090930 A1 (Eisai Co., Ltd.), 31 August, 2006 (31.08.06), Full text; particularly, Claims; examples (Family: none) | 1-19,39-57, 96-114 |

Form PCT/ISA/210 (continuation of second sheet) (April 2007)

**INTERNATIONAL SEARCH REPORT**

International application No.
PCT/JP2008/051024

**Box No. II Observations where certain claims were found unsearchable (Continuation of item 2 of first sheet)**

This international search report has not been established in respect of certain claims under Article 17(2)(a) for the following reasons:

1. [X] Claims Nos.: 77-95
because they relate to subject matter not required to be searched by this Authority, namely:
Claims 77 to 95 pertain to methods for treatment of the human body by therapy.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying additional fees, this Authority did not invite payment of additional fees.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** the
[ ] The additional search fees were accompanied by the applicant's protest and, where applicable, payment of a protest fee.
[ ] The additional search fees were accompanied by the applicant's protest but the applicable protest fee was not paid within the time limit specified in the invitation.
[ ] No protest accompanied the payment of additional search fees.

Form PCT/ISA/210 (continuation of first sheet (2)) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2004043472 A
- WO 2004041308 A
- WO 2004032872 A
- WO 2004032937 A
- WO 2002080975 A
- WO 200232872 A
- WO 2005063713 A
- US 88573307 P
- US 60887010 P
- WO 0232872 A
- US 5464826 A
- WO 4526988 A
- US 4526988 A
- WO 9630347 A
- JP 3088018 B
- JP 3420549 B

### Non-patent literature cited in the description


- **Haller, DG.** *Int. J. Radiation Oncology Biol. Phys.,* 2003, vol. 56, 16-23
- **Baker et al.** *Cancer Research,* 2002, vol. 62, 1996-2003
- **Bruns et al.** *International Journal of Cancer,* 2002, vol. 102, 101-108
- *Asu no Shinyaku,* 06 December 2006, 81-83
- **Heinemann, V. et al.** *Cancer Research,* 1988, vol. 48, 4024-4031
- **Hertel LW. et al.** *Cancer Research,* 1990, vol. 50, 4417-4422